# EUROPEAN PATENT APPLICATION

(11) **EP 4 467 539 A1**
(43) Date of publication of application: **27.11.2024**
(21) Application number: 23743552.4
(22) Date of filing: 20.01.2023
(51) Int. Cl.: C07D 413/04, A61K 31/421, A61K 31/422, A61K 31/4709, A61K 31/4155, A61P 31/04, A61P 31/06, C07D 263/32, C07D 413/10

(54) **OXAZOLE DERIVATIVE COMPOUND HAVING ANTIBACTERIAL ACTIVITY AND MEDICAL USE THEREOF**

(30) Priority: 21.01.2022 KR 20220009418
(71) Applicant: Institut Pasteur Korea, Seongnam-si, Gyeonggi-do 13488 (KR); International Tuberculosis Research Center, Changwon-si, Gyeongsangnam-do 51755 (KR); Korean National Tuberculosis Association, Seoul 06763 (KR)
(72) Inventor: CHOI, Inhee, Seoul 05501 (KR); KIM, Young Mi, Uiwang-si, Gyeonggi-do 16036 (KR); LEE, Aram, Yongin-si, Gyeonggi-do 17081 (KR); CUI, Lianji, Yongin-si, Gyeonggi-do 16977 (KR); LEE, Jong Seok, Changwon-si, Gyeongsangnam-do 51772 (KR); SON, Eunsoon, Changwon-si, Gyeongsangnam-do 51772 (KR); LEE, Ji-im, Busan 46997 (KR); AHN, Ji Eun, Gimhae-si, Gyeongsangnam-do 50862 (KR); JEONG, Mi Ji, Changwon-si, Gyeongsangnam-do 51319 (KR); KIM, Kyungjong, Sejong 30100 (KR); WHANG, Jake, Daejeon 34140 (KR); YANG, Jeong Seong, Cheongju-si, Chungcheongbuk-do 28617 (KR); PARK, Yumi, Sejong 30146 (KR)
(74) Representative: Engelhard, Markus
(86) International application number: PCT/KR2023/001070
(87) International publication number: WO 2023/140709

(57) **Abstract**

The present disclosure provides a novel oxazole derivative compound having anti-Mycobacterium tuberculosis activity or a pharmaceutically acceptable salt thereof. The present disclosure also provides an antibacterial composition comprising the compound or a pharmaceutically acceptable salt thereof. The novel oxazole compound according to the present disclosure has remarkably excellent antibacterial activity, particularly anti-Mycobacterium tuberculosis activity, and thus can be effectively used in the prevention or treatment of diseases caused by bacterial infection.

## Description

### [Technical Field]

This application claims priority based on Korean Patent Application No. 10-2022-0009418 filed on January 21, 2022, all contents disclosed in the specification and drawings of which are incorporated herein by reference in their entirety.

The present disclosure relates to compounds containing an oxazole structure or pharmaceutically acceptable salts thereof. The present disclosure also relates to an antibacterial pharmaceutical composition comprising such a compound or a pharmaceutically acceptable salt thereof. The present disclosure also relates to useful methods for treating or preventing diseases caused by bacterial infections, particularly Mycobacterium tuberculosis infections, using these compounds. That is, the present disclosure relates to medical uses of the compounds according to the present disclosure for treating or preventing bacterial diseases, especially tuberculosis.

### [Background Art]

Tuberculosis causes 10 million new patients every year and is one of the top 10 causes of death worldwide. Additionally, many of the new tuberculosis patients are reported to be drug-resistant tuberculosis patients.

Inappropriate use of chemotherapy has led to multi-drug resistant (MDR) tuberculosis (TB), a situation that is worsening with the emergence and spread of widespread drug-resistant TB strains. The most urgent clinical need is to develop treatments that can effectively combat multidrug-resistant tuberculosis (MDR-TB) or extensively drug-resistant tuberculosis (XDR-TB), which can shorten the treatment time for tuberculosis.

Current chemotherapy consists of compounds that target Mycobacterium tuberculosis directly by targeting one of the major components of its cell wall or the synthesis of macromolecules such as DNA, RNA or protein. The most widely used anti-tuberculosis drugs are isoniazid, ethionamide, and pyrazinamide, and are used in co-administration combinations of these first-line drugs. They mainly exhibit inhibitory activity by targeting cell wall synthesis and/or a wide range of mycobacteria that have not yet been characterized.

Since standard treatment was proposed in the 1940s, only two drugs, delamanid and bedaquiline, have been approved as new anti-tuberculosis drugs, and given the continued emergence of drug-resistant tuberculosis and the speed at which resistance develops, the development of additional clinical candidates is necessary.

### [Disclosure]

### [Technical Problem]

Therefore, the object that the present disclosure aims to solve is to provide a compound that has antibacterial activity and is effective in preventing and treating a disease caused by bacterial infection (particularly, tuberculosis).

Another object of the present disclosure is to provide a pharmaceutical composition for treating or preventing a disease caused by bacterial infection, especially tuberculosis, comprising the above compound or a pharmaceutically acceptable salt thereof as an active ingredient.

Yet another object of the present disclosure is to provide a method for treating or preventing a disease caused by bacterial infection, especially tuberculosis, comprising administering to a subject in need of treatment or prevention the compound according to the present invention or a pharmaceutically acceptable salt thereof.

### [Technical Solution]

### Compounds of the present invention

In order to solve the above problem, one embodiment of the present invention provides a compound represented by the following Chemical Formula 1 or a pharmaceutically acceptable salt thereof.

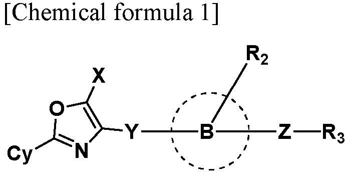

In the chemical formula 1,
Cy is phenyl, benzo[d][1,3]dioxole, 2,3-dihydrobenzofuran, 2,3-dihydrobenzo[b][1,4]dioxine, imidazo[1,2-a]pyridine, naphthalene, or quinoline, which are unsubstituted or substituted with one or more R₁,
R₁ is each independently halogen; C₁₋₄alkyl; C₁₋₄alkoxy; C₃₋₆cycloalkyl; C₁₋₄haloalkoxy (preferably, -OCF₃); C₁₋₄haloalkyl (preferably, -CF₃, -CHF₂); -OH; phenoxy; phenyl; -CN; -NH₂; -N(CH₃)₂; -NO₂; -NHCH₃; -NHAc; -CO₂H; -CO₂CH₃; -C(O)NHCH₃; -C(O)N(CH₃)₂; -NHC(O)-C₁₋₄alkyl; -NHC(O)phenyl; phenyl substituted with one or more selected from the group consisting of halogen, C₁₋₄alkyl, C₃₋₆cycloalkyl, C₁₋₄haloalkoxy (preferably, -OCF₃), C₁₋₄haloalkyl (preferably, -CF₃, -CHF₂), -OH, phenoxy, phenyl, C₁₋₄alkoxy, -CN, -NH₂, and -N(CH₃)₂; pyridine unsubstituted or substituted with halogen; naphthalene unsubstituted or substituted with C₁₋₄alkoxy; benzotriazole; quinoline or isoquinoline unsubstituted or substituted with one or more selected from the group consisting of C₁₋₄alkyl, C₁₋₄alkoxy, and halogen; indazole unsubstituted or substituted with C₁₋₄alkyl; C₃₋₆cycloalkyl; benzothiophene; benzofuran; indole unsubstituted or substituted with C₁₋₄alkyl; pyrazole unsubstituted or substituted with C₁₋₄alkyl; imidazole unsubstituted or substituted with C₁₋₄alkyl; triazole; pyrrolidine; piperidine unsubstituted or substituted with C₁₋₄alkyl; piperazine unsubstituted or substituted with C₁₋₄alkyl; pyrrole; furan; morpholine; or thiophene,
X is hydrogen, halogen, C₁₋₄alkyl, C₁₋₄haloalkyl (preferably, -CF₃, -CHF₂), C₃₋₆cycloalkyl, C₁₋₄alkoxy, C₁₋₄haloalkoxy (preferably, -OCF₃), -OH, phenoxy, phenyl, -CN, -NH₂, or -N(CH₃)₂,
Y is -NH- or -CH₂-,
B is phenyl; piperidine; piperazine; or 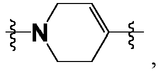 which are unsubstituted or substituted with one or more R₂; or B is 3 or 4-spiropiperidine which is a spiro compound linked by sharing carbon with chemical formula 2a, 2b or 2c, 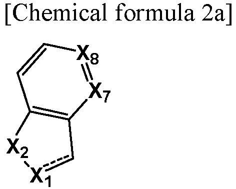 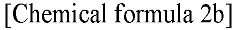 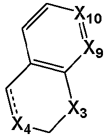 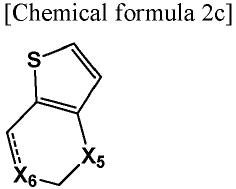
   in the chemical formula 2a to 2c,
   X₁ to X₆ are each independently CH, CH₂, O, NH, or N,
   X₇ to X₁₀ are each independently CH or N,
   - - - - - - is a single bond or double bond,
R₂ is -OH, phenyl, halogen, C₁₋₄alkyl, C₃₋₆cycloalkyl, C₁₋₄haloalkoxy (preferably, -OCF₃), C₁₋₄haloalkyl (preferably, -CF₃, -CHF₂), phenoxy, C₁₋₄alkoxy, -CN, -NH₃, -N(CH₃)₂, or =O,
Z is absent (forming a spiro compound), direct linking, -NH-, -N(CH₃)-, -O-, or -O(CH₂)-,
R₃ is hydrogen; halogen; C₁₋₄alkyl; C₃₋₆cycloalkyl; C₁₋₄haloalkoxy (preferably, -OCF₃); C₁₋₄haloalkyl (preferably, -CF₃, -CHF₂); -OH; phenoxy; phenyl; C₁₋₄alkoxy; -CN; -NH₂; -N(CH₃)₂; phenyl substituted with one or more selected from the group consisting of halogen, C₁₋₄alkyl, C₃₋₆cycloalkyl, C₁₋₄haloalkoxy (preferably, -OCF₃), C₁₋₄haloalkyl (preferably, -CF₃, -CHF₂), -OH, phenoxy, phenyl, C₁₋₄alkoxy, -CN, -NO₂, -NH₂, and -N(CH₃)₂; pyridine unsubstituted or substituted with halogen; naphthalene unsubstituted or substituted with C₁₋₄alkoxy; benzotriazole; quinoline or isoquinoline substituted with one or more selected from the group consisting of C₁₋₄alkyl, C₁₋₄alkoxy, and halogen; indazole unsubstituted or substituted with C₁₋₄alkyl; C₃₋₆ cycloalkyl; benzothiophene; benzofuran; indole unsubstituted or substituted with C₁₋₄alkyl; pyrazole; imidazole; pyrrolidine; piperidine unsubstituted or substituted with C₁₋₄alkyl; piperazine unsubstituted or substituted with C₁₋₄alkyl; pyrrole; furan; thiophene; morpholine or thiophene.

In one embodiment of the present invention, the 'chemical formula 2a, 2b or 2c' is a 5 to 6-membered monocyclic heterocycle (preferably, tetrahydrofuran, tetrahydropyran, dihydropyran, cyclopentane, cyclohexane, cyclopentene, cyclohexene, dihydrpyrrole, pyrrolidine, piperidine or oxazine) fused with benzene or a 5 to 7-membered monocyclic heteroaryl (preferably, pyridine, pyridazine, pyrimidine, pyrazine or thiophene).

In one embodiment of the present invention, 3 or 4-spiropiperidine may be, for example, one of the following substituents:

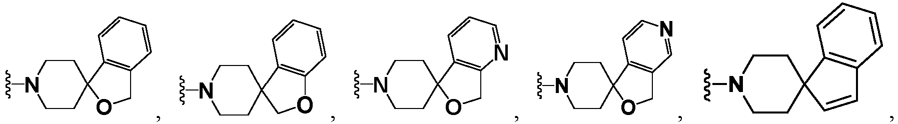

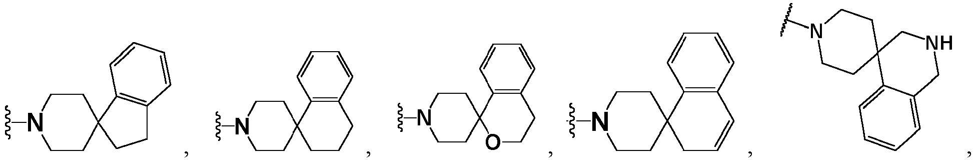

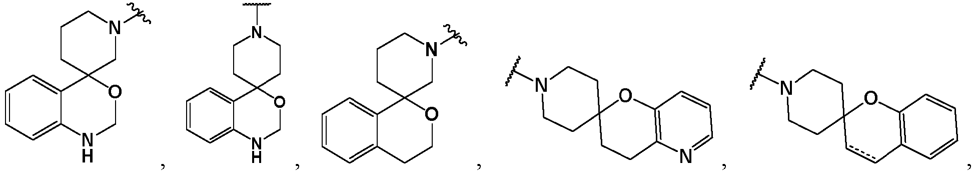

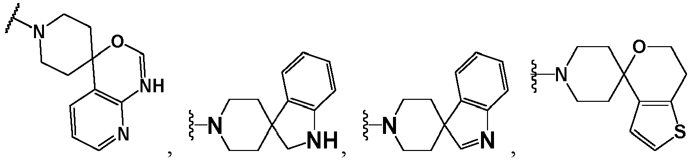

In one embodiment of the present invention, the 3 or 4-spiropiperidine is substituted with one or more R₂. For example, the 3 or 4-spiropiperidine may have a carboxyl group in the ring by replacing two hydrogens with =O. For example, in the 3 or 4-spiropiperidine, one or more hydrogens may be substituted with halogen (e.g., F, Cl), C₁₋₄alkoxy (e.g., methoxy), etc.

In a preferred embodiment of the present invention, the 3 or 4-spiropiperidine is, for example, one of the following substituents:

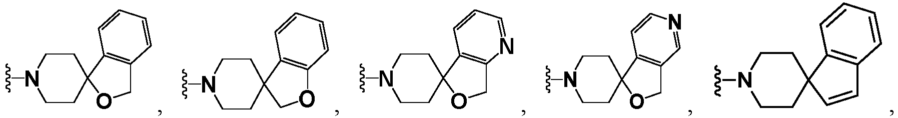

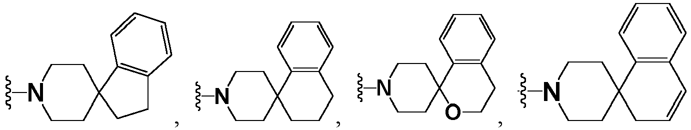

Preferably, in another embodiment of the present invention, the present disclosure provides a compound of the chemical formula 1 or a pharmaceutically acceptable salt thereof, wherein
Cy is phenyl, benzo[d][1,3]dioxole, 2,3-dihydrobenzofuran, 2,3-dihydrobenzo[b][1,4]dioxine, imidazo[1,2-a]pyridine, naphthalene or quinoline, which are unsubstituted or substituted with one or more R₁,
R₁ is each independently halogen; C₁₋₄alkyl; C₁₋₄alkoxy; C₃₋₆cycloalkyl; C₁₋₄haloalkoxy (preferably, -OCF₃); C₁₋₄haloalkyl (preferably, -CF₃, -CHF₂); -OH; phenoxy; phenyl; -CN; -NH₂; -N(CH₃)₂; -NO₂; -NHCH₃; -NHAc; -CO₂H; -CO₂CH₃; -C(O)NHCH₃; -C(O)N(CH₃)₂; -NHC(O)-C₁₋₄alkyl; -NHC(O)phenyl; phenyl substituted with one or more selected from the group consisting of halogen, C₁₋₄alkyl, C₃₋₆cycloalkyl, C₁₋₄haloalkoxy (preferably, -OCF₃), C₁₋₄haloalkyl (preferably, -CF₃, -CHF₂), -OH, phenoxy, phenyl, C₁₋₄alkoxy, -CN, -NH₂, and -N(CH₃)₂; pyridine unsubstituted or substituted with halogen; naphthalene unsubstituted or substituted with C₁₋₄alkoxy; benzotriazole; quinoline or isoquinoline unsubstituted or substituted with one or more selected from the group consisting of C₁₋₄alkyl, C₁₋₄alkoxy, and halogen; indazole unsubstituted or substituted with C₁₋₄alkyl; C₃₋₆cycloalkyl; benzothiophene; benzofuran; indole unsubstituted or substituted with C₁₋₄alkyl; pyrazole unsubstituted or substituted with C₁₋₄alkyl; imidazole unsubstituted or substituted with C₁₋₄alkyl; triazole; pyrrolidine; piperidine unsubstituted or substituted with C₁₋₄alkyl; piperazine unsubstituted or substituted with C₁₋₄alkyl; pyrrole; furan; morpholine; or thiophene,
X is hydrogen, halogen, C₁₋₄alkyl, C₁₋₄haloalkyl (preferably, -CF₃, -CHF₂), C₃₋₆cycloalkyl, C₁₋₄alkoxy, C₁₋₄haloalkoxy (preferably, -OCF₃), -OH, phenoxy, phenyl, -CN, -NH₂, or -N(CH₃)₂,
Y is -CH₂-,
B is phenyl; piperidine; or piperazine, which are unsubstituted or substituted with one or more R₂; or B is 3 or 4-spiropiperidine which is a spiro compound linked by sharing carbon with chemical formula 2a, 2b or 2c,
R₂ is -OH, phenyl, halogen, C₁₋₄alkyl, C₃₋₆cycloalkyl, C₁₋₄haloalkoxy (preferably, -OCF₃), C₁₋₄haloalkyl (preferably, -CF₃, -CHF₂), phenoxy, C₁₋₄alkoxy, -CN, -NH₃, or -N(CH₃)₂,
Z is absent (forming a spiro compound), direct linking, -O-, or -O(CH₂)-,
R₃ is hydrogen; halogen; C₁₋₄alkyl; C₃₋₆cycloalkyl; C₁₋₄haloalkoxy (preferably, -OCF₃); C₁₋₄haloalkyl (preferably, -CF₃, -CHF₂); -OH; phenoxy; phenyl; C₁₋₄alkoxy; -CN; -NH₂; -N(CH₃)₂; phenyl substituted with one or more selected from the group consisting of halogen, C₁₋₄alkyl, C₃₋₆cycloalkyl, C₁₋₄haloalkoxy (preferably, -OCF₃), C₁₋₄haloalkyl (preferably, -CF₃, -CHF₂), -OH, phenoxy, phenyl, C₁₋₄alkoxy, -CN, -NO₂, -NH₂, and -N(CH₃)₂; pyridine unsubstituted or substituted with halogen; naphthalene unsubstituted or substituted with C₁₋₄alkoxy; benzotriazole; quinoline or isoquinoline substituted with one or more selected from the group consisting of C₁₋₄alkyl, C₁₋₄alkoxy, and halogen; indazole unsubstituted or substituted with C₁₋₄alkyl; C₃₋₆ cycloalkyl; benzothiophene; benzofuran; indole unsubstituted or substituted with C₁₋₄alkyl; pyrazole; imidazole; pyrrolidine; piperidine unsubstituted or substituted with C₁₋₄alkyl; piperazine unsubstituted or substituted with C₁₋₄alkyl; pyrrole; furan; thiophene; morpholine or thiophene.

More preferably, in another embodiment of the present invention, the present disclosure provides a compound of the chemical formula 1 or a pharmaceutically acceptable salt thereof, wherein
Cy is phenyl unsubstituted or substituted with one or more R₁,
R₁ is each independently halogen; C₁₋₄alkyl; C₁₋₄alkoxy; C₃₋₆cycloalkyl; C₁₋₄haloalkoxy (preferably, -OCF₃); C₁₋₄haloalkyl (preferably, -CF₃, -CHF₂); -OH; phenoxy; phenyl; -CN; -NH₂; -N(CH₃)₂; -NO₂; -NHCH₃; -NHAc; -CO₂H; -CO₂CH₃; -C(O)NHCH₃; -C(O)N(CH₃)₂; -NHC(O)-C₁₋₄alkyl; -NHC(O)phenyl; phenyl substituted with one or more selected from the group consisting of halogen, C₁₋₄alkyl, C₃₋₆cycloalkyl, C₁₋₄haloalkoxy (preferably, -OCF₃), C₁₋₄haloalkyl (preferably, -CF₃, -CHF₂), -OH, phenoxy, phenyl, C₁₋₄alkoxy, -CN, -NH₂, and -N(CH₃)₂; pyridine unsubstituted or substituted with halogen; naphthalene unsubstituted or substituted with C₁₋₄alkoxy; benzotriazole; quinoline or isoquinoline unsubstituted or substituted with one or more selected from the group consisting of C₁₋₄alkyl, C₁₋₄alkoxy, and halogen; indazole unsubstituted or substituted with C₁₋₄alkyl; C₃₋₆cycloalkyl; benzothiophene; benzofuran; indole unsubstituted or substituted with C₁₋₄alkyl; pyrazole unsubstituted or substituted with C₁₋₄alkyl; imidazole unsubstituted or substituted with C₁₋₄alkyl; triazole; pyrrolidine; piperidine unsubstituted or substituted with C₁₋₄alkyl; piperazine unsubstituted or substituted with C₁₋₄alkyl; pyrrole; furan; morpholine; or thiophene,
X is hydrogen, halogen, C₁₋₄alkyl, C₁₋₄haloalkyl (preferably, -CF₃, -CHF₂), C₃₋₆cycloalkyl, C₁₋₄alkoxy, C₁₋₄haloalkoxy (preferably, -OCF₃), -OH, -CN, -NH₂, or -N(CH₃)₂,
Y is -CH₂-,
B is unsubstituted piperidine, 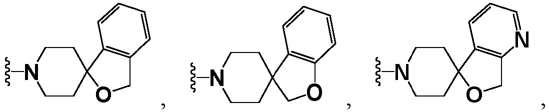 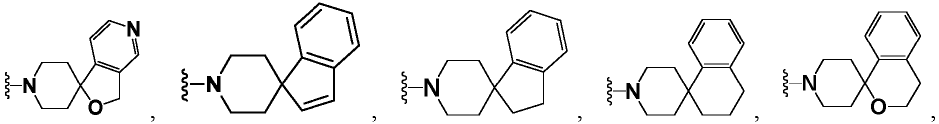 or 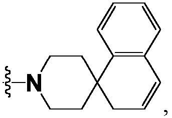
Z is absent (forming a spiro compound) or direct linking,
R₃ is hydrogen; halogen; C₁₋₄alkyl; C₃₋₆cycloalkyl; C₁₋₄haloalkoxy (preferably, -OCF₃); C₁₋₄haloalkyl (preferably, -CF₃, -CHF₂); -OH; phenoxy; phenyl; C₁₋₄alkoxy; -CN; -NH₂; -N(CH₃)₂; phenyl substituted with one or more selected from the group consisting of halogen, C₁₋₄alkyl, C₃₋₆cycloalkyl, C₁₋₄haloalkoxy (preferably, -OCF₃), C₁₋₄haloalkyl (preferably, -CF₃, -CHF₂), -OH, phenoxy, phenyl, C₁₋₄alkoxy, -CN, -NO₂, -NH₂, and -N(CH₃)₂; pyridine unsubstituted or substituted with halogen; naphthalene unsubstituted or substituted with C₁₋₄alkoxy; benzotriazole; quinoline or isoquinoline substituted with one or more selected from the group consisting of C₁₋₄alkyl, C₁₋₄alkoxy, and halogen; indazole unsubstituted or substituted with C₁₋₄alkyl; C₃₋₆ cycloalkyl; benzothiophene; benzofuran; indole unsubstituted or substituted with C₁₋₄alkyl; pyrazole; imidazole; pyrrolidine; piperidine unsubstituted or substituted with C₁₋₄alkyl; piperazine unsubstituted or substituted with C₁₋₄alkyl; pyrrole; furan; thiophene; morpholine or thiophene.

As used herein, the terms "substituent", "radical", "group", "moiety", and "fragment" may be used interchangeably.

If a substituent is described as "optionally substituted", the substituent may be (1) unsubstituted or (2) substituted with one or more of the defined substituents. If the substitutable position is unsubstituted, the default substituent is hydrogen.

As used herein, the term "alkyl" means a saturated straight chain or branched non-cyclic hydrocarbon, unless the context clearly dictates otherwise, having from 1 to 10 carbon atoms. "Lower alkyl" means alkyl having from 1 to 4 carbon atoms. Representative saturated straight chain alkyls include -methyl, -ethyl, -n-propyl, -n-butyl, -n-pentyl, -n-hexyl, -n-heptyl, -n-octyl, - n-nonyl and -n-decyl, while saturated branched alkyls include -isopropyl, - sec-butyl, -isobutyl, - tert-butyl, -isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl and the like.

As used herein, the term "alkoxy" means -O-(alkyl), including -OCH₃, -OCH₂CH₃, - O(CH₂)₂CH₃, -O(CH₂)₃CH₃, -O(CH₂)₄CH₃, -O(CH₂)₅CH₃, and the like, wherein the alkyl is as defined above.

As used herein, if the term "C₁₋₆", "C1-6", or "C1-C6" is used, it means the number of carbon atoms is from 1 to 6. For example, C₁₋₆alkyl means an alkyl which carbon number is any integer of from 1 to 6.

As used herein, the terms "halogen" and "halo" mean fluorine, chlorine, bromine or iodine. In a preferred embodiment of the present invention, the halogen is chlorine or fluorine.

As used herein, the term "haloalkyl" or "haloalkoxy" means an alkyl or alkoxy group, wherein one or more hydrogen atoms are substituted with halogen atoms. For example, the haloalkyl includes -CF₃, -CHF₂, -CH₂F, -CBr₃, -CHBr₂, -CH₂Br, -CC1₃, -CHC1₂, -CH₂CI, -CI₃, - CHI₂, -CH₂I, -CH₂-CF₃, -CH₂-CHF₂, -CH₂-CH₂F, -CH₂-CBr₃, -CH₂-CHBr₂, -CH₂-CH₂Br, -CH₂-CC1₃, -CH₂-CHC1₂, -CH₂-CH₂CI, -CH₂-CI₃, -CH₂-CHI₂, -CH₂-CH₂I, and the like, wherein alkyl, alkoxy and halogen are as described above. In a preferred embodiment of the present invention, haloalkyl is -CF₃.

As used herein, the term "cycloalkyl" means a monocyclic or polycyclic saturated ring having carbon and hydrogen atoms and having no carbon-carbon multiple bonds. Examples of monocyclic rings include, but are not limited to, (C₃-C₇)cycloalkyl groups, including cyclopropyl, cyclobutyl, cyclopentyl, cyclohexyl, and cycloheptyl. Examples of polycyclic rings include, but are not limited to, fused bicyclic rings such as octahydropentalene and decahydronaphthalene; spiro rings such as spiro[3.3]heptane, spiro[3.4]octane, spiro[3.5]nonane, spiro[4.4]nonane, spiro[4.5]decane, and spiro[5.5]undecane; and bridged bicycle rings such as bicyclo[2.1.1]hexane, bicyclo[2.2.1]heptane, and bicyclo[2.2.2]octane. A cycloalkyl group can be unsubstituted or optionally substituted. In an embodiment of the present invention, cycloalkyl is monocyclic ring.

The term "heterocycle" or "heterocycloalkyl" means a 5- to 7-membered monocyclic, or 7- to 12-membered bicyclic, saturated heterocyclic ring which contains from 1 to 4 heteroatoms independently selected from nitrogen, oxygen and sulfur, and wherein the nitrogen and sulfur heteroatoms can be optionally oxidized, and the nitrogen heteroatom can be optionally quaternized. Representative heterocycles include oxiran, oxetan, tetrahydrofuran, tetrahydropyran, 1,4-dioxane, aziridine, azetidine, pyrrolidine, piperidine, piperazine, pyrrolidinone, hydantoine, valerolactam, thiirane, thietane, tetrahydrothiophene, tetrahydrothiopyran, morpholine, tetrahydropyridine, and tetrahydropyrimidine. Heterocycles include a bicyclic ring in which part of the heterocycle is fused to a benzene or cyclopenta-1,3-diene ring. The heterocycle can be attached via any heteroatom or carbon atom. In addition, heterocycles include fused bicyclic rings, spiro rings and bridged bicyclic rings in which one or more carbon atoms of the aforementioned polycyclic rings are replaced with nitrogen, oxygen or sulfur atoms.

As used herein, the term "aryl" means a carbocyclic aromatic group containing from 5 to 10 atoms. Representative examples include, but are not limited to, phenyl, tolyl, xylyl, naphthyl, tetrahydronaphthyl, anthracenyl, fluorenyl, indenyl, and azulenyl. A carbocyclic aromatic group can be unsubstituted or optionally substituted.

As used herein, the term "heteroaryl" means an aromatic heterocycle ring of 5- to 10 members and having at least one heteroatom selected from nitrogen, oxygen and sulfur, and containing at least 1 carbon atom, including both mono- and bicyclic ring systems. Representative heteroaryls are furan, 4H-pyran, pyrrole, imidazole, pyrazole, triazole, tetrazole, pyridine, pyrimidine, pyridazine, pyrazine, triazine, thiophene, ozaxole, isoxazole, thiazole, isothiazole, oxadiazole, benzofuran, benzothiophene, quinoline, indole, benzoxazole, benzimidazole, benzothiazole, cinnoline, phthalazine, quinazoline, 1H-azepine, etc.

In this specification, * or 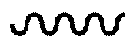 means connected to another moiety.

Non-limiting examples of compounds of the chemical formula 1 according to the present disclosure are those in Table 1 below.

**[Table 1]**

| cpd# | IUPAC NAME |
|---|---|
| 1 | 2-(6-chlorobenzo[d][1,3]dioxol-5-yl)-5-methyl-4-(4-phenoxybenzyl)oxazole |
| 2 | 4-(4-(4-fluorophenoxy)benzyl)-2-(4-methoxyphenyl)-5-methyloxazole |
| 3 | 4-([1,1'-biphenyl]-3-ylmethyl)-2-(4-methoxyphenyl)-5-methyloxazole |
| 4 | 2-(4-chlorophenyl)-5-methyl-4-(4-phenoxybenzyl)oxazole |
| 5 | 5-methyl-4-(4-phenoxybenzyl)-2-(4-(trifluoromethyl)phenyl)oxazole |
| 6 | 2-(4-methoxyphenyl)-5-methyl-4-(4-phenoxybenzyl)oxazole |
| 7 | 5-methyl-4-(4-phenoxybenzyl)-2-(4-(trifluoromethoxy)phenyl)oxazole |
| 8 | 4-(5-methyl-4-(4-phenoxybenzyl)oxazol-2-yl)benzonitrile |
| 9 | 2-(4-methoxyphenyl)-4-(4-phenoxybenzyl)-5-phenyloxazole |
| 10 | N-(4-(5-methyl-4-(4-phenoxybenzyl)oxazol-2-yl)phenyl)acetamide |
| 11 | methyl 4-(5-methyl-4-(4-phenoxybenzyl)oxazol-2-yl)benzoate |
| 12 | N-methyl-4-(5-methyl-4-(4-phenoxybenzyl)oxazol-2-yl)aniline |
| 13 | N,N-dimethyl-4-(5-methyl-4-(4-phenoxybenzyl)oxazol-2-yl)aniline |
| 14 | 5-methyl-4-(4-phenoxybenzyl)-2-(p-tolyl)oxazole |
| 15 | 5-methyl-2-(naphthalen-2-yl)-4-(4-phenoxybenzyl)oxazole |
| 16 | 5-methyl-4-(4-phenoxybenzyl)-2-(quinolin-6-yl)oxazole |
| 17 | 4-(4-(4-(4-fluorophenoxy)benzyl)-5-methyloxazol-2-yl)benzonitrile |
| 18 | 5-methyl-4-(4-phenoxybenzyl)-2-(quinolin-7-yl)oxazole |
| 19 | 2-(4-chlorophenyl)-4-(4-(4-fluorophenoxy)benzyl)-5-methyloxazole |
| 20 | 4-(4-(4-fluorophenoxy)benzyl)-5-methyl-2-(4-(trifluoromethyl)phenyl)oxazole |
| 21 | 4-(4-(4-fluorophenoxy)benzyl)-2-(4-fluorophenyl)-5-methyloxazole |
| 22 | 4-(4-(4-(4-fluorophenoxy)benzyl)-5-methyloxazol-2-yl)phenol |
| 23 | N-(4-(5-methyl-4-(4-phenoxybenzyl)oxazol-2-yl)phenyl)isobutyramide |
| 24 | N-(4-(5-methyl-4-(4-phenoxybenzyl)oxazol-2-yl)phenyl)benzamide |
| 25 | 4-(4-(4-fluorophenoxy)benzyl)-5-methyl-2-(4-nitrophenyl)oxazole |
| 26 | 2-(4-chlorophenyl)-4-(4-phenoxybenzyl)-5-phenyloxazole |
| 27 | 4-(4-phenoxybenzyl)-5-phenyl-2-(4-(trifluoromethyl)phenyl)oxazole |
| 28 | 4-(5-methyl-4-(4-phenoxybenzyl)oxazol-2-yl)benzoic acid |
| 29 | 2-(4-fluorophenyl)-5-methyl-4-(4-phenoxybenzyl)oxazole |
| 30 | 2-(4-(1H-pyrazol-1-yl)phenyl)-5-methyl-4-(4-phenoxybenzyl)oxazole |
| 31 | 2-(2-chloro-4-fluorophenyl)-5-methyl-4-(4-phenoxybenzyl)oxazole |
| 32 | 4-((5-methyl-2-(4-(trifluoromethyl)phenyl)oxazol-4-yl)methyl)-N-phenylaniline |
| 33 | 4-((5-methyl-2-(4-(trifluoromethyl)phenyl)oxazol-4-yl)methyl)-N-(4-(trifluoromethoxy)phenyl)aniline |
| 34 | N-(4-(5-methyl-4-(4-phenoxybenzyl)oxazol-2-yl)phenyl)propionamide |
| 35 | 2-(2,4-dichlorophenyl)-5-methyl-4-(4-phenoxybenzyl)oxazole |
| 36 | 2-(4-(difluoromethoxy)phenyl)-5-methyl-4-(4-phenoxybenzyl)oxazole |
| 37 | N-methyl-4-((5-methyl-2-(4-(trifluoromethyl)phenyl)oxazol-4-yl)methyl)-N-(4-(trifluoromethoxy)phenyl)aniline |
| 38 | 5-methyl-4-(4-(p-tolyloxy)benzyl)-2-(4-(trifluoromethyl)phenyl)oxazole |
| 39 | 4-([1,1'-biphenyl]-3-ylmethyl)-5-isopropyl-2-(4-(trifluoromethyl)phenyl)oxazole |
| 40 | N-methyl-4-(5-methyl-4-(4-phenoxybenzyl)oxazol-2-yl)benzamide |
| 41 | N,N-dimethyl-4-(5-methyl-4-(4-phenoxybenzyl)oxazol-2-yl)benzamide |
| 42 | 2-(4-chlorophenyl)-4-(4-phenoxybenzyl)oxazole |
| 43 | 2-(4-(1H-pyrazol-1-yl)phenyl)-4-(4-(4-fluorophenoxy)benzyl)-5-methyloxazole |
| 44 | 4-(4-(4-(4-fluorophenoxy)benzyl)-5-methyloxazol-2-yl)-N,N-dimethylaniline |
| 45 | 4-(4-(4-fluorophenoxy)benzyl)-5-methyl-2-(quinolin-6-yl)oxazole |
| 46 | 4-(4-(4-(4-(4-fluorophenoxy)benzyl)-5-methyloxazol-2-yl)phenyl)morpholine |
| 47 | 4-(4-(4-fluorophenoxy)benzyl)-5-methyl-2-(4-(4-methylpiperidin-1-yl)phenyl)oxazole |
| 49 | 2-(4-(1H-pyrazol-1-yl)phenyl)-5-methyl-4-(4-(4-(trifluoromethoxy)phenoxy)benzyl)oxazole |
| 51 | 4-((2-(4-(1H-pyrazol-1-yl)phenyl)-5-methyloxazol-4-yl)methyl)-N-(4-fluorophenyl)aniline |
| 52 | 4-((2-(4-(1H-pyrazol-1-yl)phenyl)-5-methyloxazol-4-yl)methyl)-N-(4-(trifluoromethoxy)phenyl)aniline |
| 53 | 4-(4-(4-fluorophenoxy)benzyl)-5-methyl-2-(4-(pyridin-3-yl)phenyl)oxazole |
| 54 | 4-(4-(4-fluorophenoxy)benzyl)-5-methyl-2-(4-(pyridin-4-yl)phenyl)oxazole |
| 55 | 5-methyl-2-(4-(pyridin-3-yl)phenyl)-4-(4-(4-(trifluoromethoxy)phenoxy)benzyl)oxazole |
| 56 | 5-methyl-2-(4-(pyridin-4-yl)phenyl)-4-(4-(4-(trifluoromethoxy)phenoxy)benzyl)oxazole |
| 57 | 4-(4-(2-fluorophenoxy)benzyl)-5-methyl-2-(4-(pyridin-3-yl)phenyl)oxazole |
| 58 | 4-(4-(3-fluorophenoxy)benzyl)-5-methyl-2-(4-(pyridin-3-yl)phenyl)oxazole |
| 59 | 4-(3-(4-fluorophenoxy)benzyl)-5-methyl-2-(4-(pyridin-3-yl)phenyl)oxazole |
| 60 | 2-(4-(4-ethylpiperazin-1-yl)phenyl)-4-(4-(4-fluorophenoxy)benzyl)-5-methyloxazole |
| 61 | 4-(4-(3-fluorophenoxy)benzyl)-5-methyl-2-(4-(pyridin-4-yl)phenyl)oxazole |
| 62 | 2-(4-(4-ethylpiperazin-1-yl)phenyl)-4-(4-(2-fluorophenoxy)benzyl)-5-methyloxazole |
| 63 | 2-(4-(4-ethylpiperazin-1-yl)phenyl)-4-(4-(3-fluorophenoxy)benzyl)-5-methyloxazole |
| 64 | 2-(4-(4-ethylpiperazin-1-yl)phenyl)-4-(3-(4-fluorophenoxy)benzyl)-5-methyloxazole |
| 65 | 4-(4-(4-fluorophenoxy)benzyl)-5-methyl-2-(2-methyl-4-(pyridin-4-yl)phenyl)oxazole |
| 66 | 2-(3-fluoro-4-(pyridin-4-yl)phenyl)-4-(4-(4-fluorophenoxy)benzyl)-5-methyloxazole |
| 67 | 4-((4-(4-fluorophenyl)piperidin-1-yl)methyl)-5-methyl-2-(4-(pyridin-4-yl)phenyl)oxazole |
| 68 | 4-(4-fluorophenyl)-1-((5-methyl-2-(4-(pyridin-4-yl)phenyl)oxazol-4-yl)methyl)piperidin-4-ol |
| 69 | 5-methyl-2-(4-(pyridin-4-yl)phenyl)-4-((4-(4-(trifluoromethoxy)phenoxy)piperidin-1-yl)methyl)oxazole |
| 70 | 5-methyl-2-(4-(pyridin-4-yl)phenyl)-4-((4-(4-(trifluoromethoxy)phenyl)piperidin-1-yl)methyl)oxazole |
| 71 | 2-(4-(1H-imidazol-1-yl)phenyl)-4-(4-(4-fluorophenoxy)benzyl)-5-methyloxazole |
| 72 | 2-(4-(2H-1,2,3-triazol-2-yl)phenyl)-4-((4-(4-fluorophenyl)piperidin-1-yl)methyl)-5-methyloxazole |
| 73 | 2-(4-(2H-1,2,3-triazol-2-yl)phenyl)-5-methyl-4-((4-(4-(trifluoromethoxy)phenoxy)piperidin-1-yl)methyl)oxazole |
| 74 | 2-(4-(2H-1,2,3-triazol-2-yl)phenyl)-5-methyl-4-((4-(4-(trifluoromethoxy)phenoxy)piperidin-1-yl)methyl)oxazole |
| 75 | 2-(4-(1H-imidazol-1-yl)phenyl)-5-methyl-4-((4-(4-(trifluoromethoxy)phenoxy)piperidin-1-yl)methyl)oxazole |
| 76 | 4-(4-((4-fluorobenzyl)oxy)benzyl)-5-methyl-2-(4-(pyridin-4-yl)phenyl)oxazole |
| 77 | 5-methyl-2-(4-(pyridin-4-yl)phenyl)-4-(4-((4-(trifluoromethyl)benzyl)oxy)benzyl)oxazole |
| 78 | 5-methyl-2-(4-(pyridin-4-yl)phenyl)-4-(4-((4-(trifluoromethoxy)benzyl)oxy)benzyl)oxazole |
| 79 | 2-(4-(1H-1,2,3-triazol-1-yl)phenyl)-4-(4-(4-fluorophenoxy)benzyl)-5-methyloxazole |
| 80 | 2-(4-(2H-1,2,3-triazol-2-yl)phenyl)-5-methyl-4-((4-(4-(trifluoromethoxy)phenyl)piperidin-1-yl)methyl)oxazole |
| 81 | 2-(benzo[d][1,3]dioxol-5-yl)-5-methyl-4-((4-(4-(trifluoromethoxy)phenyl)piperidin-1-yl)methyl)oxazole |
| 82 | 2-(benzo[d][1,3]dioxol-5-yl)-4-(4-(4-fluorophenoxy)benzyl)-5-methyloxazole |
| 83 | 2-(4-(1H-pyrazol-1-yl)phenyl)-5-methyl-4-((4-(4-(trifluoromethoxy)phenyl)piperidin-1-yl)methyl)oxazole |
| 84 | 5-methyl-2-(4-(pyridin-4-yl)phenyl)-4-((4-(4-(trifluoromethoxy)phenyl)piperazin-1-yl)methyl)oxazole |
| 85 | 2-(4-(1H-pyrazol-1-yl)phenyl)-5-methyl-4-((4-(4-(trifluoromethoxy)phenyl)piperazin-1-yl)methyl)oxazole |
| 86 | 2-(benzo[d][1,3]dioxol-5-yl)-5-methyl-4-((4-(4-(trifluoromethoxy)phenyl)piperazin-1-yl)methyl)oxazole |
| 87 | 1'-((2-(4-(1H-pyrazol-1-yl)phenyl)-5-methyloxazol-4-yl)methyl)-3H-spiro[isobenzofuran-1,4'-piperidine] |
| 88 | 2-(4-(1H-imidazol-1-yl)phenyl)-5-methyl-4-((4-(4-(trifluoromethoxy)phenyl)piperazin-1-yl)methyl)oxazole |
| 89 | 1'-((2-(4-(1H-imidazol-1-yl)phenyl)-5-methyloxazol-4-yl)methyl)-3H-spiro[isobenzofuran-1,4'-piperidine] |
| 90 | 2-(4-(1H-imidazol-1-yl)phenyl)-5-methyl-4-(spirofindene-1,4'-piperidin]-1'-ylmethyl)oxazole |
| 91 | 2-(4-(1H-pyrazol-1-yl)phenyl)-5-methyl-4-(spiro[indene-1,4'-piperidin]-1'-ylmethyl)oxazole |
| 92 | 2-(benzo[d][1,3]dioxol-5-yl)-4-((2,3-dihydrospiro[indene-1,4'-piperidin]-1'-yl)methyl)-5-methyloxazole |
| 93 | 1'-((2-(benzo[d][1,3]dioxol-5-yl)-5-methyloxazol-4-yl)methyl)-3H-spiro[isobenzofuran-1,4'-piperidine] |
| 94 | 2-(4-(1H-imidazol-1-yl)phenyl)-5-methyl-4-((4-(4-(trifluoromethoxy)phenyl)piperidin-1-yl)methyl)oxazole |
| 95 | 2-(4-(1H-pyrazol-1-yl)phenyl)-4-((2,3-dihydrospirofindene-1,4'-piperidin]-1'-yl)methyl)-5-methyloxazole |
| 96 | 2-(4-(1H-imidazol-1-yl)phenyl)-4-((2,3-dihydrospiro[indene-1,4'-piperidin]-1'-yl)methyl)-5-methyloxazole |
| 97 | 2-(4-(1H-pyrazol-1-yl)phenyl)-5-methyl-4-((4-(3-(trifluoromethoxy)phenyl)piperidin-1-yl)methyl)oxazole |
| 98 | 2-(4-(1H-imidazol-1-yl)phenyl)-5-methyl-4-((4-(3-(trifluoromethoxy)phenyl)piperidin-1-yl)methyl)oxazole |
| 99 | 2-(4-(1H-pyrazol-1-yl)phenyl)-5-methyl-4-((4-(2-(trifluoromethoxy)phenyl)piperidin-1-yl)methyl)oxazole |
| 100 | 2-(4-(1H-imidazol-1-yl)phenyl)-5-methyl-4-((4-(2-(trifluoromethoxy)phenyl)piperidin-1-yl)methyl)oxazole |
| 101 | 2-(4-(1H-pyrazol-1-yl)phenyl)-5-methyl-4-((4-(2-(trifluoromethoxy)phenyl)-3,6-dihydropyridin-1(2H)-yl)methyl)oxazole |
| 102 | 2-(4-(1H-pyrazol-1-yl)phenyl)-5-methyl-4-((4-(3-(trifluoromethoxy)phenyl)-3,6-dihydropyridin-1 (2H)-yl)methyl)oxazole |
| 103 | 2-(4-(1H-pyrazol-1-yl)phenyl)-5-methyl-4-((4-(4-(trifluoromethoxy)phenyl)-3,6-dihydropyridin-1 (2H)-yl)methyl)oxazole |
| 104 | 2-(4-(1H-imidazol-1-yl)phenyl)-5-methyl-4-((4-(2-(trifluoromethoxy)phenyl)-3,6-dihydropyridin-1(2H)- |
| | yl)methyl)oxazole |
| 105 | 2-(4-(1H-imidazol-1-yl)phenyl)-5-methyl-4-((4-(3-(trifluoromethoxy)phenyl)-3,6-dihydropyridin-1(2H)-yl)methyl)oxazole |
| 106 | 2-(4-(1H-imidazol-1-yl)phenyl)-5-methyl-4-((4-(4-(trifluoromethoxy)phenyl)-3,6-dihydropyridin-1(2H)-yl)methyl)oxazole |
| 107 | 5-methyl-2-(4-(pyridin-4-yl)phenyl)-4-(spiro[indene-1,4'-piperidin]-1'-ylmethyl)oxazole |
| 108 | 1'-((5-methyl-2-(4-(pyridin-4-yl)phenyl)oxazol-4-yl)methyl)-3H-spiro[isobenzofuran-1,4'-piperidine] |
| 109 | 2-(4-(1H-pyrazol-1-yl)phenyl)-5-methyl-4-((4-phenylpiperidin-1-yl)methyl)oxazole |
| 110 | 2-(4-(1H-pyrazol-1-yl)phenyl)-5-methyl-4-((4-(4-nitrophenyl)piperidin-1-yl)methyl)oxazole |
| 111 | 2-(4-(1H-imidazol-1-yl)phenyl)-5-methyl-4-((4-phenylpiperidin-1-yl)methyl)oxazole |
| 112 | 2-(4-(1H-imidazol-1-yl)phenyl)-5-methyl-4-((4-(4-nitrophenyl)piperidin-1-yl)methyl)oxazole |
| 113 | 4-((2,3-dihydrospiro[indene-1,4'-piperidin]-1'-yl)methyl)-5-methyl-2-(4-(pyridin-4-yl)phenyl)oxazole |
| 114 | 4-(4-(4-fluorophenoxy)benzyl)-5-methyl-2-(4-(2-methyl-1H-imidazol-1-yl)phenyl)oxazole |
| 115 | 5-methyl-2-(4-(2-methyl-1H-imidazol-1-yl)phenyl)-4-((4-(4-(trifluoromethoxy)phenyl)piperidin-1-yl)methyl)oxazole |
| 116 | 5-methyl-2-(4-(2-methyl-1H-imidazol-1-yl)phenyl)-4-(spiro[indene-1,4'-piperidin]-1'-ylmethyl)oxazole |
| 117 | 2-(4-(1H-pyrazol-1-yl)phenyl)-4-((4-(4-chlorophenyl)piperidin-1-yl)methyl)-5-methyloxazole |
| 118 | 2-(4-(1H-pyrazol-1-yl)phenyl)-4-((4,4-diphenylpiperidin-1-yl)methyl)-5-methyloxazole |
| 119 | 2-(4-(1H-pyrazol-1-yl)phenyl)-4-((4-(4-methoxy-2-methylphenyl)piperidin-1-yl)methyl)-5-methyloxazole |
| 120 | 2-(4-(1H-pyrazol-1-yl)phenyl)-4-((4-(2,4-dimethoxyphenyl)piperidin-1-yl)methyl)-5-methyloxazole |
| 121 | 2-(4-(1H-imidazol-1-yl)phenyl)-4-((4-(4-chlorophenyl)piperidin-1-yl)methyl)-5-methyloxazole |
| 122 | 5-methyl-2-(4-(4-methyl-1H-imidazol-1-yl)phenyl)-4-((4-(4-(trifluoromethoxy)phenyl)piperidin-1-yl)methyl)oxazole |
| 123 | 2-(2,3-dihydrobenzofuran-7-yl)-4-((2,3-dihydrospiro[indene-1,4'-piperidin]-1'-yl)methyl)-5-methyloxazole |
| 124 | 2-(2,3-dihydrobenzofuran-7-yl)-5-methyl-4-((4-(4-(trifluoromethoxy)phenyl)piperidin-1-yl)methyl)oxazole |
| 125 | 2-(2,3-dihydrobenzo[b]1,4]dioxin-5-yl)-5-methyl-4-(spiro[indene-1,4'-piperidin]-1'-ylmethyl)oxazole |
| 126 | 2-(2,3-dihydrobenzo[b][1,4]dioxin-5-yl)-5-methyl-4-((4-(4-(trifluoromethoxy)phenyl)piperidin-1-yl)methyl)oxazole |
| 127 | (4-(5-methyl-4-(spiro[indene-1,4'-piperidin]-1'-ylmethyl)oxazol-2-yl)phenyl)morpholine |
| 128 | 4-(4-(5-methyl-4-((4-(4-(trifluoromethoxy)phenyl)piperidin-1-yl)methyl)oxazol-2-yl)phenyl)morpholine |
| 129 | 4-(4-(4-((2,3-dihydrospiro[indene-1,4'-piperidin]-1'-yl)methyl)-5-methyloxazol-2-yl)phenyl)morpholine |
| 130 | 2-(imidazo[1,2-a]pyridin-6-yl)-5-methyl-4-(spiro[indene-1,4'-piperidin]-1'-ylmethyl)oxazole |
| 131 | 2-(imidazo[1,2-a]pyridin-6-yl)-5-methyl-4-((4-(4-(trifluoromethoxy)phenyl)piperidin-1-yl)methyl)oxazole |
| 132 | 1'-((5-methyl-2-(4-morpholinophenyl)oxazol-4-yl)methyl)-3H-spiro[isobenzofuran-1,4'-piperidine] |
| 133 | 2-(4-(1H-pyrazol-1-yl)phenyl)-4-((4-(4-(difluoromethoxy)phenyl)piperidin-1-yl)methyl)-5-methyloxazole |
| 134 | 2-(4-(1H-imidazol-1-yl)phenyl)-4-((4-(4-(difluoromethoxy)phenyl)piperidin-1-yl)methyl)-5-methyloxazole |

Among these compounds, the anti-tuberculosis activity of the compounds in Table 2 below is particularly excellent. Accordingly, the present disclosure preferably provides an antibacterial pharmaceutical composition comprising a compound of Table 2 below or a pharmaceutically acceptable salt thereof as an active ingredient.

**[Table 2]**

| cpd# | IUPAC NAME |
|---|---|
| 2 | 4-(4-(4-fluorophenoxy)benzyl)-2-(4-methoxyphenyl)-5-methyloxazole |
| 6 | 2-(4-methoxyphenyl)-5-methyl-4-(4-phenoxybenzyl)oxazole |
| 13 | N,N-dimethyl-4-(5-methyl-4-(4-phenoxybenzyl)oxazol-2-yl)aniline |
| 16 | 5-methyl-4-(4-phenoxybenzyl)-2-(quinolin-6-yl)oxazole |
| 17 | 4-(4-(4-(4-fluorophenoxy)benzyl)-5-methyloxazol-2-yl)benzonitrile |
| 18 | 5-methyl-4-(4-phenoxybenzyl)-2-(quinolin-7-yl)oxazole |
| 20 | 4-(4-(4-fluorophenoxy)benzyl)-5-methyl-2-(4-(trifluoromethyl)phenyl)oxazole |
| 30 | 2-(4-(1H-pyrazol-1-yl)phenyl)-5-methyl-4-(4-phenoxybenzyl)oxazole |
| 33 | 4-((5-methyl-2-(4-(trifluoromethyl)phenyl)oxazol-4-yl)methyl)-N-(4-(trifluoromethoxy)phenyl)aniline |
| 43 | 2-(4-(1H-pyrazol-1-yl)phenyl)-4-(4-(4-fluorophenoxy)benzyl)-5-methyloxazole |
| 44 | 4-(4-(4-(4-fluorophenoxy)benzyl)-5-methyloxazol-2-yl)-N,N-dimethylaniline |
| 45 | 4-(4-(4-fluorophenoxy)benzyl)-5-methyl-2-(quinolin-6-yl)oxazole |
| 46 | 4-(4-(4-(4-(4-fluorophenoxy)benzyl)-5-methyloxazol-2-yl)phenyl)morpholine |
| 49 | 2-(4-(1H-pyrazol-1-yl)phenyl)-5-methyl-4-(4-(4-(trifluoromethoxy)phenoxy)benzyl)oxazole |
| 51 | 4-((2-(4-(1H-pyrazol-1-yl)phenyl)-5-methyloxazol-4-yl)methyl)-N-(4-fluorophenyl)aniline |
| 53 | 4-(4-(4-fluorophenoxy)benzyl)-5-methyl-2-(4-(pyridin-3-yl)phenyl)oxazole |
| 54 | 4-(4-(4-fluorophenoxy)benzyl)-5-methyl-2-(4-(pyridin-4-yl)phenyl)oxazole |
| 55 | 5-methyl-2-(4-(pyridin-3-yl)phenyl)-4-(4-(4-(trifluoromethoxy)phenoxy)benzyl)oxazole |
| 56 | 5-methyl-2-(4-(pyridin-4-yl)phenyl)-4-(4-(4-(trifluoromethoxy)phenoxy)benzyl)oxazole |
| 58 | 4-(4-(3-fluorophenoxy)benzyl)-5-methyl-2-(4-(pyridin-3-yl)phenyl)oxazole |
| 59 | 4-(3-(4-fluorophenoxy)benzyl)-5-methyl-2-(4-(pyridin-3-yl)phenyl)oxazole |
| 61 | 4-(4-(3-fluorophenoxy)benzyl)-5-methyl-2-(4-(pyridin-4-yl)phenyl)oxazole |
| 65 | 4-(4-(4-fluorophenoxy)benzyl)-5-methyl-2-(2-methyl-4-(pyridin-4-yl)phenyl)oxazole |
| 66 | 2-(3-fluoro-4-(pyridin-4-yl)phenyl)-4-(4-(4-fluorophenoxy)benzyl)-5-methyloxazole |
| 67 | 4-((4-(4-fluorophenyl)piperidin-1-yl)methyl)-5-methyl-2-(4-(pyridin-4-yl)phenyl)oxazole |
| 69 | 5-methyl-2-(4-(pyridin-4-yl)phenyl)-4-((4-(4-(trifluoromethoxy)phenoxy)piperidin-1-yl)methyl)oxazole |
| 70 | 5-methyl-2-(4-(pyridin-4-yl)phenyl)-4-((4-(4-(trifluoromethoxy)phenyl)piperidin-1-yl)methyl)oxazole |
| 71 | 2-(4-(1H-imidazol-1-yl)phenyl)-4-(4-(4-fluorophenoxy)benzyl)-5-methyloxazole |
| 73 | 2-(4-(2H-1,2,3-triazol-2-yl)phenyl)-5-methyl-4-((4-(4-(trifluoromethoxy)phenoxy)piperidin-1-yl)methyl)oxazole |
| 74 | 2-(4-(2H-1,2,3-triazol-2-yl)phenyl)-5-methyl-4-((4-(4-(trifluoromethoxy)phenoxy)piperidin-1-yl)methyl)oxazole |
| 75 | 2-(4-(1H-imidazol-1-yl)phenyl)-5-methyl-4-((4-(4-(trifluoromethoxy)phenoxy)piperidin-1-yl)methyl)oxazole |
| 76 | 4-(4-((4-fluorobenzyl)oxy)benzyl)-5-methyl-2-(4-(pyridin-4-yl)phenyl)oxazole |
| 77 | 5-methyl-2-(4-(pyridin-4-yl)phenyl)-4-(4-((4-(trifluoromethyl)benzyl)oxy)benzyl)oxazole |
| 78 | 5-methyl-2-(4-(pyridin-4-yl)phenyl)-4-(4-((4-(trifluoromethoxy)benzyl)oxy)benzyl)oxazole |
| 79 | 2-(4-(1H-1,2,3-triazol-1-yl)phenyl)-4-(4-(4-fluorophenoxy)benzyl)-5-methyloxazole |
| 80 | 2-(4-(2H-1,2,3-triazol-2-yl)phenyl)-5-methyl-4-((4-(4-(trifluoromethoxy)phenyl)piperidin-1-yl)methyl)oxazole |
| 81 | 2-(benzo[d][1,3]dioxol-5-yl)-5-methyl-4-((4-(4-(trifluoromethoxy)phenyl)piperidin-1-yl)methyl)oxazole |
| 82 | 2-(benzo[d][1,3]dioxol-5-yl)-4-(4-(4-fluorophenoxy)benzyl)-5-methyloxazole |
| 83 | 2-(4-(1H-pyrazol-1-yl)phenyl)-5-methyl-4-((4-(4-(trifluoromethoxy)phenyl)piperidin-1-yl)methyl)oxazole |
| 84 | 5-methyl-2-(4-(pyridin-4-yl)phenyl)-4-((4-(4-(trifluoromethoxy)phenyl)piperazin-1-yl)methyl)oxazole |
| 85 | 2-(4-(1H-pyrazol-1-yl)phenyl)-5-methyl-4-((4-(4-(trifluoromethoxy)phenyl)piperazin-1-yl)methyl)oxazole |
| 86 | 2-(benzo[d][1,3]dioxol-5-yl)-5-methyl-4-((4-(4-(trifluoromethoxy)phenyl)piperazin-1-yl)methyl)oxazole |
| 87 | 1'-((2-(4-(1H-pyrazol-1-yl)phenyl)-5-methyloxazol-4-yl)methyl)-3H-spiro[isobenzofuran-1,4'-piperidine] |
| 88 | 2-(4-(1H-imidazol-1-yl)phenyl)-5-methyl-4-((4-(4-(trifluoromethoxy)phenyl)piperazin-1-yl)methyl)oxazole |
| 89 | 1'-((2-(4-(1H-imidazol-1-yl)phenyl)-5-methyloxazol-4-yl)methyl)-3H-spiro[isobenzofuran-1,4'-piperidine] |
| 90 | 2-(4-(1H-imidazol-1-yl)phenyl)-5-methyl-4-(spirofindene-1,4'-piperidin]-1'-ylmethyl)oxazole |
| 91 | 2-(4-(1H-pyrazol-1-yl)phenyl)-5-methyl-4-(spiro[indene-1,4'-piperidin]-1'-yimethyl)oxazole |
| 92 | 2-(benzo[d][1,3]dioxol-5-yl)-4-((2,3-dihydrospiro[indene-1,4'-piperidin]-1'-yl)methyl)-5-methyloxazole |
| 93 | 1'-((2-(benzo[d][1,3]dioxol-5-yl)-5-methyloxazol-4-yl)methyl)-3H-spiro[isobenzofuran-1,4'-piperidine] |
| 94 | 2-(4-(1H-imidazol-1-yl)phenyl)-5-methyl-4-((4-(4-(trifluoromethoxy)phenyl)piperidin-1-yl)methyl)oxazole |
| 95 | 2-(4-(1H-pyrazol-1-yl)phenyl)-4-((2,3-dihydrospirofindene-1,4'-piperidin]-1'-yl)methyl)-5-methyloxazole |
| 96 | 2-(4-(1H-imidazol-1-yl)phenyl)-4-((2,3-dihydrospiro[indene-1,4'-piperidin]-1'-yl)methyl)-5-methyloxazole |
| 98 | 2-(4-(1H-imidazol-1-yl)phenyl)-5-methyl-4-((4-(3-(trifluoromethoxy)phenyl)piperidin-1-yl)methyl)oxazole |
| 99 | 2-(4-(1H-pyrazol-1-yl)phenyl)-5-methyl-4-((4-(2-(trifluoromethoxy)phenyl)piperidin-1-yl)methyl)oxazole |
| 100 | 2-(4-(1H-imidazol-1-yl)phenyl)-5-methyl-4-((4-(2-(trifluoromethoxy)phenyl)piperidin-1-yl)methyl)oxazole |
| 101 | 2-(4-(1H-pyrazol-1-yl)phenyl)-5-methyl-4-((4-(2-(trifluoromethoxy)phenyl)-3,6-dihydropyridin-1(2H)-yl)methyl)oxazole |
| 103 | 2-(4-(1 H-pyrazol-1-yl)phenyl)-5-methyl-4-((4-(4-(trifluoromethoxy)phenyl)-3,6-dihydropyridin-1(2H)-yl)methyl)oxazole |
| 106 | 2-(4-(1 H-imidazol-1-yl)phenyl)-5-methyl-4-((4-(4-(trifluoromethoxy)phenyl)-3,6-dihydropyridin-1 (2H)-yl)methyl)oxazole |
| 107 | 5-methyl-2-(4-(pyridin-4-yl)phenyl)-4-(spirofindene-1,4'-piperidin]-1'-ylmethyl)oxazole |
| 108 | 1'-((5-methyl-2-(4-(pyridin-4-yl)phenyl)oxazol-4-yl)methyl)-3H-spiro[isobenzofuran-1,4'-piperidine] |
| 109 | 2-(4-(1H-pyrazol-1-yl)phenyl)-5-methyl-4-((4-phenylpiperidin-1-yl)methyl)oxazole |
| 112 | 2-(4-(1H-imidazol-1-yl)phenyl)-5-methyl-4-((4-(4-nitrophenyl)piperidin-1-yl)methyl)oxazole |
| 113 | 4-((2,3-dihydrospiro[indene-1,4'-piperidin]-1'-yl)methyl)-5-methyl-2-(4-(pyridin-4-yl)phenyl)oxazole |
| 114 | 4-(4-(4-fluorophenoxy)benzyl)-5-methyl-2-(4-(2-methyl-1H-imidazol-1-yl)phenyl)oxazole |
| 115 | 5-methyl-2-(4-(2-methyl-1H-imidazol-1-yl)phenyl)-4-((4-(4-(trifluoromethoxy)phenyl)piperidin-1-yl)methyl)oxazole |
| 116 | 5-methyl-2-(4-(2-methyl-1H-imidazol-1-yl)phenyl)-4-(spiro[indene-1,4'-piperidin]-1'-ylmethyl)oxazole |
| 117 | 2-(4-(1H-pyrazol-1-yl)phenyl)-4-((4-(4-chlorophenyl)piperidin-1-yl)methyl)-5-methyloxazole |
| 121 | 2-(4-(1H-imidazol-1-yl)phenyl)-4-((4-(4-chlorophenyl)piperidin-1-yl)methyl)-5-methyloxazole |
| 122 | 5-methyl-2-(4-(4-methyl-1H-imidazol-1-yl)phenyl)-4-((4-(4-(trifluoromethoxy)phenyl)piperidin-1-yl)methyl)oxazole |
| 127 | (4-(5-methyl-4-(spiro[indene-1,4'-piperidin]-1'-ylmethyl)oxazol-2-yl)phenyl)morpholine |
| 128 | 4-(4-(5-methyl-4-((4-(4-(trifluoromethoxy)phenyl)piperidin-1-yl)methyl)oxazol-2-yl)phenyl)morpholine |
| 129 | 4-(4-(4-((2,3-dihydrospiro[indene-1,4'-piperidin]-1'-yl)methyl)-5-methyloxazol-2-yl)phenyl)morpholine |
| 131 | 2-(imidazo[1,2-a]pyridin-6-yl)-5-methyl-4-((4-(4-(trifluoromethoxy)phenyl)piperidin-1-yl)methyl)oxazole |
| 132 | 1'-((5-methyl-2-(4-morpholinophenyl)oxazol-4-yl)methyl)-3H-spiro[isobenzofuran-1,4'-piperidine] |
| 133 | 2-(4-(1H-pyrazol-1-yl)phenyl)-4-((4-(4-(difluoromethoxy)phenyl)piperidin-1-yl)methyl)-5-methyloxazole |
| 134 | 2-(4-(1H-imidazol-1-yl)phenyl)-4-((4-(4-(difluoromethoxy)phenyl)piperidin-1-yl)methyl)-5-methyloxazole |

As used herein, the term "pharmaceutically acceptable salt(s)" refers to a salt prepared from active compounds according to the present disclosure with relatively non-toxic acids or bases, depending on the particular substituents of those compounds. When the compounds have a relatively acidic group, base-added salts can be obtained by contacting the neutral compounds with a sufficient amount of the desired base and a pure or inert solvent. Suitable pharmaceutically acceptable base addition salts include, but are not limited to sodium, potassium, calcium, ammonium, organic amino, magnesium salts and the like. When the compounds have a relatively basic group, acid-added salts can be obtained by contacting the neutral compounds with a sufficient amount of the desired acid and pure or inert solvent. Suitable pharmaceutically acceptable acid addition salts include salts derived from non-toxic organic acids including, but are not limited to, acetic acid, propionic acid, isobutyl acid, oxalic acid, maleic acid, malonic acid, benzoic acid, succinic acid, suberic acid, fumaric acid, mandelic acid, phthalic acid, benzenesulfonic acid, p-tolylsulfonic acid, citric acid, tartaric acid, methanesulfonic acid, and the like, and non-toxic inorganic acids including, but are not limited to, hydrochloric acid, hydrobromic acid, nitric acid, carbonic acid, monohydrogencarbonic acid, phosphoric acid, monohydrogenphosphoric acid, dihydrogenphosphoric acid, sulfuric acid, monohydrogensulfuric acid, hydrogen iodide, phosphorous acid and the like. Also it includes a salt of amino acid such as arginate or its analogues, and it also includes analogues of organic acid such as glucuronic or galacturonic acid. Other examples of salts are well known through literature known in the art to which the present invention pertains.

As used herein, the phrase "compound(s) of this/the invention" includes any compound(s) of Chemical Formula 1, as well as clathrates, hydrates, solvates, or polymorphs thereof. And, even if the term "compound(s) of the invention" does not mention its pharmaceutically acceptable sat, the term includes salts thereof. In one embodiment, the compounds of this disclosure include stereo-chemically pure compounds, e.g., those substantially free (e.g., greater than 85% ee, greater than 90% ee, greater than 95% ee, greater than 97% ee, or greater than 99% ee) of other stereoisomers. That is, if the compounds of Chemical Formula 1 according to the present disclosure or salts thereof are tautomeric isomers and/or stereoisomers (e.g., geometrical isomers and conformational isomers), such isolated isomers and their mixtures also are included in the scope of this disclosure. If the compounds of the present disclosure or salts thereof have an asymmetric carbon in their structures, their active optical isomers and their racemic mixtures also are included in the scope of this disclosure.

As used herein, the term "polymorph" refers to solid crystalline forms of a compound of this disclosure or complex thereof. Different polymorphs of the same compound can exhibit different physical, chemical and/or spectroscopic properties. Different physical properties include, but are not limited to stability (e.g., to heat or light), compressibility and density (important in formulation and product manufacturing), and dissolution rates (which can affect bioavailability). Differences in stability can result from changes in chemical reactivity (e.g., differential oxidation, such that a dosage form discolors more rapidly when comprised of one polymorph than when comprised of another polymorph) or mechanical characteristics (e.g., tablets crumble on storage as a kinetically favored polymorph converts to thermodynamically more stable polymorph) or both (e.g., tablets of one polymorph are more susceptible to breakdown at high humidity). Different physical properties of polymorphs can affect their processing. For example, one polymorph might be more likely to form solvates or might be more difficult to filter or wash free of impurities than another due to, for example, the shape or size distribution of particles of it.

As used herein, the term "solvate" means a compound or its salt according to this disclosure that further includes a stoichiometric or non-stoichiometric amount of a solvent bound by non-covalent intermolecular forces. Preferred solvents are volatile, non-toxic, and acceptable for administration to humans in trace amounts.

As used herein, the term "hydrate" means a compound or its salt according to this disclosure that further includes a stoichiometric or non-stoichiometric amount of water bound by non-covalent intermolecular forces.

As used herein, the term "clathrate" means a compound or its salt in the form of a crystal lattice that contains spaces (e.g., channels) that have a guest molecule (e.g., a solvent or water) trapped within.

As used herein, the term "purified" means that when isolated, the isolate is greater than 90% pure, in one embodiment greater than 95% pure, in another embodiment greater than 99% pure and in another embodiment greater than 99.9% pure.

### Medical uses and methods of treatment of the compounds according to the present invention

The present invention further provides methods for treating a disease or condition in a subject having or susceptible to having such a disease or condition, by administering to the subject a therapeutically-effective amount of one or more compounds as described above. In one embodiment, the treatment is preventative treatment. In another embodiment, the treatment is palliative treatment. In another embodiment, the treatment is restorative treatment.

### 1. Diseases or conditions

The oxazole derivative compounds of the present invention are useful for treating or preventing diseases caused by bacterial infection. That is, the compounds of the present invention can be used to inhibit bacteria, preferably Mycobacterium genus bacteria, and more preferably Mycobacterium tuberculosis. The method of the present invention comprises administering a pharmaceutical composition comprising a therapeutically or prophylactically effective amount of a compound according to the present invention to a subject in need of treatment or prevention.

In one embodiment of the invention, the disease caused by bacterial infection may be selected from the group consisting of non-tuberculous mycobacteria (NTM) infection, salmonellosis, food poisoning, typhoid fever, paratyphoid fever, sepsis, septic shock, systemic inflammatory response syndrome (SIRS), multiple organ dysfunction syndrome (MODS), pneumonia, pulmonary tuberculosis, tuberculosis, cold, influenza, respiratory tract infection, rhinitis, nasopharyngitis, otitis media, bronchitis, lymphadenitis, parotitis, lymphadenitis, cheilitis, stomatitis, arthritis, myositis, dermatitis, vasculitis, gingivitis, periodontitis, keratitis, conjunctivitis, wound infection, peritonitis, hepatitis, osteomyelitis, cellulitis, meningitis, encephalitis, brain abscess, encephalomyelitis, meningitis, osteomyelitis, nephritis, carditis, endocarditis, enteritis, gastritis, esophagitis, duodenitis, colitis, uritis, cystitis, vaginitis, cervicitis, salpingitis, erythema infectious, dysentery, abscesses and ulcers, bacteremia, diarrhea, dysentery, gastroenteritis, genitourinary abscess, open wound or wound infection, purulent inflammation, abscess, boil, pyoderma, impetigo, folliculitis, cellulitis, postoperative wound infection, scalded skin syndrome, skin burn syndrome, thrombotic thrombocytopenia, hemolytic uremic syndrome, renal failure, pyelonephritis, glomerulonephritis, nervous system abscess, otitis media, sinusitis, pharyngitis, tonsillitis, mastoiditis, cellulitis, odontogenic infection, dacryocystitis, pleurisy, abdominal abscess, liver abscess, cholecystitis, splenic abscess, pericarditis, myocarditis, placentitis, amniotitis, mastitis, puerperal fever, toxic shock syndrome, lyme disease, gas gangrene, atherosclerosis, *Mycobacterium avium* syndrome (MAC), enterohemorrhagic *Escherichia coli* (EHEC) infection, enteropathogenic *Escherichia coli* (EPEC) infection, enteroinvasive *E. coli* infection (EIEC), methicillin-resistant *Staphylococcus aureus* (MRSA) infection, Vancomycin-resistant *Staphylococcus aureus* (VRSA) infection, and listerosis, and is preferably tuberculosis.

In another embodiment, the present invention provides a method for treating a disease caused by bacterial infection, preferably tuberculosis, comprising administering to a subject a therapeutically effective amount of a compound of chemical formula 1 or a pharmaceutically acceptable salt thereof. This method comprises administering to a subject in need of treatment a compound of the invention in an amount sufficient to inhibit bacteria, preferably *Mycobacterium tuberculosis,* i.e., a therapeutically effective amount. In this method, the compound of the invention can be administered to the subject in the form of a pharmaceutical composition described herein.

That is, the present invention provides a medical use of the compound of chemical formula 1 or a pharmaceutically acceptable salt thereof for treating or preventing a disease caused by bacterial infection, preferably tuberculosis.

### 2. Subjects

Suitable subjects to be treated according to the present invention include mammalian subjects. Mammals according to the present disclosure include, but are not limited to, human, canine, feline, bovine, caprine, equine, ovine, porcine, rodents, lagomorphs, primates, and the like, and encompass mammals *in utero.*

In one embodiment, the suitable subject to be treated according to the present invention is human.

### 3. Administration and Dosing

The compounds of the present invention are generally administered in a therapeutically effective amount.

As used herein, "effective amount" refers to an amount of a compound of the invention sufficient to slow or minimize the progression of a disease caused by bacterial infection, preferably tuberculosis or to provide a therapeutic benefit in the treatment or management of the disease. "Effective amount" also refers to an amount sufficient to inhibit or reduce the activity of tuberculosis bacteria, either *in vitro* or *in vivo.*

The compounds of the present invention can be administered by any suitable route in the form of a pharmaceutical composition adapted to such a route, and in a dose effective for the treatment intended. An effective dosage is typically in the range of about 0.001 to about 100 mg per kg body weight per day, preferably about 0.01 to about 50 mg/kg/day, in single or divided doses. Depending on age, species and disease or condition being treated, dosage levels below the lower limit of this range may be suitable. In other cases, still larger doses may be used without harmful side effects. Larger doses may also be divided into several smaller doses, for administration throughout the day.

### Pharmaceutical compositions of the compounds of the present invention

In another embodiment, the present invention provides a pharmaceutical composition comprising the compound of Chemical Formula 1 or a pharmaceutically acceptable salt thereof, and a pharmaceutically acceptable carrier or excipient. In one embodiment of the present invention, the pharmaceutical composition is used for the treatment or prevention of a disease caused by bacterial infection, preferably tuberculosis, which is described above.

The term "pharmaceutically acceptable" means suitable for use as a pharmaceutical preparation, and generally considered safe for such use. The term also means that it has been officially approved by the governing body of a country for this use, or is listed in the Korean Pharmacopoeia or the United States Pharmacopoeia.

### Pharmaceutical compositions, dosage forms and administration routes

For the treatment of the diseases or conditions referred to above, the compounds described herein or pharmaceutically acceptable salts thereof can be administered as follows:

### Oral administration

The compounds of the present invention may be administered orally, including by swallowing, so that the compound enters the gastrointestinal tract, or absorbed into the blood stream directly from the mouth (e.g., buccal or sublingual administration).

Suitable compositions for oral administration include solid, liquid, gel or powder formulations, and have a dosage form such as tablet, lozenge, capsule, granule or powder.

Compositions for oral administration may optionally be enteric coated and may exhibit delayed or sustained release through the enteric coating. That is, the composition for oral administration according to the present invention may be a formulation having an immediate or modified release pattern.

Liquid formulations can include solutions, syrups and suspensions, which can be used in soft or hard capsules. Such formulations may include a pharmaceutically acceptable carrier, for example, water, ethanol, polyethylene glycol, cellulose, or an oil. The formulation may also include one or more emulsifying agents and/or suspending agents.

In a tablet dosage form the amount of drug, active ingredient, present may be from about 0.05% to about 95% by weight, more typically from about 2% to about 50% by weight of the dosage form. In addition, tablets may contain a disintegrant, comprising from about 0.5% to about 35% by weight, more typically from about 2% to about 25% of the dosage form. Examples of disintegrants include, but are not limited to, lactose, starch, sodium starch glycolate, crospovidone, croscarmellose sodium, maltodextrin, or mixtures thereof.

Suitable lubricants, for use in a tablet, may be present in amounts from about 0.1% to about 5% by weight, and include, but are not limited to, talc, silicon dioxide, stearic acid, calcium, zinc or magnesium stearate, sodium stearyl fumarate and the like.

Suitable binders, for use in a tablet, include, but are not limited to, gelatin, polyethylene glycol, sugars, gums, starch, polyvinyl pyrrolidone, hydroxypropyl cellulose, hydroxypropylmethyl cellulose and the like. Suitable diluents, for use in a tablet, include, but are not limited to, mannitol, xylitol, lactose, dextrose, sucrose, sorbitol, microcrystalline cellulose and starch.

Suitable solubilizers, for use in a tablet, may be present in amounts from about 0.1% to about 3% by weight, and include, but are not limited to, polysorbates, sodium lauryl sulfate, sodium dodecyl sulfate, propylene carbonate, diethyleneglycol monoethyl ether, dimethyl isosorbide, polyethylene glycol (natural or hydrogenated) castor oil, HCOR^{™} (Nikkol), oleyl ester, Gelucire^{™}, caprylic/caprylic acid mono/diglyceride, sorbitan fatty acid esters, and Solutol HS^{™}.

### Parenteral Administration

Compounds of the present disclosure may be administered directly into the blood stream, muscle, or internal organs. Suitable means for parenteral administration include intravenous, intramuscular, subcutaneous intraarterial, intraperitoneal, intrathecal, intracranial, and the like. Suitable devices for parenteral administration include injectors (including needle and needle-free injectors) and infusion methods.

Compositions for parenteral administration may be formulated as immediate or modified release, including delayed or sustained release.

Most parenteral formulations are liquid compositions, and the liquid composition is an aqueous solution containing the active ingredient according to the present invention, a salt, a buffering agent, an isotonic agent, and the like.

Parenteral formulations may also be prepared in a dehydrated form (e.g., by lyophilization) or as sterile non-aqueous solutions. These formulations can be used with a suitable vehicle, such as sterile water. Solubility-enhancing agents may also be used in preparation of parenteral solutions.

### [Advantageous Effects]

The compound of chemical formula 1 or a pharmaceutically acceptable salt thereof according to the present invention, especially the compound of Table 2 or a pharmaceutically acceptable salt thereof, is very effective in inhibiting the proliferation of bacteria, and thus very useful for the prevention or treatment of bacterial infectious diseases, especially tuberculosis.

### [Mode for Invention]

Hereinafter, the present invention is described in considerable detail with examples to help those skilled in the art understand the present invention. However, the following examples are offered by way of illustration and are not intended to limit the scope of the invention. It is apparent that various changes may be made without departing from the spirit and scope of the invention or sacrificing all of its material advantages.

### [Experimental Example 1] Evaluation of the efficacy of new drug candidates based on minimum inhibitory concentration

The effects of the compounds of the present invention were evaluated as follows. The minimum inhibitory concentration for approximately 180 candidate substances was measured. Based on culture in OADC-enriched Middlebrook 7H9 liquid medium containing 2,3-diphenyl-5-thienyl-(2)-tetrazolium chloride, the efficacy of killing susceptible tuberculosis was evaluated according to the concentration of the test compound. The H37Rv strain, a standard strain, was cultured with the candidate compound to evaluate the 100% growth inhibition concentration. To measure the minimum inhibitory concentration, pure culture of *Mycobacterium tuberculosis* was used to determine the minimum inhibitory concentration of each anti-tuberculosis drug tested in a test tube. The minimum inhibitory concentration was generally measured using the broth microdilution method, and a specific concentration of cultured *Mycobacterium tuberculosis* was inoculated with a medium containing a new drug candidate and a control medium (rifampicin) to compare growth. The lowest concentration that inhibits the growth *of Mycobacterium tuberculosis* was determined as the minimum inhibitory concentration. In general, the concentration conditions of each new drug candidate were analyzed using a 96-well plate. The standard *Mycobacterium tuberculosis* used to measure the minimum inhibitory concentration was H37Rv (ATCC27294). For testing, approximately 10,000 CFU *of Mycobacterium tuberculosis* were inoculated into each well of the plate using the most diluted suspension. Using a calibrated multi-channel pipette, 50 µl of the suspension diluted to 10,000 CFU/100 µl was inoculated onto the plate by adding it to 50 µl of 7H9 medium containing the anti-tuberculosis drug (control) or test compound. Afterwards, the top was sealed with an oxygen-permeable plate cover and cultured at 37±1 °C in an incubator until the control group showed sufficient growth (typically 7-14 days). Sufficient growth of *Mycobacterium tuberculosis* was confirmed using the minimum inhibitory concentration measurement plate cultured on day 14 at the control or lowest concentration, and the concentration at which there was no growth was confirmed by comparing with the control. The lowest concentration without growth was determined as the minimum inhibitory concentration, and the minimum inhibitory concentration value is the lowest concentration (µM) of the anti-tuberculosis drug that inhibits the growth of a given *Mycobacterium tuberculosis.* Through the above process, the oxazole derivative according to the present invention was selected from among 180 candidate compounds.

### [Experimental Example 2] Preparation of the compound represented by chemical formula 1 of the present invention using Schemes 1 to 6

The present inventors synthesized compounds containing the oxazole scaffold selected in Experimental Example 1 and various derivatives thereof, using Schemes 1 to 6 below.

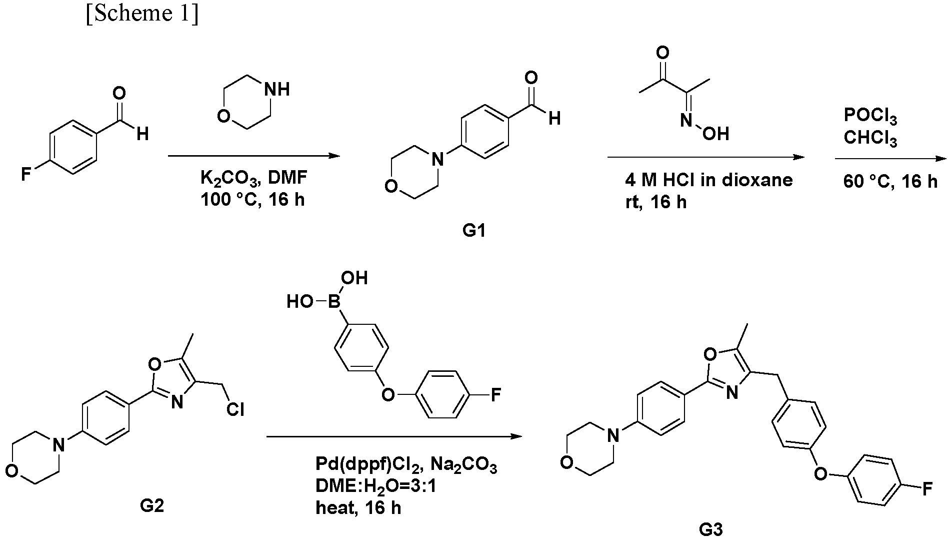

### General method of G1 synthesis

K₂CO₃ (1.1 g, 8.06 mmol), and 4-fluorobenzaldehyde (500 mg, 4.03 mmol) were added to a solution of morpholine (386 mg, 4.43 mmol) dissolved in anhydrous DMF (10 mL) and heated at 80 °C for 16 hours. After the reaction was completed, the reaction mixture was diluted with EtOAc and water, and then the solution was extracted with EtOAc. The organic layer was washed with water, dried over anhydrous Na₂SO₄ and evaporated *in vacuo.* The residue was purified by column chromatography (CH₂Cl₂: MeOH = 20: 1) to give G1 product (361 mg, 47%).

### General method of G2 synthesis

2,3-Butanedione monoxime (191 mg, 1.89 mmol) was added to a solution of compound G1 (361 mg, 1.89 mmol) dissolved in 4M HCl in dioxane (3.6 mL) and stirred at 25°C for 16 hours. After concentrating the reaction, the reaction mixture was dissolved in chloroform (4 mL), POCl₃ (0.19 mL, 2.08 mmol) was added, and the mixture was heated at 60°C for 16 hours. After the reaction was completed, it was cooled to room temperature. The reaction mixture was neutralized with saturated NaHCO₃ solution and then extracted with CH₂Cl₂. The organic layer was washed with water, dried over anhydrous Na₂SO₄ and evaporated *in vacuo.* The residue was purified by column chromatography (EtOAc: Hexane, 1:5) to obtain G2 product (255 mg, 46%).

### General method of G3 synthesis

G2 product (255 mg, 0.87 mmol), (4-(4-fluorophenoxy)phenyl)boronic acid (202 mg, 0.87mmol), Pd(dppf)Cl₂ (31.9 mg, 43.6 µmοl), and Na₂CO₃ (238 mg, 2.25 mmol) were added to DME : H₂O (v/v 3:1) and heated at 120°C for 16 hours. After the reaction was completed, it was cooled to room temperature. The reaction mixture was diluted with EtOAc and water, and then the solution was extracted with EtOAc. The organic layer was washed with water, dried over anhydrous Na₂SO₄ and evaporated *in vacuo.* The residue was purified by column chromatography (EtOAc: Hexane, 1:5) to obtain G3 product (199 mg, 51%).

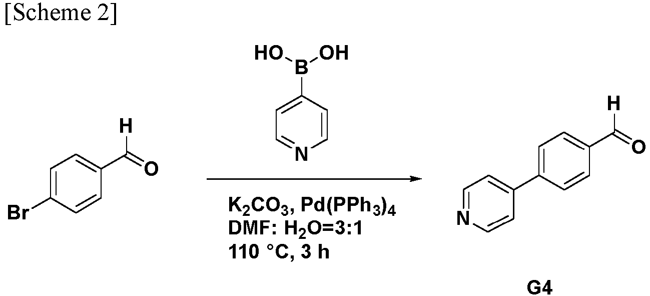

### General method of G4 synthesis

4-bromobenzaldehyde (1.0 g, 5.40 mmol), pyridin-4-ylboronic acid (663 mg, 5.40 mmol), K₂CO₃(1.5 g, 16.2 mmol) and Pd(PPh₃)₄ (247 mg, 0.27 mmol) were added to DMF : H₂O (v/v 3:1) and heated at 110 °C for 3 hours. After the reaction was completed, it was cooled to room temperature. The reaction mixture was diluted with EtOAc and water, and then the solution was extracted with EtOAc. The organic layer was washed with water, dried over anhydrous Na₂SO₄ and evaporated *in vacuo.* The residue was purified by column chromatography (EtOAc: Hexane, 1:1) to obtain G4 product (429 mg, 43%).

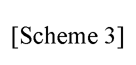

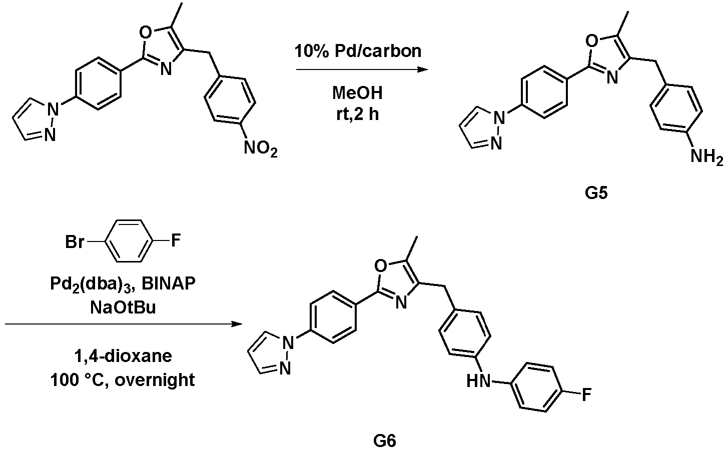

### General method of G5 synthesis

10% palladium/carbon was added to a solution of 2-(4-(1H-pyrazol-1-yl)phenyl)-5-methyl-4-(4-nitrobenzyl)oxazole (197 mg, 0.55 mmol) dissolved in anhydrous MeOH (5 mL) and the mixture was stirred at room temperature under hydrogen gas for 2 hours. After the reaction was completed, the reaction was cooled with cold water. The reaction mixture was filtered through a pad of Celite and evaporated *in vacuo.* The residue was purified by column chromatography (CH₂Cl₂: MeOH = 20: 1) to give G5 product (139 mg, 77%).

### General method of G6 synthesis

1-bromo-4-fluorobenzene (39.7 mg, 0.23 mmol), Pd₂(dba)₃ (13.8 mg, 0.015 mmol), 2,2'-bis(diphenyphosphino)-1,1'-binaphthyl) (9.4 mg, 0.015 mmol), and NaOtBu (43.5 mg, 0.45 mmol) were added to a solution of G5 (50 mg, 0.15 mmol) dissolved in anhydrous 1,4-dioxane (2 mL), and the mixture was stirred at 100 °C for 16 hours. After the reaction was completed, it was cooled to room temperature. The reaction mixture was diluted with EtOAc and water, and then the solution was extracted with EtOAc. The organic layer was washed with water, dried over anhydrous Na₂SO₄ and evaporated *in vacuo.* The residue was purified by column chromatography (EtOAc: Hexane, 1:3) to obtain G6 product (37.3 mg, 58%).

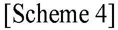

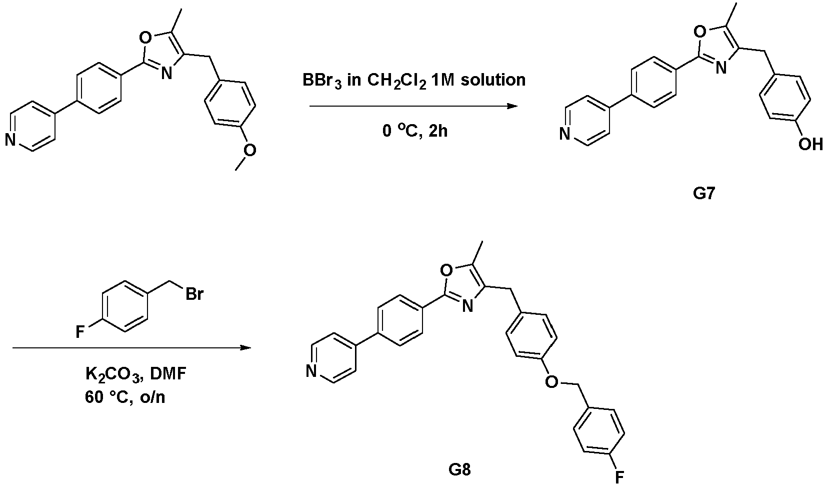

### General method of G7 synthesis

4-(4-methoxybenzyl)-5-methyl-2-(4-(pyridin-4-yl)phenyl)oxazole (320 mg, 0.90 mmol) was added to a solution of 1 M BBr₃ (2.1 ml) dissolved in in methylene chloride, and then the mixture was stirred at 0 °C for 3 hours. After evaporating the organic solvent in the reaction mixture under reduced pressure, the obtained G7 product was used in the next experiment without purification.

### General method of G8 synthesis

K₂CO₃ (30.2, 0.22 mmol) and 1-(bromomethyl)-4-fluorobenzene (33.2 mg, 0.18 mmol) were added to a solution of compound G9 (50 mg, 0.15 mmol) dissolved in anhydrous DMF (2 mL), and the mixture was stirred at 60 °C for 16 hours. After the reaction was completed, it was cooled to room temperature. After evaporating the organic solvent in the reaction mixture under reduced pressure, the residue was purified by column chromatography (CH₂Cl₂: Methanol = 10:1) to obtain G8 product (11.6 mg, 18%).

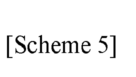

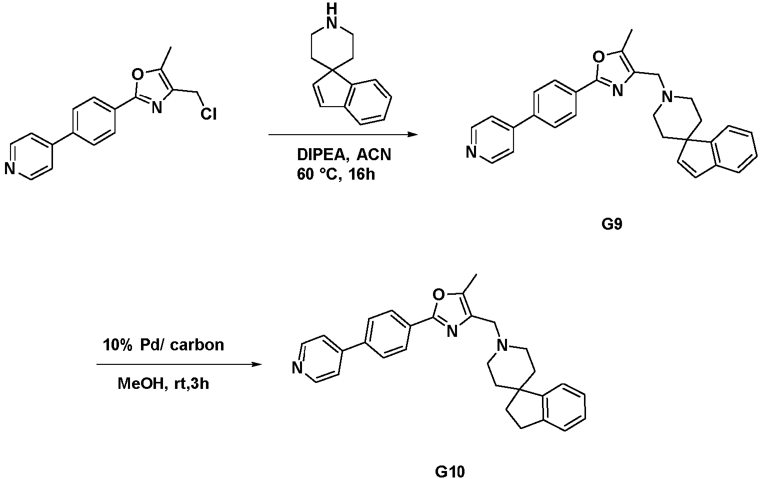

### General method of G9 synthesis

4-(chloromethyl)-5-methyl-2-(4-(pyridin-4-yl)phenyl)oxazole (80 mg, 0.281 mmol), DIPEA (0.07 mL, 0.422 mmol), and spiro[indene-1,4'-piperidine] (62.6 mg, 0.338 mmol) were added to acetonitrile (2 mL), and heated at 60 °C for 16 hours. After the reaction was completed, it was cooled to room temperature. The organic solvent in the reaction mixture was evaporated under reduced pressure. The residue was purified by column chromatography (CH₂Cl₂: Methanol = 20:1) to obtain G9 product (98.4 mg, 81%).

### General method of G10 synthesis

10% palladium on carbon (30 mg) was added to a solution of 5-methyl-2-(4-(pyridin-4-yl)phenyl)-4-(spiro[indene-1,4'-piperidin]-1'-ylmethyl)oxazole (50 mg, 0.115 mmol) dissolved in MeOH (5 mL), and H₂ (g) was purged several times in vacuum to remove the gas. The reaction mixture was stirred under H₂ (g) at room temperature for 3 hours. The reaction mixture was filtered through a pad of Celite and evaporated *in vacuo.* The residue was purified by column chromatography (CH₂Cl₂: MeOH = 20: 1) to give G10 product (50.7 mg, 100%).

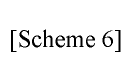

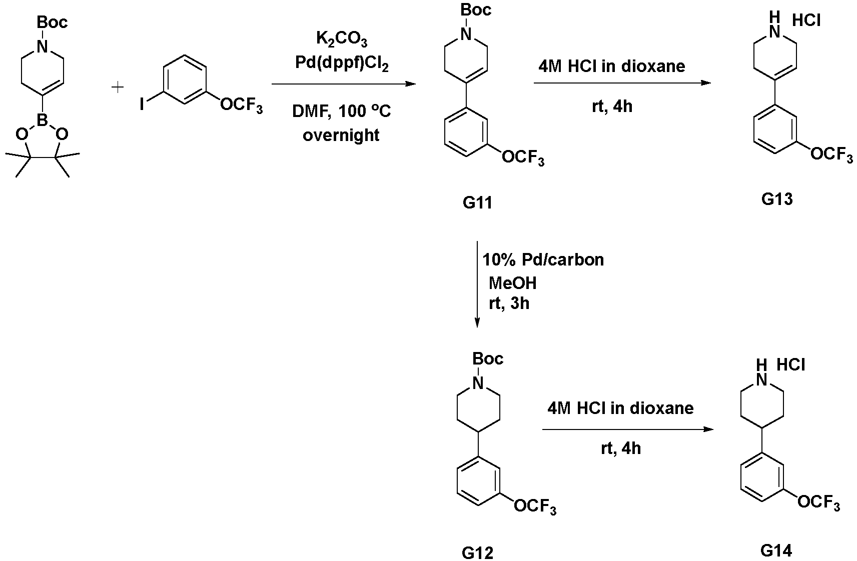

### General method of G11 synthesis

Tert-butyl 4-(4,4,5,5-tetramethyl-1,3,2-dioxaborolan-2-yl)-3,6-dihydropyridine-1(2H)-carboxylate (500 mg, 1.62 mmol), K₂CO₃(670 mg, 4.85 mmol), and Pd(dppf)Cl₂ (118 mg, 0.16 mmol) were added to a solution of 1-iodo-3-(trifluoromethoxy)benzene (558 mg, 1.94 mmol) dissolved in anhydrous DMF (10 mL), and heated at 100 °C for 16 hours. After the reaction was completed, it was cooled to room temperature. The reaction mixture was diluted with EtOAc and water, and then the solution was extracted with EtOAc. The organic layer was washed with water, dried over anhydrous Na₂SO₄ and evaporated *in vacuo.* The residue was purified by column chromatography (EtOAc: Hexane, 1:3) to obtain G11 product (192 mg, 35%).

### General method of G12 synthesis

10% palladium on carbon (30 mg) was added to a solution of compound G11 (192 mg, 0.56 mmol) dissolved in MeOH (5 mL), and the gas was removed by purging with H₂ (g) several times in vacuum. The reaction mixture was stirred under H₂ (g) at room temperature for 3 hours. The reaction mixture was filtered through a pad of Celite and evaporated *in vacuo.* The residue was purified by column chromatography (EtOAc: Hexane, 1:3) to obtain G12 product (179 mg, 93%).

### General method of G14 synthesis

4M HCl solution (0.4 mL) dissolved in 1,4-dioxane was added to G12 (179 mg, 0.52 mmol), and the reaction was stirred at room temperature for 3 hours. After evaporating the organic solvent in the reaction mixture under reduced pressure, the obtained G14 product was used in the next experiment without purification.

### [Experimental Example 3] Preparation of a new compound of chemical formula 1 according to the present invention and evaluation of anti-tuberculosis activity

Using the methods of Experimental Examples 1 and 2, 132 compounds represented by chemical formula 1 of the present invention were prepared, and the anti-tuberculosis activity of the prepared compounds was evaluated. The results are summarized and shown in Table 3.

**[Table 3]**

| No. | Structure | IUPAC name | Characterization | Activity MIC₁₀₀ (µM) |
|---|---|---|---|---|
| 1 | 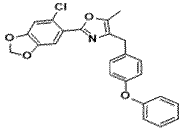 | 2-(6-chlorobenzo[d][1,3]dioxol-5-yl)-5-methyl-4-(4-phenoxybenzyl)oxazole | yellow solid; ¹H NMR (400 MHz, CDCl₃) δ 2.29 (s, 3H), 3.86 (s, 2H), 6.03 (s, 2H), 6.91 (s, 1H), 6.93-7.00 (m, 4H), 7.07 (t, *J* = 7.4 Hz, 1H), 7.25 (d, *J* = 8.0 Hz, 2H), 7.31 (t, *J =* 8.0 Hz, 2H), 7.37 (s, 1H); LCMS (ESI) m/z 420 [M + H]⁺. | >10 |
| 2 | 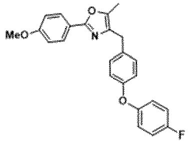 | 4-(4-(4-fluorophenoxy)benzyl)-2-(4-methoxyphenyl)-5-methyloxazole | yellow solid; ¹H NMR (400 MHz, CDCl₃) δ 2.29 (s, 3H), 3.85 (s, 5H), 6.89-7.02 (m, 8H), 7.24 (t, *J* = 7.4 Hz, 2H), 7.92 (d, *J* = 8.8 Hz, 2H); LCMS (ESI) m/z 390 [M + H]+. | 4 |
| 3 | 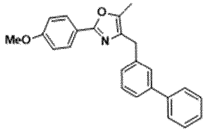 | 4-([1,1'-biphenyl]-3-ylmethyl)-2-(4-methoxyphenyl)-5-methyloxazole | yellow solid; ¹H NMR (400 MHz, CDCl₃) δ 2.29 (s, 3H), 3.85 (s, 5H), 6.89-7.02 (m, 9H), 7.24 (t, *J* = 7.4 Hz, 2H), 7.92 (d, *J* = 8.8 Hz, 2H); LCMS (ESI) m/z 356 [M + H]+. | >10 |
| 4 | 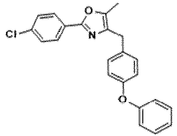 | 2-(4-chlorophenyl)-5-methyl-4-(4-phenoxybenzyl) oxazole | white solid; ¹H NMR (400 MHz, CDCl₃) δ 2.30 (s, 3H), 3.85 (s, 2H), 6.94-7.00 (m, 4H), 7.07 (t, *J* = 7.4 Hz, 1H), 7.24 (t, *J* = 7.4 Hz, 2H), 7.31 (t, *J* = 7.0 Hz, 2H), 7.39 (d, *J* = 8.8 Hz, 2H), 7.92 (d, *J* = 8.8 Hz, 2H); LCMS (ESI) m/z 376 [M + H]⁺. | >10 |
| 5 | 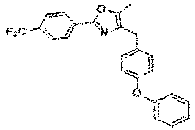 | 5-methyl-4-(4-phenoxybenzyl)-2-(4-(trifluoromethyl)phenyl)ox azole | pale yellow solid; ¹H NMR (400 MHz, CDCl₃) δ 2.26 (s, 3H), 3.81 (s, 2H), 6.88-6.94 (m, 4H), 7.01 (t, *J* = 7.8 Hz, 1H), 7.17-7.26 (m, 4H), 7.60 (d, *J* = 8.0 Hz, 2H), 8.03 (d, *J* = 8.0 Hz, 2H); LCMS (ESI) m/z 410 [M + H]⁺. | >10 |
| 6 | 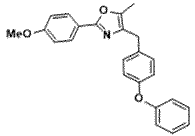 | 2-(4-methoxyphenyl)-5-methyl-4-(4-phenoxybenzyl) oxazole | white solid; ¹H NMR (400 MHz, CDCl₃) δ 2.28 (s, 3H), 3.85 (s, 5H), 6.92-6.96 (m, 4H), 6.98 (d, *J* = 8.0 Hz, 2H), 7.07 (t, *J* = 7.4 Hz, 1H), 7.24 (d, *J* = 8.4 Hz, 2H), 7.31 (t, *J* = 8.0 Hz, 2H), 7.92 (d, *J* = 9.2 Hz, 2H); LCMS (ESI) m/z 372 [M + H]⁺. | 8 |
| 7 | 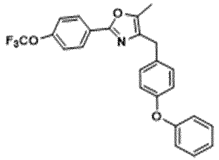 | 5-methyl-4-(4-phenoxybenzyl)-2-(4-(trifluoromethoxy)phenyl)o xazole | yellow sticky solid; ¹H NMR (400 MHz, CDCl₃) δ 2.31 (s, 3H), 3.85 (s, 2H), 6.93-7.00 (m, 4H), 7.07 (t, *J* = 7.2 Hz, 1H), 7.22-7.33 (m, 6H), 8.02 (d, *J* = 8.8 Hz, 2H); LCMS (ESI) m/z 426 [M + H]⁺. | >10 |
| 8 | 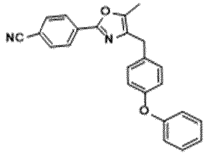 | 4-(5-methyl-4-(4-phenoxybenzyl)oxazol-2-yl)benzonitrile | white solid; ¹H NMR (400 MHz, CDCl₃) δ 2.64 (s, 3H), 4.17 (s, 2H), 7.25-7.30 (m, 4H), 7.39 (t, *J* = 7.4 Hz, 1H), 7.54 (d, *J* = 8.4 Hz, 2H), 7.61 (t, *J* = 16 Hz, 2H), 8.01 (d, *J* = 8.4 Hz, 2H), 8.39 (d, *J* = 8.4 Hz, 2H); LCMS (ESI) m/z 367 [M + H]⁺. | >10 |
| 9 | 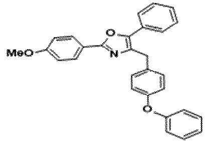 | 2-(4-methoxyphenyl)-4-(4-phenoxybenzyl)-5-phenyloxazole | white solid; ¹H NMR (400 MHz, CDCl₃) δ 3.87 (s, 3H), 4.18 (s, 2H), 6.95-7.00 (m, 6H), 7.07 (t, *J* = 7.6 Hz, 1H), 7.26-7.35 (m, 5H), 7.44 (t, *J* = 7.6 Hz, 2H), 7.66 (d, *J* = 8.4 Hz, 2H), 8.05 (d, *J* = 8.8 Hz, 2H); LCMS (ESI) m/z 434 [M + H]⁺. | >10 |
| 10 | 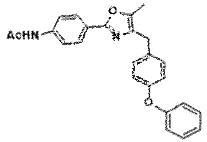 | N-(4-(5-methyl-4-(4-phenoxybenzyl)oxazol-2-yl)phenyl)acetamide | white solid; ¹H NMR (400 MHz, CDCl₃) δ 2.18 (s, 3H), 2.22 (s, 3H), 3.85 (s, 2H), 6.92-6.99 (m, 4H), 7.07 (t, *J* = 8.4 Hz, 1H), 7.22-7.33 (m, 4H), 7.46 (brs, 1H), 7.58 (d, *J* = 8.4 Hz, 2H), 7.93 (d, *J* = 8.8 Hz, 2H); LCMS (ESI) m/z 399 [M + H]⁺. | >10 |
| 11 | 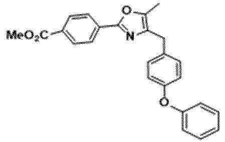 | methyl 4-(5-methyl-4-(4-phenoxybenzyl)oxazol-2-yl)benzoate | white solid; ¹H NMR (400 MHz, CDCl₃) δ 2.23 (s, 3H), 3.87 (s, 2H), 3.93 (s, 3H), 6.94-7.00 (m, 4H), 7.07 (t, *J* = 7.2 Hz, 1H), 7.24 (d, *J* = 8.8 Hz, 2H), 7.31 (t, *J* = 7.8 Hz, 2H), 8.07 (q, *J =* 8.0 Hz, 4H); LCMS (ESI) m/z 400 [M + H]⁺. | >10 |
| 12 | 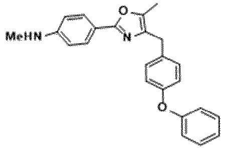 | N-methyl-4-(5-methyl-4-(4-phenoxybenzyl)oxazol-2-yl)aniline | white solid; ¹H NMR (400 MHz, CDCl₃) δ 2.26 (s, 3H), 2.87 (s, 3H), 3.84 (s, 2H), 3.97 (brs, 1H), 6.60 (d, *J* = 8.8 Hz, 2H), 6.93-7.00 (m, 4H), 7.07 (t, *J* = 7.2 Hz, 1H), 7.24 (d, *J =* 8.4 Hz, 2H), 7.31 (t, *J* = 7.8 Hz, 2H), 7.82 (d, *J* = 8.8 Hz, 2H); LCMS (ESI) m/z 371 [M + H]⁺. | 16 |
| 13 | 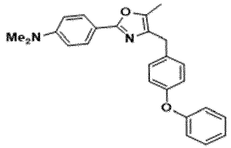 | N,N-dimethyl-4-(5-methyl-4-(4-phenoxybenzyl)oxazol-2-yl)aniline | yellow solid; ¹H NMR (400 MHz, CDCl₃) δ 2.27 (s, 3H), 3.04 (s, 6H), 3.85 (s, 2H), 6.71 (d, *J* = 8.8 Hz, 2H), 6.94-7.00 (m, 4H), 7.07 (t, *J* = 7.2 Hz, 1H), 7.25 (d, *J* = 8.4 Hz, 2H), 7.31 (t, *J* = 7.8 Hz, 2H), 7.86 (d, *J* = 8.8 Hz, 2H); LCMS (ESI) m/z 385 [M + H]⁺. | 8 |
| 14 | 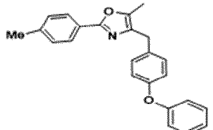 | 5-methyl-4-(4-phenoxybenzyl)-2-(p-tolyl)oxazole | yellow solid; ¹H NMR (400 MHz, CDCl₃) δ 2.30 (s, 3H), 2.39 (s, 3H), 3.87 (s, 2H), 6.95-7.02 (m, 4H), 7.08 (t, *J* = 8.0 Hz, 1H), 7.23-7.30 (m, 4H), 7.32 (t, *J* = 7.2 Hz, 2H), 7.90 (d, *J* = 8.4 Hz, 2H); LCMS (ESI) m/z 356 [M + H]⁺. | >10 |
| 15 | 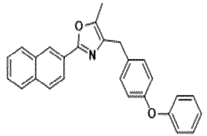 | 5-methyl-2-(naphthalen-2-yl)-4-(4-phenoxybenzyl) oxazole | white solid; ¹H NMR (400 MHz, CDCl₃) δ 2.35 (s, 3H), 3.91 (s, 2H), 6.96-7.01 (m, 4H), 7.08 (t, *J* = 7.2 Hz, 1H), 7.26-7.34 (m, 4H), 7.50-7.52 (m, 2H), 7.83-7.92 (m, 3H), 8.10 (dd, *J* = 8.6, 1.4 Hz, 1H), 8.90 (s, 1H); LCMS (ESI) m/z 392 [M + H]⁺. | >16 |
| 16 | 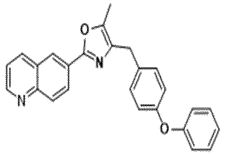 | 5-methyl-4-(4-phenoxybenzyl)-2-(quinolin-6-yl)oxazole | yellow solid; ¹H NMR (400 MHz, CDCl₃) δ 2.36 (s, 3H), 3.90 (s, 2H), 6.95-7.00 (m, 4H), 7.07 (t, *J* = 7.4 Hz, 1H), 7.26-7.33 (m, 4H), 7.42 (q, *J* = 4.1 Hz, 1H), 8.14 (d, *J* = 8.8 Hz, 1H), 8.21 (d, *J* = 8.4 Hz, 1H), 8.33 (dd, *J =* 8.8, 2.0 Hz, 1H), 8.45 (d, *J* = 1.6 Hz, 1H), 8.93 (dd, J = 4.4, 1.6 Hz, 1H); LCMS (ESI) m/z 393 [M + H]⁺. | 8 |
| 17 | 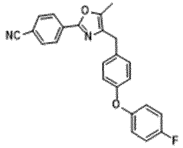 | 4-(4-(4-(4-fluorophenoxy)benzyl)-5-methyloxazol-2-yl)benzonitrile | white solid; ¹H NMR (400 MHz, CDCl₃) δ 2.33 (s, 3H), 3.85 (s, 2H), 6.89-7.03 (m, 6H), 7.22 (d, *J* = 8.8 Hz, 2H), 7.70 (d, *J* = 8.8 Hz, 2H), 8.07 (d, *J* = 8.4 Hz, 2H); LCMS (ESI) m/z 385 [M + H]+. | 8 |
| 18 | 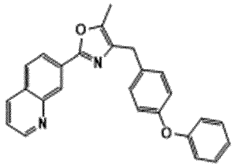 | 5-methyl-4-(4-phenoxybenzyl)-2-(quinolin-7-yl)oxazole | pale yellow solid; ¹H NMR (400 MHz, CDCl₃) δ 2.36 (s, 3H), 3.90 (s, 2H), 6.94-7.00 (m, 4H), 7.07 (t, *J* = 7.4 Hz, 1H), 7.26-7.33 (m, 4H), 7.41 (q, *J* = 4.3 Hz, 1H), 7.85 (d, *J* = 8.4 Hz, 1H), 8.14 (d, *J* = 8.4 Hz, 1H), 8.2 (dd, J = 8.4, 1.6 Hz, 1H), 8.68 (s, 1H), 8.95 (d, *J =* 2.8 Hz, 1H); LCMS (ESI) m/z 393 [M + H]⁺. | 8 |
| 19 | 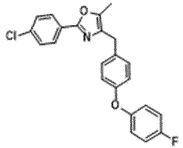 | 2-(4-chlorophenyl)-4-(4-(4-fluorophenoxy)benzyl)-5-methyloxazole | white solid; ¹H NMR (400 MHz, CDCl₃) δ 2.30 (s, 3H), 3.84 (s, 2H), 6.89 -7.02 (m, 6H), 7.22 (d, *J* = 8.4 Hz, 2H), 7.39 (d, *J* = 8.4 Hz, 2H), 7.91 (d, *J* = 8.4 Hz, 2H); LCMS (ESI) m/z 394 [M + H]⁺. | 16 |
| 20 | 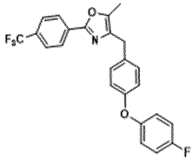 | 4-(4-(4-fluorophenoxy)benzyl)-5-methyl-2-(4-(trifluoromethyl)phenyl)ox azole | white solid; ¹H NMR (400 MHz, CDCl₃) δ 2.33 (s, 3H), 3.86 (s, 2H), 6.89-7.03 (m, 6H), 7.23 (d, *J* = 8.4 Hz, 2H), 7.67 (d, *J* = 8.4 Hz, 2H), 8.09 (d, *J* = 8.4 Hz, 2H); LCMS (ESI) m/z 428 [M + H]⁺. | 8 |
| 21 | 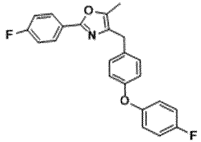 | 4-(4-(4-fluorophenoxy)benzyl)-2-(4-fluorophenyl)-5-methyloxazole | white solid; ¹H NMR (400 MHz, CDCl₃) δ 2.31 (s, 3H), 3.90 (s, 2H), 6.89-7.03 (m, 6H), 7.13 (t, *J* = 8.6 Hz, 2H), 7.26 (t, *J* = 4.4 Hz, 2H), 8.07 (dd, J = 8.4, 5.6 Hz, 2H); LCMS (ESI) m/z 378 [M + H]⁺. | 16 |
| 22 | 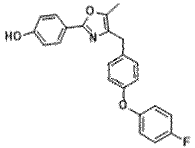 | 4-(4-(4-(4-fluorophenoxy)benzyl)-5-methyloxazol-2-yl)phenol | white solid; ¹H NMR (400 MHz, CDCl₃) δ 2.32 (s, 3H), 3.98 (s, 2H), 6.89-6.98 (m, 4H), 7.00-7.04 (m, 4H), 7.30 (d, *J* = 8.4 Hz, 2H), 8.04 (d, *J* = 8.4 Hz, 2H) one resonance obscured or overlapping; LCMS (ESI) m/z 376 [M + H]⁺. | >10 |
| 23 | 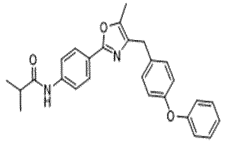 | N-(4-(5-methyl-4-(4-phenoxybenzyl)oxazol-2-yl)phenyl)isobutyramide | yellow solid; ¹H NMR (400 MHz, CDCl₃) δ 1.24 (s, 3H), 1.26 (s, 3H), 2.29 (s, 3H), 2.49-2.55 (m, 1H), 3.84 (s, 2H), 6.93-6.99 (m, 4H), 7.06 (t, *J* = 7.2 Hz, 1H), 7.23 (d, *J* = 8.8 Hz, 2H), 7.30 (t, *J* = 8.0 Hz, 2H), 7.37 (brs, 1H), 7.61 (t, *J* = 8.8 Hz, 2H), 7.93 (d, *J* = 8.8 Hz, 2H); LCMS (ESI) m/z 427 [M + H]⁺. | >10 |
| 24 | 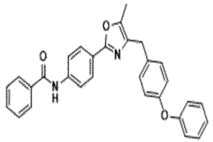 | N-(4-(5-methyl-4-(4-phenoxybenzyl)oxazol-2-yl)phenyl)benzamide | yellow solid; ¹H NMR (400 MHz, CDCl₃) δ 2.30 (s, 3H), 3.85 (s, 2H), 6.93-6.99 (m, 4H), 7.06 (t, *J* = 7.2 Hz, 1H), 7.23 (d, *J* = 8.4 Hz, 2H), 7.30 (d, *J* = 8.0 Hz, 2H), 7.46 (t, *J* = 7.4 Hz, 2H), 7.53 (t, *J* = 3.6 Hz, 1H), 7.72 (d, *J =* 8.4 Hz, 2H), 7.85 (d, *J* = 6.8 Hz, 2H), 7.97 (d, *J* = 8.8 Hz, 2H), 8.10 (brs, 1H); LCMS (ESI) m/z 461 [M + H]⁺. | 16 |
| 25 | 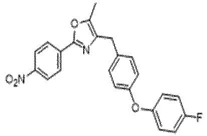 | 4-(4-(4-fluorophenoxy)benzyl)-5-methyl-2-(4-nitrophenyl)oxazole | yellow solid; ¹H NMR (400 MHz, CDCl₃) δ 2.35 (s, 3H), 3.87 (s, 2H), 6.90-7.03 (m, 6H), 7.24 (t, *J* = 9.2 Hz, 2H), 8.14 (d, *J* = 9.2 Hz, 2H), 8.28 (d, *J* = 8.8 Hz, 2H); LCMS (ESI) m/z 405 [M + H]+. | 16 |
| 26 | 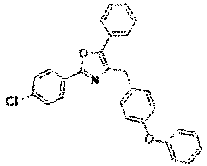 | 2-(4-chlorophenyl)-4-(4-phenoxybenzyl)-5-phenyloxazole | white solid; ¹H NMR (400 MHz, CDCl₃) δ 4.24 (s, 2H), 6.94-6.99 (m, 4H), 7.08 (t, *J =* 7.6 Hz, 1H), 7.29-7.32 (m, 4H), 7.40 (d, *J =* 7.2 Hz, 1H), 7.45-7.49 (m, 4H), 7.67 (d, *J =* 7.6 Hz, 2H), 8.14 (d, *J* = 8.4 Hz, 2H); LCMS (ESI) m/z 438 [M + H]⁺. | >10 |
| 27 | 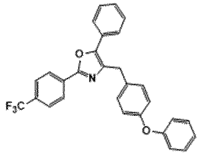 | 4-(4-phenoxybenzyl)-5-phenyl-2-(4-(trifluoromethyl)phenyl)ox azole | white solid; ¹H NMR (400 MHz, CDCl₃) δ 4.2 (s, 2H), 6.96-7.01 (m, 4H), 7.06 (t, *J* = 7.6 Hz, 1H), 7.29-7.33 (m, 4H), 7.38 (t, *J* = 7.4 Hz, 1H), 7.47 (t, *J* = 7.6 Hz, 2H), 7.68-7.74 (m, 4H), 8.22 (d, *J* = 8.4 Hz, 2H); LCMS (ESI) m/z 472 [M + H]+. | >10 |
| 28 | 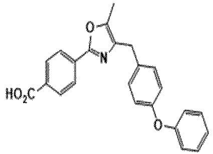 | 4-(5-methyl-4-(4-phenoxybenzyl)oxazol-2-yl)benzoic acid | white solid; ¹H NMR (400 MHz, DMSO-*d₆*) δ 2.58 (s, 3H), 3.89 (s, 2H), 6.97-7.02 (m, 4H), 7.15 (t, *J* = 7.8 Hz, 1H), 7.33 (d, *J* = 8.4 Hz, 2H), 7.40 (t, *J* = 8.0 Hz, 2H), 8.03-8.19 (m, 4H) one resonance obscured or overlapping; LCMS (ESI) m/z 386 [M + H]+. | >10 |
| 29 | 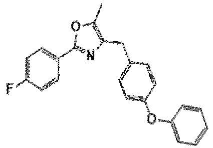 | 2-(4-fluorophenyl)-5-methyl-4-(4-phenoxybenzyl) oxazole | yellow solid; ¹H NMR (400 MHz, CDCl₃) δ 2.30 (s, 3H), 3.85 (s, 2H), 6.93-7.00 (m, 4H), 7.05- 7.12 (m, 3H), 7.24 (t, *J* = 6.6 Hz, 2H), 7.31 (t, *J* = 8.0 Hz, 2H), 7.96-7.99 (m, 2H); LCMS (ESI) m/z 360 [M + H]⁺. | >10 |
| 30 | 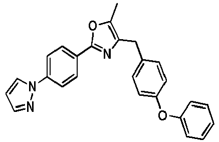 | 2-(4-(1H-pyrazol-1-yl)phenyl)-5-methyl-4-(4-phenoxybenzyl)oxazole | yellow solid; ¹H NMR (400 MHz, CDCl₃) δ 2.32 (s, 3H), 3.87 (s, 2H), 6.49 (t, *J* = 2.0 Hz, 1H), 6.94-7.00 (m, 4H), 7.07 (t, *J* = 7.4 Hz, 1H), 7.25 (d, *J* = 8.0 Hz, 2H), 7.31 (t, *J* = 8.0 Hz, 2H), 7.75-7.79 (m, 3H), 7.98 (d, *J* = 2.0 Hz, 1H), 8.08 (d, *J* = 8.8 Hz, 2H); LCMS (ESI) m/z 408 [M + H]⁺. | 8 |
| 31 | 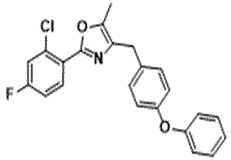 | 2-(2-chloro-4-fluorophenyl)-5-methyl-4-(4-phenoxybenzyl)oxazole | yellow sticky solid; ¹H NMR (400 MHz, CDCl₃) δ 2.31 (s, 3H), 3.88 (s, 2H), 6.94-7.10 (m, 6H), 7.22 (dd, *J* = 8.8, 2.8 Hz, 1H), 7.25 (d, *J* = 8.8 Hz, 2H), 7.31 (t, *J* = 8.0 Hz, 2H), 7.94 (q, *J =* 5.1 Hz, 1H); LCMS (ESI) m/z 394 [M + H]⁺. | >10 |
| 32 | 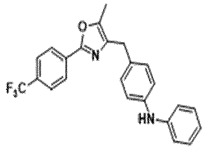 | 4-((5-methyl-2-(4-(trifluoromethyl)phenyl)ox azol-4-yl)methyl)-N-phenylaniline | green solid; ¹H NMR (400 MHz, CDCl₃) δ 2.33 (s, 3H), 3.84 (s, 2H), 5.65 (brs, 1H), 6.90 (t, *J* = 7.4 Hz, 1H), 7.03 (dd, *J* = 8.6, 2.2 Hz, 4H), 7.18 (d, *J* = 8.0 Hz, 2H), 7.24 (t, *J =* 6.6 Hz, 2H), 7.67 (d, *J=* 8.4 Hz, 2H), 8.10 (d, *J* = 8.0 Hz, 2H); LCMS (ESI) m/z 409 [M + H]⁺. | 16 |
| 33 | 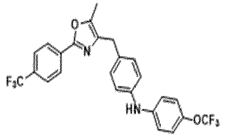 | 4-((5-methyl-2-(4-(trifluoromethyl)phenyl)ox azol-4-yl)methyl)-N-(4-(trifluoromethoxy)phenyl)a niline | yellow solid; ¹H NMR (400 MHz, CDCl₃) δ 2.33 (s, 3H), 3.85 (s, 2H), 5.66 (brs, 1H), 6.98-7.03 (m, 4H), 7.08 (d, *J* = 8.8 Hz, 2H), 7.20 (d, *J* = 8.4 Hz, 2H), 7.67 (d, *J* = 8.0 Hz, 2H), 8.10 (d, *J* = 8.0 Hz, 2H); LCMS (ESI) m/z 493 [M + H]⁺. | 8 |
| 34 | 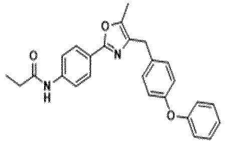 | N-(4-(5-methyl-4-(4-phenoxybenzyl)oxazol-2-yl)phenyl)propionamide | yellow solid; ¹H NMR (400 MHz, CDCl₃) δ 1.17 (t, *J* = 7.2 Hz, 3H), 2.29 (s, 3H), 2.41 (q, J = 7.6 Hz, 2H), 3.85 (s, 2H), 6.93-7.00 (m, 4H), 7.07 (t, *J* = 7.4 Hz, 1H), 7.23 -7.27 (m, 3H), 7.31 (t, *J* = 8.0 Hz, 2H), 7.60 (d, *J* = 8.4 Hz, 2H), 7.95 (d, *J* = 8.8 Hz, 2H); LCMS (ESI) m/z 413 [M + H]⁺. | >10 |
| 35 | 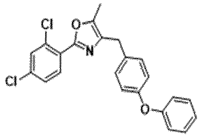 | 2-(2,4-dichlorophenyl)-5-methyl-4-(4-phenoxybenzyl) oxazole | yellow sticky solid; ¹H NMR (400 MHz, CDCl₃) δ 2.31 (s, 3H), 3.87 (s, 2H), 6.93-6.99 (m, 4H), 7.07 (t, *J* = 7.2 Hz, 1H), 7.24 (d, *J =* 8.0 Hz, 2H), 7.28-7.33 (m, 3H), 7.48 (d, *J =* 1.6 Hz, 1H), 7.90 (d, *J* = 8.4 Hz, 1H); LCMS (ESI) m/z 410 [M + H]⁺. | >10 |
| 36 | 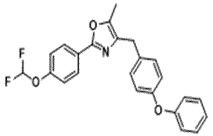 | 2-(4-(difluoromethoxy)phenyl)-5-methyl-4-(4-phenoxybenzyl) oxazole | yellow sticky solid; ¹H NMR (400 MHz, CDCl₃) δ 2.30 (s, 3H), 3.85 (s, 2H), 6.93-6.99 (m, 4H), 7.07 (t, *J* = 7.4 Hz, 1H), 7.15 (d, *J* = 8.8 Hz, 2H), 7.24 (t, *J* = 6.2 Hz, 3H), 7.31 (t, *J* = 8.0 Hz, 2H), 7.99 (d, *J* = 8.8 Hz, 2H); LCMS (ESI) m/z 408 [M + H]⁺. | >10 |
| 37 | 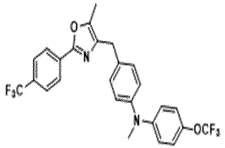 | N-methyl-4-((5-methyl-2-(4-(trifluoromethyl)phenyl)ox azol-4-yl)methyl)-N-(4-(trifluoromethoxy)phenyl)a niline | white solid; ¹H NMR (400 MHz, CDCl₃) δ 2.35 (s, 3H), 3.27 (s, 3H), 3.86 (s, 2H), 6.88 (d, *J =* 8.8 Hz, 2H), 7.01 -7.08 (m, 4H), 7.24 (t, *J* = 8.8 Hz, 2H), 7.68 (d, *J* = 8.4 Hz, 2H), 8.10 (d, *J =* 8.8 Hz, 2H); LCMS (ESI) m/z 507 [M + H]⁺. | >10 |
| 38 | 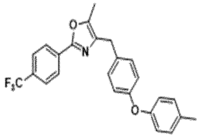 | 5-methyl-4-(4-(p-tolyloxy)benzyl)-2-(4-(trifluoromethyl)phenyl)ox azole | white solid; ¹H NMR (400 MHz, CDCl₃) δ 2.32 (s, 6H), 3.86 (s, 2H), 6.88-6.93 (m, 4H), 7.12 (d, *J* = 8.0 Hz, 2H), 7.22 (d, *J* = 8.4 Hz, 2H), 7.67 (d, *J* = 8.4 Hz, 2H), 8.10 (d, *J* = 8.0 Hz, 2H); LCMS (ESI) m/z 424 [M + H]⁺. | >16 |
| 39 | 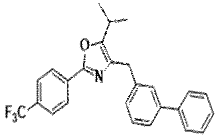 | 4-([1,1'-biphenyl]-3-ylmethyl)-5-isopropyl-2-(4-(trifluoromethyl)phenyl)ox azole | colorless oil; ¹H NMR (400 MHz, CDCl₃) δ 1.31 (s, 3H), 1.33 (s, 3H), 3.08-3.13 (m, 1H), 3.99 (s, 2H), 7.25 -7.32 (m, 1H), 7.33-7.46 (m, 5H), 7.51 (brs, 1H), 7.58 (d, *J* = 8.4 Hz, 2H), 7.68 (d, *J =* 8.8 Hz, 2H), 8.11 (d, *J =* 8.0 Hz, 2H); LCMS (ESI) m/z 422 [M + H]⁺. | >10 |
| 40 | 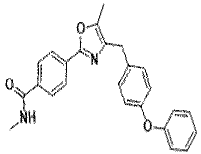 | N-methyl-4-(5-methyl-4-(4-phenoxybenzyl)oxazol-2-yl)benzamide | white solid; ¹H NMR (400 MHz, CDCl₃) δ 2.32 (s, 3H), 3.03 (d, *J* = 4.8 Hz, 3H), 3.87 (s, 2H), 6.15 (brs, 1H), 6.93-7.00 (m, 4H), 6.99 (t, *J* = 4.4 Hz, 1H), 7.24 (d, *J* = 8.8 Hz, 2H), 7.31 (t, *J* = 8.0 Hz, 2H), 7.81 (d, *J* = 8.4 Hz, 2H), 8.04 (d, *J* = 8.4 Hz, 2H); LCMS (ESI) m/z 399 [M + H]⁺. | >10 |
| 41 | 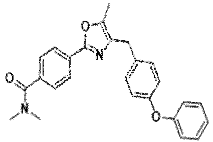 | N,N-dimethyl-4-(5-methyl-4-(4-phenoxybenzyl)oxazol-2-yl)benzamide | yellow solid; ¹H NMR (400 MHz, CDCl₃) δ 2.31 (s, 3H), 2.98 (brs, 3H), 3.12 (brs, 3H), 3.86 (s, 2H), 6.93-7.00 (m, 4H), 7.07 (t, *J =* 7.4Hz, 1H), 7.24 (d, *J* = 8.8 Hz, 2H), 7.31 (t, *J* = 7.0 Hz, 2H), 7.48 (d, *J* = 8.0 Hz, 2H), 8.02 (d, *J* = 8.4 Hz, 2H); LCMS (ESI) m/z 413 [M + H]⁺. | >10 |
| 42 | 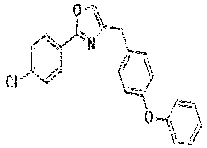 | 2-(4-chlorophenyl)-4-(4-phenoxybenzyl) oxazole | white solid; ¹H NMR (400 MHz, CDCl₃) δ 3.92 (s, 2H), 6.96-7.02 (m, 4H), 7.09 (t, *J =* 7.4 Hz, 1H), 7.27 (d, *J* = 8.4 Hz, 2H), 7.31-7.35 (m, 3H), 7.42 (d, *J* = 8.8 Hz, 2H), 7.96 (d, *J* = 8.8 Hz, 2H); LCMS (ESI) m/z 362 [M + H]⁺. | >10 |
| 43 | 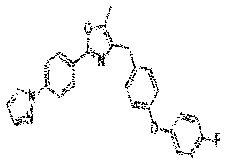 | 2-(4-(1H-pyrazol-1-yl)phenyl)-4-(4-(4-fluorophenoxy)benzyl)-5-methyloxazole | white solid; ¹H NMR (400 MHz, Acetone-*d₆*) δ 2.40 (s, 3H), 3.87 (s, 2H), 6.55 (s, 1H), 6.93 (d, *J* = 8.8 Hz, 2H), 7.03-7.04 (m, 2H), 7.14 (t, *J* = 8.6 Hz, 2H), 7.34 (d, *J* = 8.4 Hz, 2H), 7.74 (s, 1H), 7.98 (d, *J* = 8.4 Hz, 2H), 8.07 (d, *J =* 8.8 Hz, 2H), 8.42 (s, 1H); LCMS (ESI) m/z 426 [M + H]⁺. | 4 |
| 44 | 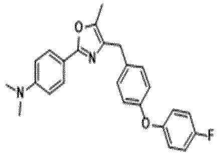 | 4-(4-(4-(4-fluorophenoxy)benzyl)-5-methyloxazol-2-yl)-N, N-dimethylaniline | Pale orange solid; ¹H NMR (400 MHz, Acetone-*d₆*) δ 2.34 (s, 3H), 3.01 (s, 6H), 3.81 (s, 2H), 6.78 (d, *J* = 8.8 Hz, 2H), 6.91 (d, *J =* 8.8 Hz, 2H), 7.00-7.03 (m, 2H), 7.13 (t, *J =* 8.8 Hz, 2H), 7.32 (d, *J =* 8.4 Hz, 2H), 7.79 (d, *J* = 8.8 Hz, 2H); LCMS (ESI) m/z 403 [M + H]⁺. | 4 |
| 45 | 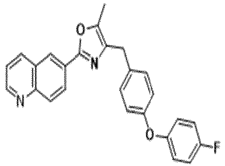 | 4-(4-(4-fluorophenoxy)benzyl)-5-methyl-2-(quinolin-6-yl)oxazole | pale yellow solid; ¹H NMR (400 MHz, Acetone-*d₆*) δ 2.45 (s, 3H), 3.91 (s, 2H), 6.94 (d, *J* = 8.4 Hz, 2H), 7.01-7.04 (m, 2H), 7.14 (t, *J* = 8.6 Hz, 2H), 7.36 (d, *J* = 8.4 Hz, 2H), 7.56-7.58 (m, 1H), 8.12 (d, *J* = 8.8 Hz, 1H), 8.32 (d, *J* = 7.6 Hz, 1H), 8.46 (d, *J* = 8.4 Hz, 1H), 8.51 (s, 1H), 8.95 (brs, 1H); LCMS (ESI) m/z 411 [M + H]⁺. | 4 |
| 46 | 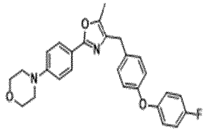 | 4-(4-(4-(4-(4-fluorophenoxy)benzyl)-5-methyloxazol-2-yl)phenyl)morpholine | white solid; ¹H NMR (400 MHz, DMSO-*d₆*) δ 2.34 (s, 3H), 3.20 (s, 4H), 3.73 (s, 4H), 3.78 (s, 2H), 6.92 (d, *J* = 8.4 Hz, 2H), 6.99-7.03 (m, 4H), 7.19 (d, *J* = 9.2 Hz, 2H), 7.27 (d, *J* = 8.4 Hz, 2H), 7.73 ( d, *J* = 8.8 Hz, 2H); LCMS (ESI) m/z 445 [M + H]⁺. | 2 |
| 47 | 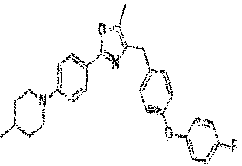 | 4-(4-(4-fluorophenoxy)benzyl)-5-methyl-2-(4-(4-methylpiperidin-1-yl)phenyl)oxazole | yellow solid; ¹H NMR (400 MHz, DMSO-*d₆*) δ 0.93 (d, *J* = 6.4 Hz, 3H), 1.14-1.24(m, 2H), 1.56 (brs, 1H), 1.67-1.70 (m, 2H), 2.34 (s, 3H), 2.72-2.78 (m, 2H), 3.78 (s, 2H), 3.82 (brs, 2H), 6.93 (d, *J* = 8.8 Hz, 2H), 6.97-7.04 (m, 4H), 7.20 (t, *J* = 8.8 Hz, 2H), 7.27 (d, *J =* 8.0 Hz, 2H), 7.69 (d, *J* = 8.8 Hz, 2H); LCMS (ESI) m/z 457 [M + H]+. | >16 |
| 49 | 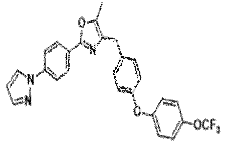 | 2-(4-(1H-pyrazol-1-yl)phenyl)-5-methyl-4-(4-(4-(trifluoromethoxy)phenoxy )benzyl)oxazole | pale yellow powder; ¹H NMR (400 MHz, DMSO-*d₆*) δ 2.40 (s, 3H), 3.86 (s, 2H), 6.58 (s, 1H), 6.99-7.07 (m, 4H), 7.32-7.36 (m, 4H), 7.79 (s, 1H), 7.99 (brs, 4H), 8.58 (s, 1H); LCMS (ESI) m/z 492 [M + H]⁺. | 2 |
| 51 | 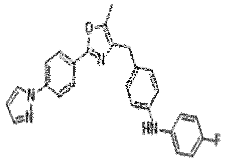 | 4-((2-(4-(1 H-pyrazol-1-yl)phenyl)-5-methyloxazol-4-yl)methyl)-N-(4-fluorophenyl)aniline | yellow solid; ¹H NMR (400 MHz, DMSO-*d₆*) δ 2.38 (s, 3H), 3.75 (s, 2H), 6.58 (s, 1H), 6.95 (d, *J* = 8.0 Hz, 2H), 7.02 -7.30 (m, 4H), 7.13 (d, *J* = 8.4 Hz, 2H), 7.79 (s, 1H), 7.99 (brs, 4H), 8.59 (s, 1H); LCMS (ESI) m/z 425 [M + H]⁺. | 4 |
| 52 | 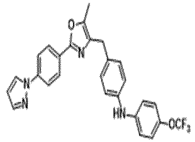 | 4-((2-(4-(1H-pyrazol-1-yl)phenyl)-5-methyloxazol-4-yl)methyl)-N-(4-(trifluoromethoxy)phenyl)a niline | yellow solid; ¹H NMR (400 MHz, DMSO-*d₆*) δ 2.41 (s, 3H), 3.84 (s, 2H), 6.59 (s, 1H), 7.02-7.06 (m, 4H), 7.26-7.29 (m, 4H), 7.79 (s, 1H), 7.99 (brs, 4H), 8.59 (s, 1H); LCMS (ESI) m/z 491 [M + H]⁺. | >16 |
| 53 | 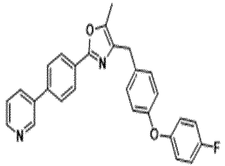 | 4-(4-(4-fluorophenoxy)benzyl)-5-methyl-2-(4-(pyridin-3-yl)phenyl)oxazole | white solid; ¹H NMR (400 MHz, DMSO-*d₆*) δ 2.40 (s, 3H), 3.85 (s, 2H), 6.93 (d, *J* = 8.4 Hz, 2H), 7.01 - 7.04 (m, 2H), 7.20 (t, *J* = 8.8 Hz, 2H), 7.30 (d, *J* = 8.4 Hz, 2H), 7.50 - 7.53 (m, 1H), 7.88 (d, *J* = 8.0 Hz, 2H), 8.01 (d, *J* = 8.4 Hz, 2H), 8.14 (d, *J* = 8.4 Hz, 1H), 8.60 (d, *J* = 8.0 Hz, 1H), 8.95 (s, 1H); LCMS (ESI) m/z 437 [M + H]⁺. | 2 |
| 54 | 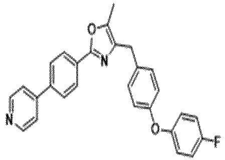 | 4-(4-(4-fluorophenoxy)benzyl)-5-methyl-2-(4-(pyridin-4-yl)phenyl)oxazole | pale yellow solid; ¹H NMR (400 MHz, DMSO-*d₆*) δ 2.40 (s, 3H), 3.85 (s, 2H), 6.93 (d, *J* = 8.0 Hz, 2H), 7.03 (t, *J* = 4.4 Hz, 2H), 7.20 (t, *J* = 8.4 Hz, 2H), 7.30 (d, *J* = 8.0 Hz, 2H), 7.76 (d, *J* = 4.4 Hz, 2H), 7.94 (d, *J* = 8.0 Hz, 2H), 8.03 (d, *J* = 8.0 Hz, 2H), 8.66 (d, *J* = 4.4 Hz, 2H); LCMS (ESI) m/z 437 [M + H]⁺. | 1 |
| 55 | 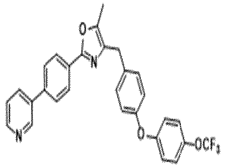 | 5-methyl-2-(4-(pyridin-3-yl)phenyl)-4-(4-(4-(trifluoromethoxy)phenoxy )benzyl)oxazole | pale yellow solid; ¹H NMR (400 MHz, Acetone-*d₆*) δ 2.44 (s, 3H), 3.92 (s, 2H), 7.02 (d, *J* = 8.4 Hz, 2H), 7.09 (d, *J* = 8.8 Hz, 2H), 7.34 (d, *J* = 8.8 Hz, 2H), 7.41 (d, *J* = 8.0 Hz, 2H), 7.48 - 7.51 (m, 1H), 7.87 (d, *J* = 8.4 Hz, 2H), 8.11 - 8.13 (m, 3H), 8.62 (d, *J* = 3.6 Hz, 1H), 8.95 (s, 1H); LCMS (ESI) m/z 503 [M + H]⁺. | 8 |
| 56 | 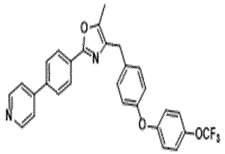 | 5-methyl-2-(4-(pyridin-4-yl)phenyl)-4-(4-(4-(trifluoromethoxy)phenoxy )benzyl)oxazole | pale yellow solid; ¹H NMR (400 MHz, Acetone-*d₆*) δ 2.43 (s, 3H), 3.91 (s, 2H), 7.00 (d, *J* = 8.4 Hz, 2H), 7.08 (d, *J* = 9.2 Hz,2H), 7.33 (d, *J* = 8.8 Hz, 2H), 7.39 (d, *J* = 8.0 Hz, 2H), 7.71 (d, *J* = 5.6 Hz, 2H), 7.92 (d, *J* = 8.8 Hz, 2H), 8.11 (d, *J* = 8.4 Hz, 2H), 8.67 (s, 2H); LCMS (ESI) m/z 503 [M + H]⁺. | 8 |
| 57 | 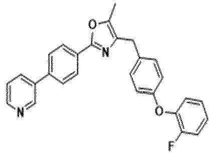 | 4-(4-(2-fluorophenoxy)benzyl)-5-methyl-2-(4-(pyridin-3-yl)phenyl)oxazole | pale yellow solid; ¹H NMR (400 MHz, Acetone-*d₆*) δ 2.41 (s, 3H), 3.88 (s, 2H), 6.92 (d, *J* = 8.8Hz, 2H), 7.09 - 7.35 (m, 4H), 7.48 (q, *J* = 4.8 Hz, 1H), 7.84 (d, *J* = 8.4 Hz, 2H), 8.08 - 8.10 (m, 3H), 8.60 (d, *J* = 4.4 Hz, 1H), 8.93 (s, 1H); LCMS (ESI) m/z 437 [M + H]+. | 16 |
| 58 | 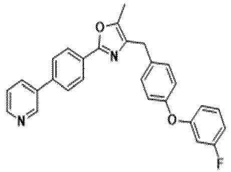 | 4-(4-(3-fluorophenoxy)benzyl)-5-methyl-2-(4-(pyridin-3-yl)phenyl)oxazole | pale yellow solid; ¹H NMR (400 MHz, Acetone-*d₆*) δ 2.45 (s, 3H), 3.93 (s, 2H), 6.75 (d, *J* = 10.4 Hz, 1H), 6.81 (d, *J* = 8.4 Hz, 1H), 6.87 (t, *J* = 8.4 Hz, 1H), 7.03 (d, *J* = 8.0 Hz, 2H), 7.40 - 7.43 (m, 3H), 7.49 (t, *J* = 4.8 Hz, 1H), 7.86 (d, *J* = 8.0 Hz, 2H), 8.11 - 8.13 (m, 3H), 8.61 (s, 1H), 8.95 (s, 1H); LCMS (ESI) m/z 437 [M + H]⁺. | 8 |
| 59 | 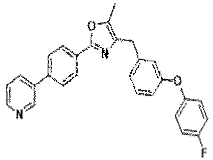 | 4-(3-(4-fluorophenoxy)benzyl)-5-methyl-2-(4-(pyridin-3-yl)phenyl)oxazole | pale yellow solid; ¹H NMR (400 MHz, Acetone-*d₆*) δ 2.41 (s, 3H), 3.90 (s, 2H), 6.83 (d, *J* = 8.0 Hz, 1H), 7.04 - 7.18 (m, 6H), 7.31 (t, *J* = 7.6 Hz, 1H), 7.50 (q, *J* = 4.8 Hz, 1H), 7.86 (d, *J* = 8.4 Hz, 2H), 8.08 - 8.13 (m, 3H), 8.62 (d, *J* = 3.6 Hz, 1H), 8.96 (s, 1H); LCMS (ESI) m/z 437 [M + H]⁺. | 8 |
| 60 | 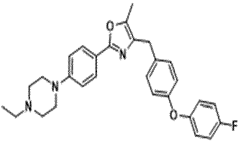 | 2-(4-(4-ethylpiperazin-1-yl)phenyl)-4-(4-(4-fluorophenoxy)benzyl)-5-methyloxazole | pale pink solid; ¹H NMR (400 MHz, Acetone-*d₆*) δ 0.87 (t, *J* = 7.2 Hz, 3H), 2.36 (s, 3H), 2.42 (q, *J* = 6.8 Hz, 2H), 2.56 (s, 4H), 3.30 (s, 4H), 3.83 (s, 2H), 6.93 (d, *J* = 8.4 Hz, 2H), 7.01 - 7.05 (m, 4H), 7.12 - 7.17 (m, 2H), 7.33 (d, *J* = 8.4 Hz, 2H), 7.82 (d, *J* = 9.2 Hz, 2H); LCMS (ESI) m/z 472 [M + H]⁺. | 16 |
| 61 | 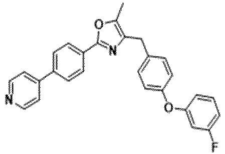 | 4-(4-(3-fluorophenoxy)benzyl)-5-methyl-2-(4-(pyridin-4-yl)phenyl)oxazole | pale yellow solid; ¹H NMR (400 MHz, Acetone-*d₆*) δ 2.41 (s, 3H), 3.90 (s, 2H), 6.83 (d, *J* = 8.0 Hz, 1H), 7.04 - 7.18 (m, 6H), 7.31 (t, *J* = 7.6 Hz, 1H), 7.75 (brs, 2H), 7.93 (d, *J* = 8.0 Hz, 2H), 8.10 (d, *J* = 8.0 Hz, 2H), 8.73 (brs, 2H); LCMS (ESI) m/z 437 [M + H]⁺. | 8 |
| 62 | 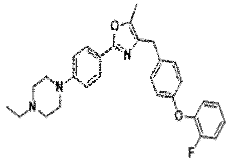 | 2-(4-(4-ethylpiperazin-1-yl)phenyl)-4-(4-(2-fluorophenoxy)benzyl)-5-methyloxazole | pale pink solid; ¹H NMR (400 MHz, Acetone-*d₆*) δ 1.09 (t, *J* = 7.2 Hz, 3H), 2.35 (s, 3H), 2.42 (q, *J* = 6.4 Hz, 2H), 2.57 (s, 4H), 3.30 (s, 4H), 3.83 (s, 2H), 6.92 (d, *J =* 8.8 Hz, 2H), 7.02 (d, *J =* 8.8 Hz, 2H), 7.12 (brs, 1H), 7.20 - 7.21 (m, 2H), 7.21 - 7.34 (m, 3H), 7.55 (d, J = 8.8 Hz, 2H); LCMS (ESI) m/z 472 [M + H]⁺. | >16 |
| 63 | 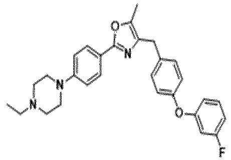 | 2-(4-(4-ethylpiperazin-1-yl)phenyl)-4-(4-(3-fluorophenoxy)benzyl)-5-methyloxazole | pale pink solid; ¹H NMR (400 MHz, Acetone-*d₆*) δ 1.09 (t, *J* = 7.2 Hz, 3H), 2.37 (s, 3H), 2.42 (q, *J* = 6.4 Hz, 2H), 2.57 (s, 4H), 3.31 (s, 4H), 3.86 (s, 2H), 6.74 (d, *J* = 10.4 Hz, 2H), 6.80 (d, *J* = 8.4 Hz, 1H), 6.87 (t, *J* = 8.4 Hz, 1H), 7.00 -7.17 (m, 4H), 7.36 - 7.41 (m, 3H), 7.83 (d, *J* = 8.8 Hz, 2H) ; LCMS (ESI) m/z 472 [M + H]⁺. | >16 |
| 64 | 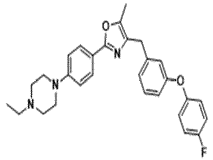 | 2-(4-(4-ethylpiperazin-1-yl)phenyl)-4-(3-(4-fluorophenoxy)benzyl)-5-methyloxazole | pale pink solid; ¹H NMR (400 MHz, Acetone-*d₆*) δ 1.10 (t, *J* = 7.2 Hz, 3H), 2.40 (s, 3H), 2.44 (q, *J* = 6.4 Hz, 2H), 2.60 (s, 4H), 3.32 (s, 4H), 3.83 (s, 2H), 6.81 (d, *J* = 7.2 Hz, 1H), 7.01 - 7.10 (m, 6H), 7.13 - 7.17 (m, 2H), 7.29 (d, *J* = 8.0 Hz, 1H), 7.80 (d, *J* = 8.4 Hz, 2H); LCMS (ESI) m/z 472 [M + H]⁺. | >16 |
| 65 | 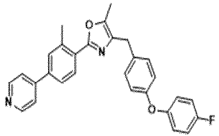 | 4-(4-(4-fluorophenoxy)benzyl)-5-methyl-2-(2-methyl-4-(pyridin-4-yl)phenyl)oxazole | pale yellow solid; ¹H NMR (400 MHz, Acetone-*d₆*) δ 2.34 (s, 3H), 2.40 (s, 3H), 3.88 (s, 2H), 6.93 (d, *J* = 7.2 Hz, 2H), 7.02 (brs, 2H), 7.11 - 7.15 (m, 2H), 7.33 - 7.40 (m, 5H), 7.88 (d, *J* = 8.0 Hz, 1H), 7.92 (s, 1H), 8.69 (brs, 2H); LCMS (ESI) m/z 451 [M + H]⁺. | 4 |
| 66 | 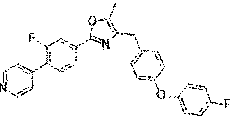 | 2-(3-fluoro-4-(pyridin-4-yl)phenyl)-4-(4-(4-fluorophenoxy)benzyl)-5-methyloxazole | ¹H NMR (400 MHz, Acetone-*d₆*) δ 2.44 (s, 3H), 3.91 (s, 2H), 6.95 (d, *J* = 8.4 Hz, 2H), 7.06 6.95 (m, 2H), 7.15 (t, *J* = 8.8 Hz,2H), 7.36 (d, *J* = 8.0 Hz, 2H), 7.61 - 7.58 (m, 2H), 7.83 - 7.76 (m, 2H), 7.93 (d, *J* = 8.0 Hz, 1H), 8.72 (d, *J* = 5.2 Hz, 2H); LCMS (ESI) m/z 455 [M + H]⁺. | 2 |
| 67 | 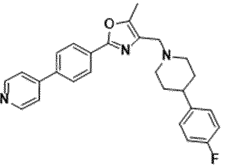 | 4-((4-(4-fluorophenyl)piperidin-1-yl)methyl)-5-methyl-2-(4-(pyridin-4-yl)phenyl)oxazole | ¹H NMR (400 MHz, CDCl₃) δ 1.88-1.80 (m, 4H), 2.23-2.18 (m, 2H), 2.46 (s, 3H), 2.54-2.50 (m, 1H), 3.16-3.13 (m, 2H), 3.54 (s, 2H), 7.00 (t, *J* = 8.8 Hz, 2H), 7.22-7.18 (m, 2H), 7.57 (d, *J* = 6.0 Hz, 2H), 7.74 (d, *J* = 8.4 Hz, 2H), 8.17 (d, *J* = 8.4 Hz, 2H), 7.71 (d, *J* = 5.6 Hz, 2H); LCMS (ESI) m/z 428 [M + H]⁺. | 4 |
| 68 | 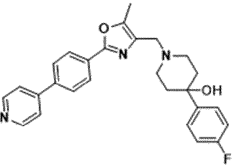 | 4-(4-fluorophenyl)-1-((5-methyl-2-(4-(pyridin-4-yl)phenyl)oxazol-4-yl)methyl)piperidin-4-ol | ¹H NMR (400 MHz, Acetone-*d₆*) δ 1.73-1.70 (m, 2H), 2.08-2.06 (m, 2H), 2.50 (s, 3H), 2.67-2.62 (m, 2H), 2.81 (m, 2H), 3.55 (s, 2H), 3.88 (brs, 1H), 7.08 (t, *J* = 8.8 Hz, 2H), 7.61-7.58 (m, 2H), 7.74 (dd, *J* = 4.8, 1.6 Hz, 2H), 7.95 (d, *J* = 8.4 Hz, 2H), 8.15 (d, *J* = 8.0 Hz, 2H), 8.69 (d, *J* = 6.0 Hz, 2H); LCMS (ESI) m/z 444 [M + H]⁺. | 16 |
| 69 | 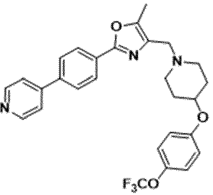 | 5-methyl-2-(4-(pyridin-4-yl)phenyl)-4-((4-(4-(trifluoromethoxy)phenoxy )piperidin-1-yl)methyl)oxazole | ¹H NMR (400 MHz, Acetone-*d₆*) δ 1.80-1.74 (m, 2H), 2.10 (m, 2H), 2.45-2.40 (m, 2H), 2.47 (s, 3H), 2.90-2.87 (m, 2H), 2.81 (m, 2H), 3.52 (s, 2H), 4.46-4.33 (m, 1H), 7.06 (dd, *J* = 6.8, 2.4 Hz, 2H), 7.24 (d, *J* = 8.8 Hz, 2H), 7.74 (dd, *J* = 4.8, 2.0 Hz, 2H), 7.95 (d, *J* = 8.4 Hz, 2H), 8.14 (d, *J* = 8.4 Hz, 2H), 8.69 (d, *J* = 6.0 Hz, 2H); LCMS (ESI) m/z 510 [M + H]⁺. | 2 |
| 70 | 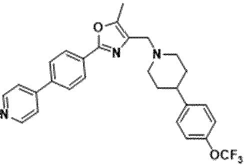 | 5-methyl-2-(4-(pyridin-4-yl)phenyl)-4-((4-(4-(trifluoromethoxy)phenyl)p iperidin-1-yl)methyl)oxazole | ¹H NMR (400 MHz, Acetone-*d₆*) δ 1.83-1.74 (m, 4H), 2.22-2.18 (m, 2H), 2.49 (s, 3H), 2.61 (m, 1H), 3.11-3.09 (m, 2H), 3.52 (s, 2H), 7.26 (d, *J* = 8.0 Hz, 2H), 7.41 (d, *J* = 8.4 Hz, 2H), 7.74 (dd, *J* = 4.4, 1.6 Hz, 2H), 7.96 (d, *J* = 8.8 Hz, 2H), 8.15 (d, *J* = 8.4 Hz, 2H), 8.69 (dd, J = 4.4, 1.6 Hz, 2H); LCMS (ESI) m/z 494 [M + H]⁺. | 2 |
| 71 | 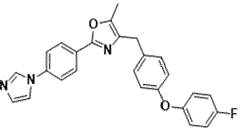 | 2-(4-(1H-imidazol-1-yl)phenyl)-4-(4-(4-fluorophenoxy)benzyl)-5-methyloxazole | ¹H NMR (400 MHz, Acetone-*d₆*) δ 2.43 (s, 3H), 3.89 (s, 2H), 6.94 (d, *J =* 8.8 Hz, 2H), 7.06-7.02 (m, 2H), 7.18-7.13 (m, 3H), 7.35 (d, *J* = 8.8 Hz, 2H), 7.68 (s, 1H), 7.77 (d, *J* = 8.8 Hz, 2H), 8.10 (d, *J* = 8.8 Hz, 2H), 8.18 (s, 1H); LCMS (ESI) m/z 426 [M + H]⁺. | |
| 72 | 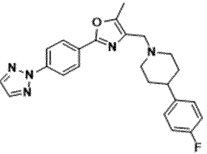 | 2-(4-(2H-1,2,3-triazol-2-yl)phenyl)-4-((4-(4-fluorophenyl)piperidin-1-yl)methyl)-5-methyloxazole | ¹H NMR (400 MHz, Acetone-*d₆*) δ 1.80-1.71 (m, 4H), 2.23-2.17 (m, 2H), 2.48 (s, 3H), 2.55 (m, 1H), 3.10-3.07 (m, 2H), 3.51 (s, 2H), 7.05 (t, *J* = 8.8 Hz, 2H), 7.33-7.29 (m, 2H), 8.05 (s, 2H), 8.19 (d, *J* = 8.8 Hz, 2H), 8.24 (d, *J* = 8.8 Hz, 2H); LCMS (ESI) m/z 418 [M + H]⁺. | >16 |
| 73 | 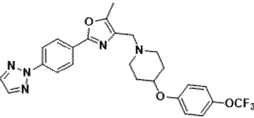 | 2-(4-(2H-1,2,3-triazol-2-yl)phenyl)-5-methyl-4-((4-(4-(trifluoromethoxy)phenoxy )piperidin-1-yl)methyl)oxazole | ¹H NMR (400 MHz, Acetone-*d₆*) δ 1.80-1.73 (m, 2H), 2.03 (m, 2H), 2.45-2.39 (m, 2H), 2.47 (s, 3H), 2.87-2.84 (m, 2H), 3.52 (s, 2H), 4.46-4.42 (m, 1H), 7.06 (d, *J* = 8.8 Hz, 2H), 7.25 (d, *J* = 8.8 Hz, 2H), 8.05 (s, 2H), 8.18 (d, *J* = 8.8 Hz, 2H), 8.24 (d, *J* = 8.8 Hz, 2H); LCMS (ESI) m/z 500 [M + H]⁺. | 8 |
| 74 | 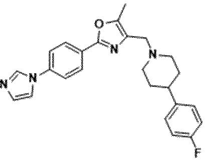 | 2-(4-(2H-1,2,3-triazol-2-yl)phenyl)-5-methyl-4-((4-(4-(trifluoromethoxy)phenoxy )piperidin-1-yl)methyl)oxazole | ¹H NMR (400 MHz, Acetone-*d₆*) δ 1.80-1.74 (m, 4H), 2.22-2.19 (m, 2H), 2.47 (s, 3H), 2.55 (m, 1H), 3.10-3.07 (m, 2H), 3.51 (s, 2H), 7.05 (t, *J =* 8.8 Hz, 2H), 7.16 (s, 1H), 7.32-7.29 (m, 2H), 7.69 (s, 1H), 7.79 (d, *J=* 8.8 Hz, 2H), 8.14 (d, *J* = 8.8 Hz, 2H), 8.19 (s, 1H); LCMS (ESI) m/z 417 [M + H]⁺. | 4 |
| 75 | 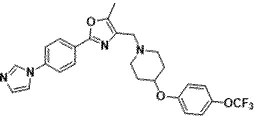 | 2-(4-(1H-imidazol-1-yl)phenyl)-5-methyl-4-((4-(4-(trifluoromethoxy)phenoxy )piperidin-1-yl)methyl)oxazole | ¹H NMR (400 MHz, Acetone-*d₆*) δ 1.80-1.72 (m, 2H), 2.05 (m, 2H), 2.44-2.39 (m, 2H), 2.46 (s, 3H), 2.86-2.84 (m, 2H), 3.51 (s, 2H), 4.45-4.43 (m, 1H), 7.06 (d, *J =* 8.8 Hz, 2H), 7.16 (s, 1H), 7.24 (d, *J* = 8.8 Hz, 2H), 7.69 (s, 1H), 7.78 (d, *J* = 8.8 Hz, 2H), 8.13 (d, *J* = 8.8 Hz, 2H), 8.19 (s, 1H); LCMS (ESI) m/z 499 [M + H]⁺. | 4 |
| 76 | 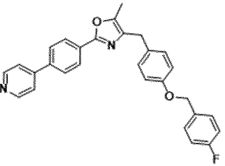 | 4-(4-((4-fluorobenzyl)oxy)benzyl)-5-methyl-2-(4-(pyridin-4-yl)phenyl)oxazole | ¹H NMR (400 MHz, DMSO-*d₆*) δ 2.39 (s, 3H), 3.76 (s, 2H), 5.83 (s, 2H), 6.69 (d, *J* = 8.4 Hz, 2H), 7.07 (d, *J* = 8.4 Hz, 2H), 7.32 (t, *J* = 8.8 Hz, 2H), 7.69-7.66 (m, 2H), 8.11 (d, *J =* 8.8 Hz, 2H), 8.20 (d, *J* = 8.8 Hz, 2H), 8.59 (d, *J* = 6.8 Hz, 2H), 9.25-9.20 (m, 2H); LCMS (ESI) m/z 451 [M + H]⁺. | 8 |
| 77 | 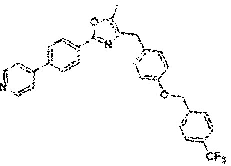 | 5-methyl-2-(4-(pyridin-4-yl)phenyl)-4-(4-((4-(trifluoromethyl)benzyl)ox y)benzyl)oxazole | ¹H NMR (400 MHz, DMSO-*d₆*) δ 2.39 (s, 3H), 3.76 (s, 2H), 5.96 (s, 2H), 6.69 (d, *J* = 8.4 Hz, 2H), 7.07 (d, *J* = 8.4 Hz, 2H), 7.76 (d, *J* = 8.0 Hz, 2H), 7.85 (d, *J* = 8.0 Hz, 2H), 8.12 (d, *J =* 8.4 Hz, 2H), 8.21 (d, *J* = 8.4 Hz, 2H), 8.62 (d, *J* = 6.8 Hz, 2H), 9.25 (d, *J* = 6.8 Hz, 2H); LCMS (ESI) m/z 501 [M + H]⁺. | 8 |
| 78 | 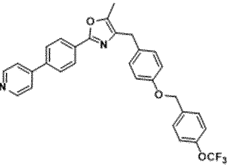 | 5-methyl-2-(4-(pyridin-4-yl)phenyl)-4-(4-((4-(trifluoromethoxy)benzyl)o xy)benzyl)oxazole | ¹H NMR (400 MHz, DMSO-*d₆*) δ 2.39 (s, 3H), 3.76 (s, 2H), 5.88 (s, 2H), 6.69 (d, *J* = 8.4 Hz, 2H), 7.07 (d, *J* = 8.4 Hz, 2H), 7.48 (d, *J* = 8.0 Hz, 2H), 7.71 (d, *J* = 8.8 Hz, 2H), 8.12 (d, *J =* 8.4 Hz, 2H), 8.21 (d, *J* = 8.4 Hz, 2H), 8.59 (d, *J* = 6.8 Hz, 2H), 9.25 (d, *J* = 7.2 Hz, 2H); LCMS (ESI) m/z 517 [M + H]⁺. | 8 |
| 79 | 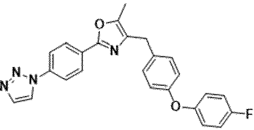 | 2-(4-(1H-1,2,3-triazol-1-yl)phenyl)-4-(4-(4-fluorophenoxy)benzyl)-5-methyloxazole | ¹H NMR (400 MHz, DMSO-*d₆*) δ 2.42 (s, 3H), 3.86 (s, 2H), 6.94 (d, *J =* 8.8 Hz, 2H), 7.05-7.02 (m, 2H), 7.23-7.19 (m, 2H), 7.31 (d, *J =* 8.8 Hz, 2H), 8.01 (s, 1H), 8.18-8.06 (m, 4H), 8.93 (s, 1H); LCMS (ESI) m/z 427 [M + H]⁺. | 2 |
| 80 | 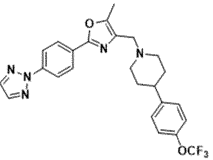 | 2-(4-(2H-1,2,3-triazol-2-yl)phenyl)-5-methyl-4-((4-(4-(trifluoromethoxy)phenyl)p iperidin-1-yl)methyl)oxazole | ¹H NMR (400 MHz, CD₃OD) δ 1.95-1.82 (m, 4H), 2.44-2.34 (m, 2H), 2.49 (s, 3H), 2.69-2.60 (m, 1H), 3.23-3.20 (m, 2H), 3.63 (s, 2H), 7.20 (d, *J* = 8.0 Hz, 2H), 7.35 (d, *J* = 8.8 Hz, 2H), 7.99 (s, 2H), 8.17 (d, *J =* 8.8 Hz, 2H), 8.23 (d, *J* = 8.8 Hz, 2H); LCMS (ESI) m/z 484 [M + H]⁺. | 8 |
| 81 | 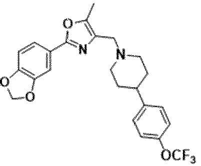 | 2-(benzo[d][1,3]dioxol-5-yl)-5-methyl-4-((4-(4-(trifluoromethoxy)phenyl)p iperidin-1-yl)methyl)oxazole | ¹H NMR (400 MHz, CD₃OD) δ 1.94-1.81 (m, 4H), 2.37-2.30 (m, 2H), 2.44 (s, 3H), 2.66-2.60 (m, 1H), 3.18-3.15 (m, 2H), 3.56 (s, 2H), 6.05 (s, 2H), 6.95 (d, *J* = 8.0 Hz, 1H), 7.20 (d, *J* = 8.0 Hz, 2H), 7.35 (d, *J* = 8.4 Hz, 2H), 7.45 (d, *J* = 1.6 Hz, 1H), 7.56 (dd, *J* = 8.4, 1.6 Hz, 1H); LCMS (ESI) m/z 461 [M + H]⁺. | 4 |
| 82 | 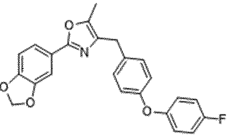 | 2-(benzo[d][1,3]dioxol-5-yl)-4-(4-(4-fluorophenoxy)benzyl)-5-methyloxazole | ¹H NMR (400 MHz, CD₃OD) δ 2.35 (s, 3H), 3.86 (s, 2H), 6.04 (s, 2H), 6.94-6.90 (m, 3H), 7.01-6.96 (m, 2H), 7.10-7.05 (m, 2H), 7.25 (d, *J* = 8.8 Hz, 2H), 7.41 (d, *J* = 1.6 Hz, 1H), 7.52 (dd, *J* = 8.4, 1.6 Hz, 1H); LCMS (ESI) m/z 404 [M + H]⁺. | 8 |
| 83 | 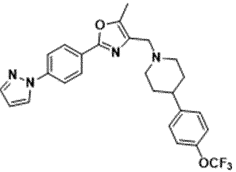 | 2-(4-(1H-pyrazol-1-yl)phenyl)-5-methyl-4-((4-(4-(trifluoromethoxy)phenyl)p iperidin-1-yl)methyl)oxazole | ¹H NMR (400 MHz, CD₃OD) δ 1.90-1.80 (m, 4H), 2.35-2.29 (m, 2H), 2.48 (s, 3H), 2.66-2.59 (m, 1H), 3.18-3.16 (m, 2H), 3.58 (s, 2H), 6.59-6.58 (m, 1H), 7.20 (d, *J* = 8.0 Hz, 2H), 7.35 (d, *J* = 8.4 Hz, 2H), 7.78 (d, *J* = 1.6 Hz, 1H), 7.93 (d, *J* = 8.8 Hz, 2H), 8.13 (d, *J* = 8.8 Hz, 2H), 8.34 (d, *J* = 2.8 Hz, 1H); LCMS (ESI) m/z 483 [M + H]⁺. | 8 |
| 84 | 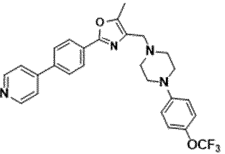 | 5-methyl-2-(4-(pyridin-4-yl)phenyl)-4-((4-(4-(trifluoromethoxy)phenyl)p iperazin-1-yl)methyl)oxazole | ¹H NMR (400 MHz, Acetone-*d₆*) δ 2.49 (s, 3H), 2.80-2.73 (m, 4H), 3.30-3.25 (m, 4H), 3.59 (s, 2H), 7.04 (d, *J* = 9.2 Hz, 2H), 7.18 (d, *J* = 9.2 Hz, 2H), 7.74 (d, *J* = 6.0 Hz, 2H), 7.95 (d, *J =* 8.8 Hz, 2H), 8.15 (d, *J* = 8.8 Hz, 2H), 8.69 (dd, *J* = 4.6, 1.6 Hz, 2H); LCMS (ESI) m/z 495 [M + H]⁺. | 4 |
| 85 | 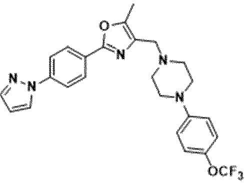 | 2-(4-(1H-pyrazol-1-yl)phenyl)-5-methyl-4-((4-(4-(trifluoromethoxy)phenyl)p iperazin-1-yl)methyl)oxazole | ¹H NMR (400 MHz, DMSO-*d₆*) δ 2.43 (s, 3H), 2.62 (m, 4H), 3.17 (m, 4H), 3.49 (s, 2H), 6.60 (m, 1H), 6.99 (d, *J* = 8.4 Hz, 2H), 7.18 (d, *J =* 8.4 Hz, 2H), 7.81 (s, 1H), 8.05-8.01 (m, 4H), 8.61 (m, 1H); LCMS (ESI) m/z 484 [M + H]⁺. | 8 |
| 86 | 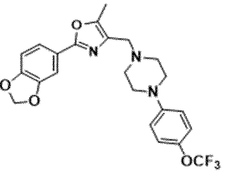 | 2-(benzo[d][1,3]dioxol-5-yl)-5-methyl-4-((4-(4-(trifluoromethoxy)phenyl)p iperazin-1-yl)methyl)oxazole | ¹H NMR (400 MHz, DMSO-*d₆*) δ 2.38 (s, 3H), 2.59 (m, 4H), 3.15 (m, 4H), 3.44 (s, 2H), 6.11 (s, 2H), 6.99 (d, *J* = 9.2 Hz, 2H), 7.04 (d, *J =* 8.4 Hz, 1H), 7.18 (d, *J* = 8.8 Hz, 2H), 7.38 (d, *J* = 1.6 Hz, 1H), 7.47 (dd, *J* = 8.4, 2.0 Hz, 1H); LCMS (ESI) m/z 462[M + H]⁺. | 8 |
| 87 | 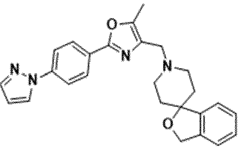 | 1'-((2-(4-(1H-pyrazol-1-yl)phenyl)-5-methyloxazol-4-yl)methyl)-3H-spiro[isobenzofuran-1,4'-piperidine] | ¹H NMR (400 MHz, DMSO-*d₆*) δ 1.65-1.62 (m, 2H), 1.95-1.89 (m, 2H), 2.43 (s, 3H), 2.82 (m, 2H), 3.48 (m, 2H), 4.97 (s, 2H), 6.61-6.60 (m, 1H), 7.27 (m, 4H), 7.81 (d, *J* = 1.6 Hz, 1H), 8.01 (d, *J* = 8.8 Hz, 2H), 8.05 (d, *J* = 9.2 Hz, 2H), 8.61 (d, *J* = 2.4 Hz, 1H); LCMS (ESI) m/z 427 [M + H]⁺. | 1 |
| 88 | 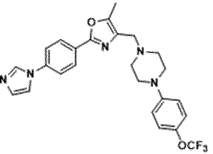 | 2-(4-(1 H-imidazol-1-yl)phenyl)-5-methyl-4-((4-(4-(trifluoromethoxy)phenyl)p iperazin-1-yl)methyl)oxazole | ¹H NMR (400 MHz, CD₃OD) δ 2.49 (s, 3H), 2.79-2.77 (m, 4H), 3.27-3.24 (m, 4H), 3.60 (s, 2H),7.02 (d, *J* = 7.2 Hz, 2H), 7.14 (d, *J* = 8.8 Hz, 2H), 7.21 (s, 1H), 7.70 (s, 1H), 7.76 (d, *J =* 8.8 Hz, 2H), 8.17 (d, *J* = 8.8 Hz, 2H), 8.28 (s, 1H); LCMS (ESI) m/z 484 [M + H]⁺. | 4 |
| 89 | 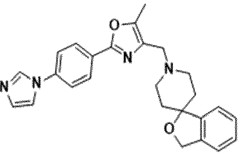 | 1'-((2-(4-(1H-imidazol-1-yl)phenyl)-5-methyloxazol-4-yl)methyl)-3H-spiro[isobenzofuran-1,4'-piperidine] | ¹H NMR (400 MHz, CD₃OD) δ 1.79-1.76 (m, 2H), 2.10-2.02 (m, 2H), 2.49 (s, 3H), 2.60-2.66 (m, 2H), 3.00-2.97 (m, 2H), 3.62 (s, 2H), 5.05(s, 2H), 7.22-7.19 (m, 2H), 7.30-7.25 (m, 3H), 7.69 (s, 1H), 7.76 (d, *J =* 8.8 Hz, 2H), 8.17 (d, *J* = 9.2 Hz, 2H), 8.27 (s, 1H); LCMS (ESI) m/z 427 [M + H]⁺. | 4 |
| 90 | 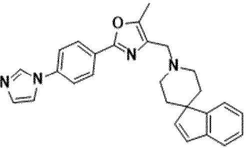 | 2-(4-(1H-imidazol-1-yl)phenyl)-5-methyl-4-(spiro[indene-1,4'-piperidin]-1'-ylmethyl)oxazole | ¹H NMR (400 MHz, CD₃OD) δ 1.38-1.34 (m, 2H), 2.28-2.21 (m, 2H), 2.51 (s, 3H), 2.64-2.59 (m, 2H), 3.16-3.13 (m, 2H), 3.67 (s, 2H), 6.78 (d, *J* = 5.6 Hz, 1H), 6.93 (d, *J* = 6.0 Hz, 1H), 7.23-7.16 (m, 3H), 7.30 (d, *J* = 8.0 Hz, 1H), 7.38 (d, *J =* 6.0 Hz, 1H), 7.70 (s, 1H), 7.76 (d, *J* = 8.4 Hz, 2H), 8.19 (d, *J* = 8.4 Hz, 2H), 8.28 (s, 1H); LCMS (ESI) m/z 427 [M + H]⁺. | 4 |
| 91 | 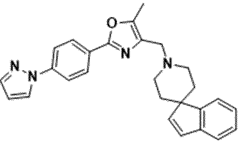 | 2-(4-(1H-pyrazol-1-yl)phenyl)-5-methyl-4-(spiro[indene-1,4'-piperidin]-1'-ylmethyl)oxazole | ¹H NMR (400 MHz, CD₃OD) δ 1.38-1.34 (m, 2H), 2.28-2.21 (m, 2H), 2.50 (s, 3H), 2.62-2.57 (m, 2H), 3.15-3.12 (m, 2H), 3.66 (s, 2H), 6.59 (m, 1H), 6.78 (d, *J* = 5.6 Hz, 1H), 6.93 (d, *J =* 6.0 Hz, 1H), 7.21-7.17 (m, 2H), 7.30 (d, *J* =8.0 Hz, 1H), 7.38 (d, *J =* 6.4 Hz, 1H), 7.78 (d, *J =* 1.6 Hz, 1H), 7.93 (d, *J* = 9.2 Hz, 2H), 8.15 (d, *J* = 8.8 Hz, 2H), 8.34 (d, *J* = 2.4 Hz, 1H); LCMS (ESI) m/z 423 [M + H]⁺. | 1 |
| 92 | 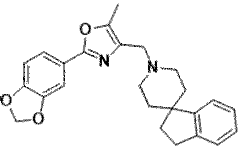 | 2-(benzo[d][1,3]dioxol-5-yl)-4-((2,3-dihydrospiro[indene-1,4'-piperidin]-1'-yl)methyl)-5-methyloxazole | ¹H NMR (400 MHz, CD₃OD) δ 1.58-1.54 (m, 2H), 2.02-1.98 (m, 2H), 2.04 (t, *J* =7.2 Hz, 2H), 2.43-2.37 (m, 2H), 2.43 (s, 3H), 2.89 (t, *J* =7.2 Hz, 2H), 3.01-2.98 (m, 2H), 3.54 (s, 2H), 6.05 (s, 2H), 6.94 (d, *J =* 8.4 Hz, 1H), 7.19-7.11 (m, 4H), 7.45 (d, *J* =1.6 Hz, 1H), 7.55 (dd, *J* =8.4, 1.6 Hz, 1H); LCMS (ESI) m/z 403 [M + H]⁺. | 0.5 |
| 93 | 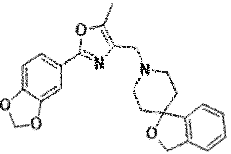 | 1'-((2-(benzo[d] [1,3]dioxol-5-yl)-5-methyloxazol-4-yl)methyl)-3H-spiro[isobenzofuran-1,4'-piperidine] | ¹H NMR (400 MHz, CD₃OD) δ 1.78-1.74 (m, 2H), 2.10-1.99 (m, 2H), 2.44 (s, 3H), 2.63-2.57 (m, 2H), 2.97-2.94 (m, 2H), 3.57 (s, 2H), 5.04 (s, 2H), 6.05 (s, 2H), 6.95 (d, *J* = 8.4 Hz, 1H), 7.19-7.15 (m, 1H), 7.29-7.25 (m, 3H), 7.45 (d, *J* = 1.2 Hz, 1H), 7.56 (dd, *J* = 8.4, 1.2 Hz, 1H); LCMS (ESI) m/z 405 [M + H]⁺. | 1 |
| 94 | 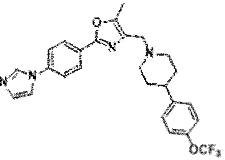 | 2-(4-(1H-imidazol-1-yl)phenyl)-5-methyl-4-((4-(4-(trifluoromethoxy)phenyl)p iperidin-1-yl)methyl)oxazole | ¹H NMR (400 MHz, CD₃OD) δ 1.95-1.88 (m, 4H), 2.39-2.32 (m, 2H), 2.49 (s, 3H), 2.68-2.60 (m, 1H), 3.20-3.15 (m, 2H), 3.60 (s, 2H), 7.21-7.19 (m, 3H), 7.35 (d, *J* = 8.8 Hz, 2H), 7.69 (s, 1H), 7.76 (d, *J* = 8.8 Hz, 2H), 8.17 (d, *J* = 8.8 Hz, 2H), 8.27 (s, 1H); LCMS (ESI) m/z 483 [M + H]⁺. | 1 |
| 95 | 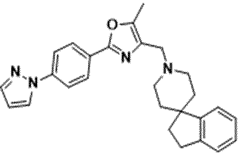 | 2-(4-(1H-pyrazol-1-yl)phenyl)-4-((2,3-dihydrospiro[indene-1,4'-piperidin]-1'-yl)methyl)-5-methyloxazole | ¹H NMR (400 MHz, CD₃OD) δ 1.59-1.56 (m, 2H), 2.03-1.98 (m, 2H), 2.06 (t, *J =* 7.2 Hz, 2H), 2.45-2.40 (m, 2H), 2.48 (s, 3H), 2.91 (t, *J* = 7.2 Hz, 2H), 3.04-3.01 (m, 2H), 3.58 (s, 2H), 6.59-6.58 (m, 1H), 7.20-7.12 (m, 4H), 7.78 (d, *J =* 2.0 Hz, 1H), 7.93 (d, *J* = 8.8 Hz, 2H), 8.14 (d, *J* = 8.8 Hz, 2H), 8.34 (d, *J* = 2.4 Hz, 1H); LCMS (ESI) m/z 425 [M + H]⁺. | 1 |
| 96 | 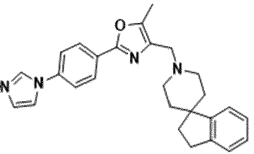 | 2-(4-(1H-imidazol-1-yl)phenyl)-4-((2,3-dihydrospiro[indene-1,4'-piperidin]-1'-yl)methyl)-5-methyloxazole | ¹H NMR (400 MHz, CD₃OD) δ 1.59-1.56 (m, 2H), 2.04-1.97 (m, 2H), 2.06 (t, *J* = 7.2 Hz, 2H), 2.46-2.40 (m, 2H), 2.49 (s, 3H), 2.91 (t, *J=* 7.2 Hz, 2H), 3.04-3.01 (m, 2H), 3.58 (s, 2H), 7.21-7.12 (m, 5H), 7.70 (s, 1H), 7.76 (d, *J* = 8.8 Hz, 2H), 8.19 (d, *J* = 8.8 Hz, 2H), 8.28 (s, 1H); LCMS (ESI) m/z 425 [M + H]⁺. | 4 |
| 97 | 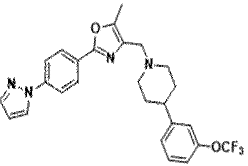 | 2-(4-(1H-pyrazol-1-yl)phenyl)-5-methyl-4-((4-(3-(trifluoromethoxy)phenyl)p iperidin-1-yl)methyl)oxazole | ¹H NMR (400 MHz, CD₃OD) δ 1.91-1.83 (m, 4H), 2.35-2.30 (m, 2H), 2.48 (s, 3H), 2.66-2.62 (m, 1H), 3.18-3.15 (m, 2H), 3.57 (s, 2H), 6.59-6.58 (m, 1H), 7.10 (d, *J* = 8.0 Hz, 1H), 7.16 (s, 1H), 7.27 (d, *J* = 8.0 Hz, 1H), 7.39 (t, *J* = 8.0 Hz, 1H), 7.78 (d, J = 1.6 Hz, 1H), 7.93 (d, *J* = 8.8 Hz, 2H), 8.13 (d, *J* = 9.2 Hz, 2H), 8.34 (d, *J* = 2.4 Hz, 1H); LCMS (ESI) m/z 483 [M + H]⁺. | 16 |
| 98 | 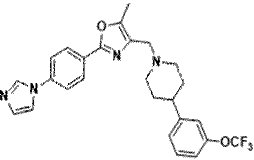 | 2-(4-(1 H-imidazol-1-yl)phenyl)-5-methyl-4-((4-(3-(trifluoromethoxy)phenyl)p iperidin-1-yl)methyl)oxazole | ¹H NMR (400 MHz, CD₃OD) δ 1.91-1.80 (m, 4H), 2.36-2.29 (m, 2H), 2.48 (s, 3H), 2.66-2.62 (m, 1H), 3.18-3.15 (m, 2H), 3.58 (s, 2H), 7.10 (d, *J* = 8.0 Hz, 1H), 7.16 (s, 1H), 7.21 (s, 1H), 7.27 (d, *J* =7.6 Hz, 1H), 7.39 (t, *J* = 8.0 Hz, 1H), 7.69 (d, *J* = 2.4 Hz, 1H), 7.75 (d, *J =* 8.8 Hz, 2H), 8.17 (d, *J* = 8.8 Hz, 2H), 8.27 (s, 1H); LCMS (ESI) m/z 483 [M + H]⁺. | 8 |
| 99 | 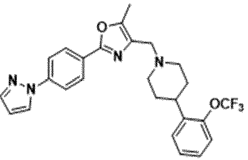 | 2-(4-(1H-pyrazol-1-yl)phenyl)-5-methyl-4-((4-(2-(trifluoromethoxy)phenyl)p iperidin-1-yl)methyl)oxazole | ¹H NMR (400 MHz, Acetone-*d₆*) δ 1.82-1.77 (m, 4H), 2.23-2.22 (m, 2H), 2.48 (s, 3H), 3.00-2.92 (m, 1H), 3.13-3.10 (m, 2H), 3.52 (s, 2H), 6.57 (m, 1H), 7.39-7.32 (m, 3H), 7.51 (dd, *J =* 7.6, 1.6 Hz, 1H), 7.76 (d, *J* = 1.6 Hz, 1H), 8.02 (d, *J* = 8.8 Hz, 2H), 8.12 (d, *J* = 8.8 Hz, 2H), 8.45 (d, *J =* 2.4 Hz, 1H); LCMS (ESI) m/z 483 [M + H]⁺. | 2 |
| 100 | 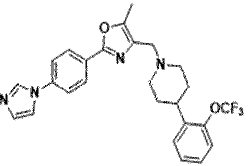 | 2-(4-(1H-imidazol-1-yl)phenyl)-5-methyl-4-((4-(2-(trifluoromethoxy)phenyl)p iperidin-1-yl)methyl)oxazole | ¹H NMR (400 MHz, Acetone-*d₆*) δ 1.84-1.77 (m, 4H), 2.25-2.19 (m, 2H), 2.48 (s, 3H), 2.97-2.92 (m, 1H), 3.13-3.10 (m, 2H), 3.52 (s, 2H), 7.17 (s, 1H), 7.40-7.30 (m, 3H), 7.51 (d, *J =* 7.6 Hz, 1H), 7.70 (brs, 1H), 7.79 (d, *J* = 8.4 Hz, 2H), 8.14 (d, *J* = 8.4 Hz, 2H), 8.20 (s,1H); LCMS (ESI) m/z 483 [M + H]⁺. | 8 |
| 101 | 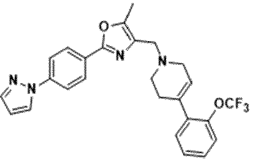 | 2-(4-(1H-pyrazol-1-yl)phenyl)-5-methyl-4-((4-(2-(trifluoromethoxy)phenyl)-3,6-dihydropyridin-1 (2H)-yl)methyl)oxazole | ¹H NMR (400 MHz, Acetone-*d₆*) δ 2.41-2.47 (m, 5H), 2.82-2.79 (m, 2H), 3.25-3.24 (m, 2H), 3.62 (s, 2H), 5.85-5.84 (m, 1H), 6.57 (t, *J =* 2.4 Hz, 1H), 7.40-7.34 (m, 4H), 7.76 (d, *J* = 1.6 Hz, 1H), 8.02 (d, *J* = 8.8 Hz, 2H), 8.12 (d, *J* = 8.8 Hz, 2H), 8.44 (d, *J* = 2.4 Hz, 1H); LCMS (ESI) m/z 481 [M + H]⁺. | 4 |
| 102 | 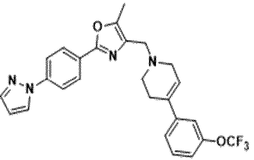 | 2-(4-(1H-pyrazol-1-yl)phenyl)-5-methyl-4-((4-(3-(trifluoromethoxy)phenyl)-3,6-dihydropyridin-1 (2H)-yl)methyl)oxazole | ¹H NMR (400 MHz, Acetone-*d₆*) δ 2.48 (s, 3H), 2.60-2.58 (m, 2H), 2.84-2.82 (m, 2H), 3.28-3.26 (m, 2H), 3.62 (s, 2H), 6.30-6.29 (m, 1H), 6.57 (t, *J* = 2.0 Hz, 1H), 7.24-7.18 (m,1H), 7.38 (m, 1H), 7.49-7.47 (m, 2H), 7.76 (d, *J* = 1.2 Hz, 1H), 8.02 (d, *J* = 8.8 Hz, 2H), 8.11 (d, *J* = 8.8 Hz, 2H), 8.45 (d, *J* = 2.8 Hz, 1H); LCMS (ESI) m/z 481 [M + H]⁺. | 16 |
| 103 | 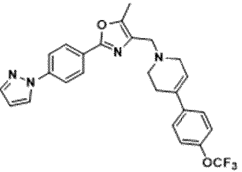 | 2-(4-(1H-pyrazol-1-yl)phenyl)-5-methyl-4-((4-(4-(trifluoromethoxy)phenyl)-3,6-dihydropyridin-1 (2H)-yl)methyl)oxazole | ¹H NMR (400 MHz, Acetone-*d₆*) δ 2.47 (s, 3H), 2.60-2.58 (m, 2H), 2.82-2.80 (m, 2H), 3.26-3.24 (m, 2H), 3.61 (s, 2H), 6.23-6.22 (m, 1H), 6.57 (m, 1H), 7.29 (d, *J* = 8.4 Hz, 2H), 7.57 (d, *J* = 8.4 Hz, 2H), 7.76 (s, 1H), 8.02 (d, *J =* 8.8 Hz, 2H), 8.11 (d, *J* = 8.8 Hz, 2H), 8.45 (s, 1H); LCMS (ESI) m/z 481 [M + H]⁺. | 4 |
| 104 | 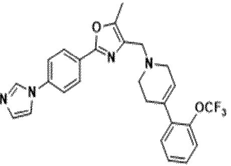 | 2-(4-(1H-imidazol-1-yl)phenyl)-5-methyl-4-((4-(2-(trifluoromethoxy)phenyl)-3,6-dihydropyridin-1 (2H)-yl)methyl)oxazole | ¹H NMR (400 MHz, Acetone-*d₆*) δ 2.51-2.48 (m, 5H), 2.82-2.80 (m, 2H), 3.25-3.24 (m, 2H), 3.61 (s, 2H), 5.85-5.84 (m, 1H), 7.16 (s, 1H), 7.41-7.32 (m, 4H), 7.70 (s, 1H), 7.80 (d, *J* = 8.8 Hz, 2H), 8.14 (d, *J =* 8.8 Hz, 2H), 8.20 (s, 1H); LCMS (ESI) m/z 481 [M + H]⁺. | 16 |
| 105 | 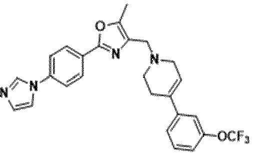 | 2-(4-(1H-imidazol-1-yl)phenyl)-5-methyl-4-((4-(3-(trifluoromethoxy)phenyl)-3,6-dihydropyridin-1 (2H)-yl)methyl)oxazole | ¹H NMR (400 MHz, Acetone-*d₆*) δ 2.48 (s, 3H), 2.60-2.58 (m, 2H), 2.85-2.82 (m, 2H), 3.28-3.26 (m, 2H), 3.63 (s, 2H), 6.30-6.29 (m, 1H), 7.16 (s, 1H), 7.23-7.22 (m, 1H), 7.37 (m, 1H), 7.49-7.47 (m, 2H), 7.69 (s, 1H), 7.79 (d, *J* = 8.8 Hz, 2H), 8.14 (d, *J* = 8.8 Hz, 2H), 8.19 (s, 1H); LCMS (ESI) m/z 481 [M + H]⁺. | 16 |
| 106 | 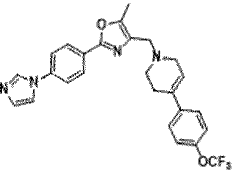 | 2-(4-(1H-imidazol-1-yl)phenyl)-5-methyl-4-((4-(4-(trifluoromethoxy)phenyl)-3,6-dihydropyridin-1 (2H)-yl)methyl)oxazole | ¹H NMR (400 MHz, Acetone-*d₆*) δ 2.48 (s, 3H), 2.59-2.57 (m, 2H), 2.81-2.79 (m, 2H), 3.26-3.25 (m, 2H), 3.62 (s, 2H), 6.24-6.22 (m, 1H), 7.16 (s, 1H), 7.29 (d, *J* = 8.4 Hz, 2H), 7.58 (d, *J* = 8.8 Hz, 2H), 7.70 (s, 1H), 7.79 (d, *J =* 8.8 Hz, 2H), 8.14 (d, *J* = 8.8 Hz, 2H), 8.20 (s, 1H); LCMS (ESI) m/z 481 [M + H]⁺. | 4 |
| 107 | 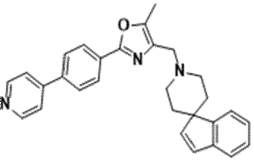 | 5-methyl-2-(4-(pyridin-4-yl)phenyl)-4-(spiro[indene-1,4'-piperidin]-1'-ylmethyl)oxazole | ¹H NMR (400 MHz, Acetone-*d₆*) δ 1.32-1.29 (m, 2H), 2.19-2.18 (m, 2H), 2.51 (s, 3H) ,2.54-2.51 (m, 2H), 3.09-3.06 (m, 2H), 3.63 (s, 2H), 6.79 (d, *J* = 6.0 Hz, 1H), 6.97 (d, *J* = 5.6 Hz, 1H), 7.22-7.19 (m, 2H), 7.33 (d, *J* = 6.4 Hz, 1H), 7.44 (dd, *J* = 8.4, 1.6 Hz, 1H), 7.75 (dd, *J* = 4.4, 1.6 Hz, 2H), 7.96 (d, *J* = 8.4 Hz, 2H), 8.16 (d, *J* = 8.8 Hz, 2H), 8.69 (dd, *J* = 4.4, 1.6 Hz, 1H); LCMS (ESI) m/z 434 [M + H]⁺. | 8 |
| 108 | 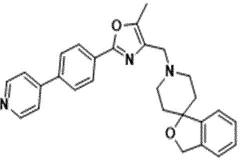 | 1'-((5-methyl-2-(4-(pyridin-4-yl)phenyl)oxazol-4-yl)methyl)-3H-spiro[isobenzofuran-1,4'-piperidine] | ¹H NMR (400 MHz, Acetone-*d₆*) δ 1.71-1.68 (m, 2H), 2.02-1.94 (m, 2H), 2.50 (s, 3H), 2.56-2.50 (m, 2H), 2.91 (m, 2H), 3.55 (s, 2H), 5.02 (s, 2H), 7.28-7.27 (m, 4H), 7.74 (dd, *J* = 6.0, 1.2 Hz, 2H), 7.96 (d, *J* = 8.0 Hz, 2H), 8.16 (d, *J* = 8.0 Hz, 2H), 8.69 (dd, *J* = 6.0, 0.8 Hz, 2H); LCMS (ESI) m/z 438 [M + H]⁺. | 4 |
| 109 | 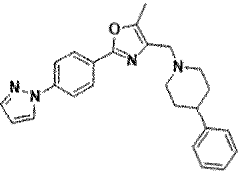 | 2-(4-(1H-pyrazol-1-yl)phenyl)-5-methyl-4-((4-phenylpiperidin-1-yl)methyl)oxazole | ¹H NMR (400 MHz, Acetone-*d₆*) δ 1.80-1.74 (m, 4H), 2.23-2.17 (m, 2H), 2.47 (s, 3H), 2.53-2.47 (m, 1H), 3.10-3.07 (m, 2H), 3.50 (s, 2H), 6.57 (t, *J* = 2.0 Hz, 1H), 7.20-7.18 (m, 1H), 7.32-7.26 (m, 4H), 7.76 (d, *J* = 1.2 Hz, 1H), 8.02 (d, *J* = 8.4 Hz, 2H), 8.11 (d, *J* = 8.8 Hz, 2H), 8.45 (d, *J* = 2.0 Hz, 1H); LCMS (ESI) m/z 399 [M + H]⁺. | 4 |
| 110 | 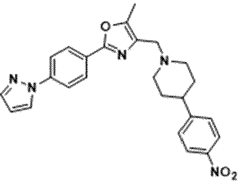 | 2-(4-(1H-pyrazol-1-yl)phenyl)-5-methyl-4-((4-(4-nitrophenyl)piperidin-1-yl)methyl)oxazole | ¹H NMR (400 MHz, Acetone-*d₆*) δ 1.86-1.81 (m, 4H), 2.24-2.21 (m, 2H), 2.47 (s, 3H), 2.78-2.68 (m, 1H), 3.13-3.10 (m, 2H), 3.52 (s, 2H), 6.57 (dd, *J* = 2.4, 1.6 Hz, 1H), 7.58 (d, *J* = 8.8 Hz, 2H), 7.76 (d, *J* = 1.6 Hz, 1H), 8.02 (d, *J =* 8.8 Hz, 2H), 8.11 (d, *J* = 8.8 Hz, 2H), 8.19 (d, *J* = 8.8 Hz, 2H), 8.45 (d, *J* = 2.4 Hz, 1H); LCMS (ESI) m/z 444 [M + H]⁺. | 16 |
| 111 | 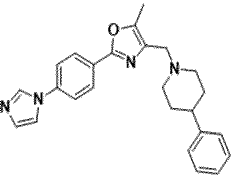 | 2-(4-(1H-imidazol-1-yl)phenyl)-5-methyl-4-((4-phenylpiperidin-1 - yl)methyl)oxazole | ¹H NMR (400 MHz, Acetone-d6) δ 1.84-1.68 (m, 4H), 2.23-2.13 (m, 2H), 2.46 (s, 3H), 2.56-2.50 (m, 1H), 3.07 (d, J = 11.5 Hz, 2H), 3.50 (s, 2H), 7.14 (s, 1H), 7.20-7.15 (m, 1H), 7.32-7.22 (m, 4H), 7.68 (s, 1H), 7.77 (dd, *J* = 6.8, 1.8 Hz, 2H), 8.12 (dd, *J* = 8.8, 1.8 Hz, 2H), 8.18 (s, 1H); LCMS (ESI) m/z 399 [M + H]⁺. | 16 |
| 112 | 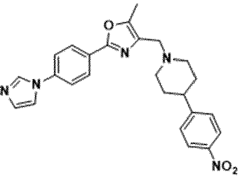 | 2-(4-(1H-imidazol-1-yl)phenyl)-5-methyl-4-((4-(4-nitrophenyl)piperidin-1-yl)methyl)oxazole | ¹H NMR (400 MHz, Acetone-*d₆*) δ 1.88-1.83 (m, 4H), 2.26-2.19 (m, 2H), 2.46 (s, 3H), 2.73-2.70 (m, 1H), 3.12-3.09 (m, 2H), 3.52 (s, 2H), 7.15 (s, 1H), 7.57 (d, *J* = 8.4 Hz, 2H), 7.68 (s, 1H), 7.78 (d, *J* = 8.8 Hz, 2H), 8.12 (d, *J* = 8.8 Hz, 2H), 8.19-8.16 (m, 3H); LCMS (ESI) m/z 444 [M + H]⁺. | 8 |
| 113 | 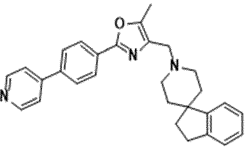 | 4-((2,3-dihydrospiro[indene-1,4'-piperidin]-1'-yl)methyl)-5-methyl-2-(4-(pyridin-4-yl)phenyl)oxazole | ¹H NMR (400 MHz, CD3OD) δ 1.54-1.45 (m, 2H), 1.95-1.84 (m, 2H), 2.03-1.99 (m, 2H), 2.34-2.26 (m, 2H), 2.48 (s, 3H), 2.97-2.86 (m, 4H), 3.51 (s, 2H), 7.23-7.08 (m, 4H), 7.73 (dd, *J* = 4.6, 1.8 Hz, 2H), 7.96-7.91 (m, 2H), 8.16-8.11 (m, 2H), 8.67 (dd, *J* = 4.6, 1.8 Hz, 2H); LCMS (ESI) m/z 436 [M + H]⁺. | 4 |
| 114 | 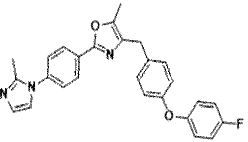 | 4-(4-(4-fluorophenoxy)benzyl)-5-methyl-2-(4-(2-methyl-1H-imidazol-1-yl)phenyl)oxazole | ¹H NMR (400 MHz, CD₃OD) δ 2.39 (s, 3H), 2.41 (s, 3H), 3.91 (s, 2H), 6.93 (d, *J* = 8.0 Hz, 2H), 7.01-6.97 (m, 5H), 7.07 (d, *J* = 8.0 Hz, 2H), 7.10 (s, 1H), 7.55 (d, *J* = 7.6 Hz, 2H), 8.14 (d, *J* = 7.2 Hz, 2H); LCMS (ESI) m/z 440 [M + H]⁺. | 8 |
| 115 | 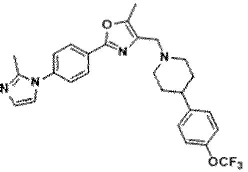 | 5-methyl-2-(4-(2-methyl-1H-imidazol-1-yl)phenyl)-4-((4-(4-(trifluoromethoxy)phenyl)p iperidin-1-yl)methyl)oxazole | ¹H NMR (400 MHz, CD₃OD) δ 1.94-1.87 (m, 4H), 2.37-2.30 (m, 2H), 2.41 (s, 3H), 2.49 (s, 3H), 2.66-2.60 (m, 1H), 3.19-3.16 (m, 2H), 3.59 (s, 2H), 7.02 (s, 1H), 7.20 (d, *J* = 8.4 Hz, 2H), 7.26 (s, 1H), 7.35 (d, *J=* 8.4 Hz, 2H), 7.57 (d, *J* = 8.4 Hz, 2H), 8.18 (d, *J* = 8.8 Hz, 2H); LCMS (ESI) m/z 497 [M + H]⁺. | 2 |
| 116 | 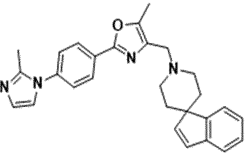 | 5-methyl-2-(4-(2-methyl-1H-imidazol-1-yl)phenyl)-4-(spiro[indene-1,4'-piperidin]-1'-ylmethyl)oxazole | ¹H NMR (400 MHz, CD₃OD) δ 1.38-1.31 (m, 2H), 2.28-2.21 (m, 2H), 2.41 (s, 3H), 2.51 (s, 3H), 2.66-2.57 (m, 2H), 3.16-3.13 (m, 2H), 3.67 (s, 2H), 6.78 (d, *J* = 4.8 Hz, 1H), 6.93 (d, *J* = 5.6 Hz, 1H),7.02 (d, *J* = 1.6 Hz, 1H), 7.23-7.18 (m, 2H), 7.27 (d, *J =* 1.6 Hz, 1H), 7.30 (d, *J* = 8.0 Hz, 1H), 7.38 (d, J = 6.4 Hz, 1H), 7.58 (d, J =8.8 Hz, 2H), 8.20 (d, *J* = 8.8 Hz, 2H); LCMS (ESI) m/z 437 [M + H]⁺. | 8 |
| 117 | 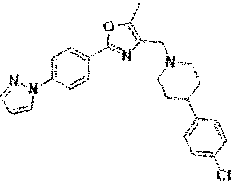 | 2-(4-(1H-pyrazol-1-yl)phenyl)-4-((4-(4-chlorophenyl)piperidin-1-yl)methyl)-5-methyloxazole | ¹H NMR (400 MHz, Acetone-*d₆*) δ 2.05-1.96 (m, 4H), 2.53 (s, 3H), 2.82 (m, 1H), 3.57-3.54 (m, 2H), 4.06 (s, 2H), 6.59-6.58 (m, 1H), 7.32 (d, *J* = 8.4 Hz, 2H), 7.35 (d, *J* = 8.8 Hz, 2H), 7.77 (d, *J* = 1.2 Hz, 1H), 8.04 (d, *J* = 8.8 Hz, 2H), 8.12 (d, *J* = 8.8 Hz, 2H), 8.45 (d, *J* = 2.4 Hz, 1H); LCMS (ESI) m/z 433 [M + H]⁺. | 8 |
| 118 | 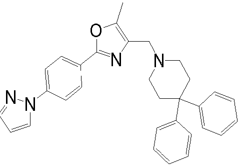 | 2-(4-(1H-pyrazol-1-yl)phenyl)-4-((4,4-diphenylpiperidin-1-yl)methyl)-5-methyloxazole | ¹H NMR (400 MHz, CD₃OD) δ 2.40 (s, 3H), 2.55 (m, 4H), 2.69 (m, 4H), 3.42 (s, 2H), 6.59-6.58 (m, 1H), 7.13 (t, *J* = 7.2 Hz, 2H), 7.36-7.24 (m, 8H), 7.78 (d, *J* = 1.6 Hz, 1H), 7.91 (d, *J* = 8.8 Hz, 2H), 8.10 (d, *J* = 8.8 Hz, 2H), 8.33 (d, *J* = 2.0 Hz, 1H); LCMS (ESI) m/z 475 [M + H]⁺. | 16 |
| 119 | 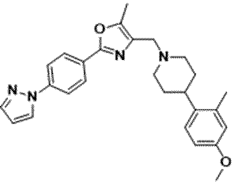 | 2-(4-(1H-pyrazol-1-yl)phenyl)-4-((4-(4-methoxy-2-methylphenyl)piperidin-1-yl)methyl)-5-methyloxazole | ¹H NMR (400 MHz, CD₃OD) δ 1.82-1.76 (m, 4H), 2.36-2.31 (m, 5H), 2.48 (s, 3H), 2.75-2.72 (m, 1H), 3.18-3.15 (m, 2H), 3.58 (s, 2H), 3.75 (s, 3H), 6.59-6.58 (m, 1H), 6.71-6.70 (m, 2H), 7.14 (d, *J* = 8.0 Hz, 1H), 7.78 (d, J = 1.6 Hz, 1H), 7.93 (d, *J* = 8.4 Hz, 2H), 8.14 (d, *J* = 8.4 Hz, 2H), 8.34 (d, *J* = 2.4 Hz, 1H); LCMS (ESI) m/z 443 [M + H]⁺. | 16 |
| 120 | 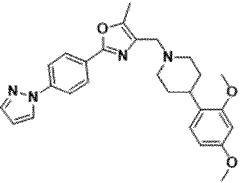 | 2-(4-(1H-pyrazol-1-yl)phenyl)-4-((4-(2,4-dimethoxyphenyl)piperidin -1-yl)methyl)-5-methyloxazole | ¹H NMR (400 MHz, CD₃OD) δ 1.81-1.76 (m, 4H), 2.36-2.29 (m, 2H), 2.48 (s, 3H), 2.91-2.87 (m, 1H), 3.17-3.15 (m, 2H), 3.58 (s, 2H), 3.77 (s, 3H), 3.82 (s, 3H), 6.50-6.46 (m, 2H), 6.59-6.58 (m, 1H), 7.08 (d, *J* =8.4 Hz, 1H), 7.78 (d, *J* = 1.6 Hz, 1H), 7.92 (d, *J* = 8.8 Hz, 2H), 8.14 (d, *J* = 8.8 Hz, 2H), 8.34 (d, *J* = 2.4 Hz, 1H); LCMS (ESI) m/z 459 [M + H]⁺. | 16 |
| 121 | 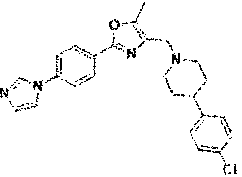 | 2-(4-(1H-imidazol-1-yl)phenyl)-4-((4-(4-chlorophenyl)piperidin-1-yl)methyl)-5-methyloxazole | ¹H NMR (400 MHz, Acetone-*d₆*) δ 1.91-1.79 (m, 4H), 2.31-2.29 (m, 2H), 2.48 (s, 3H), 2.69-2.57 (m, 1H), 3.18-3.15 (m, 2H), 3.60 (s, 2H), 7.17 (s, 1H), 7.30 (d, *J* = 8.8 Hz, 2H), 7.33 (d, *J* = 8.4 Hz, 2H), 7.70 (s, 1H), 7.80 (d, *J* = 8.4 Hz, 2H), 8.14 (d, *J* = 8.4 Hz, 2H), 8.20 (s, 1H); LCMS (ESI) m/z 433 [M + H]⁺. | 2 |
| 122 | 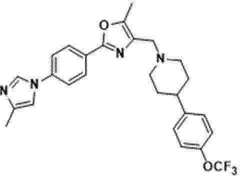 | 5-methyl-2-(4-(4-methyl-1H-imidazol-1-yl)phenyl)-4-((4-(4-(trifluoromethoxy)phenyl)p iperidin-1-yl)methyl)oxazole | 1H NMR (400 MHz, CD3OD) δ 2.13-1.76 (m, 4H), 2.23 (s, 3H), 2.34-2.30 (m, 1H), 2.47 (s, 3H), 2.67-2.57 (m, 1H), 3.20-3.11 (m, 2H), 3.56 (s, 2H), 7.19 (d, *J* = 8.6 Hz, 2H), 7.34 (d, *J* = 8.6 Hz, 2H), 7.39 (s, 1H), 7.70 (d, *J* = 8.6 Hz, 2H), 8.13 (s, 1H), 8.16-8.13 (m, 2H); LCMS (ESI) m/z 497 [M + H]⁺. | 1 |
| 123 | 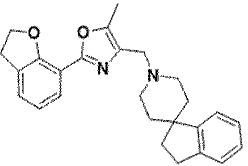 | 2-(2,3-dihydrobenzofuran-7-yl)-4-((2,3-dihydrospiro[indene-1,4'-piperidin]-1'-yl)methyl)-5-methyloxazole | ¹H NMR (400 MHz, CD₃OD) δ 1.61-1.52 (m, 2H), 2.05 (t, *J* = 7.2 Hz, 2H), 2.01-1.96 (m, 2H), 2.42-2.38 (m, 2H), 2.45 (s, 3H), 2.90 (t, *J* = 7.2 Hz, 2H), 3.02-2.99 (m, 2H), 3.30 (t, *J* = 8.8 Hz, 2H), 3.57 (s, 2H), 4.72 (t, *J =* 8.8 Hz, 2H), 6.95 (t, *J =* 7.6 Hz, 1H), 7.19-7.11 (m, 4H), 7.34 (d, *J* = 8.0 Hz, 1H), 7.71 (d, *J* = 8.0 Hz, 1H); LCMS (ESI) m/z 401 [M + H]⁺. | >16 |
| 124 | 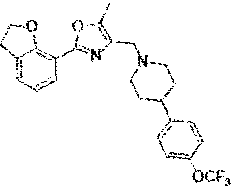 | 2-(2,3-dihydrobenzofuran-7-yl)-5-methyl-4-((4-(4-(trifluoromethoxy)phenyl)p iperidin-1-yl)methyl)oxazole | ¹H NMR (400 MHz, CD₃OD) δ 1.91-1.81 (m, 4H), 2.45-2.37 (m, 2H), 2.48 (s, 3H), 2.68-2.62 (m, 1H), 3.22-3.19 (m, 2H), 3.30 (t, *J =* 8.4 Hz, 2H), 3.64 (s, 2H), 4.72 (t, *J =* 8.8 Hz, 2H), 6.96 (t, *J* = 7.6 Hz, 1H), 7.20 (d, *J* = 8.4 Hz, 2H), 7.37-7.34 (m, 3H), 7.70 (d, *J* = 7.6 Hz, 1H); LCMS (ESI) m/z 459 [M + H]⁺. | >16 |
| 125 | 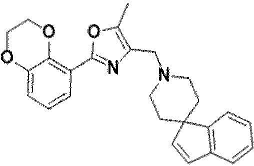 | 2-(2,3-dihydrobenzo[b][1,4]dioxi n-5-yl)-5-methyl-4-(spiro[indene-1,4'-piperidin]-1'-ylmethyl)oxazole | ¹H NMR (400 MHz, CD₃OD) δ 1.38-1.36 (m, 2H), 2.25-2.18 (m, 2H), 2.47 (s, 3H), 2.61-2.58 (m, 2H), 3.13-3.10 (m, 2H), 3.65 (s, 2H), 4.34-4.32 (m, 2H), 4.39-4.37 (m, 2H), 6.77 (d, *J =* 5.6 Hz, 1H), 6.95-6.91 (m, 2H), 6.98 (d, *J =* 2.0 Hz, 1H), 7.21-7.17 (m, 2H), 7.29 (d, *J* = 6.8 Hz, 1H), 7.36 (d, *J* = 6.8 Hz, 1H), 7.42 (dd, *J* = 7.6, 1.6 Hz, 1H); LCMS (ESI) m/z 415 [M + H]⁺. | >16 |
| 126 | 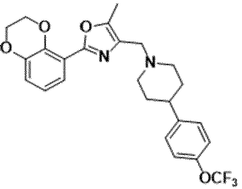 | 2-(2,3-dihydrobenzo[b][1,4]dioxi n-5-yl)-5-methyl-4-((4-(4-(trifluoromethoxy)phenyl)p iperidin-1-yl)methyl)oxazole | ¹H NMR (400 MHz, CD₃OD) δ 1.88-1.82 (m, 4H), 2.33-2.26 (m, 2H), 2.44 (s, 3H), 2.65-2.59 (m, 1H), 3.16-3.13 (m, 2H), 3.56 (s, 2H), 4.34-4.32 (m, 2H), 4.38-4.36 (m, 2H), 6.92 (t, *J =* 8.0 Hz, 1H), 6.98 (d, *J* = 8.0 Hz, 1H), 7.19 (d, *J* = 8.0 Hz, 2H), 7.35 (d, *J* = 8.4 Hz, 2H), 7.40 (dd, *J* = 7.6, 1.6 Hz, 1H); LCMS (ESI) m/z 475 [M + H]⁺. | >16 |
| 127 | 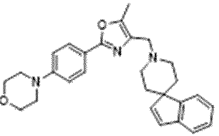 | 4-(4-(5-methyl-4-(spiro[indene-1,4'-piperidin]-1'-ylmethyl)oxazol-2-yl)phenyl)morpholine | ¹H NMR (400 MHz, CD₃OD) δ 1.35-1.32 (m, 2H), 2.26-2.19 (m, 2H), 2.44 (s, 3H), 2.60-2.54 (m, 2H), 3.12-3.09 (m, 2H), 3.28-3.26 (m, 4H), 3.61 (s, 2H), 3.86-3.84 (m, 4H), 6.76 (d, *J =* 5.6 Hz, 1H), 6.90 (d, *J* = 5.6 Hz, 1H), 7.05 (d, *J =* 8.8 Hz, 2H), 7.19-7.16 (m, 2H), 7.28 (d, *J =* 6.8 Hz, 1H), 7.36 (d, *J* = 7.2 Hz, 1H), 7.89 (d, *J =* 8.8 Hz, 2H); LCMS (ESI) m/z 442 [M + H]⁺. | 0.25 |
| 128 | 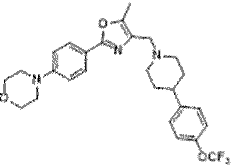 | 4-(4-(5-methyl-4-((4-(4-(trifluoromethoxy)phenyl)p iperidin-1-yl)methyl)oxazol-2-yl)phenyl)morpholine | ¹H NMR (400 MHz, CD₃OD) δ 1.87-1.78 (m, 4H), 2.31-2.25 (m, 2H), 2.42 (s, 3H), 2.63-2.57 (m, 1H), 3.14-3.11 (m, 2H), 3.27-3.25 (m, 4H), 3.51 (s, 2H), 3.86-3.84 (m, 4H), 7.04 (d, *J =* 8.8 Hz, 2H), 7.18 (d, *J* = 8.4 Hz, 2H), 7.33 (d, *J =* 8.8 Hz, 2H), 7.87 (d, *J* = 8.8 Hz, 2H); LCMS (ESI) m/z 502 [M + H]⁺. | 1 |
| 129 | 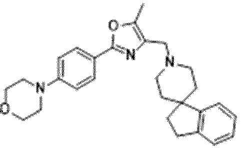 | 4-(4-(4-((2,3-dihydrospiro[indene-1,4'-piperidin]-1'-yl)methyl)-5-methyloxazol-2-yl)phenyl)morpholine | ¹H NMR (400 MHz, CD₃OD) δ 1.59-1.55 (m, 2H), 2.03-1.92 (m, 2H), 2.05 (t, *J=* 7.2 Hz, 2H), 2.45-2.40 (m, 5H), 2.90 (t, *J* = 7.2 Hz, 2H), 3.03-3.00 (m, 2H), 3.29-3.26 (m, 4H), 3.56 (s, 2H), 3.87-3.85 (m, 4H), 7.06 (d, *J* = 8.8 Hz, 2H), 7.19-7.12 (m, 4H), 7.89 (d, *J* = 8.8 Hz, 2H); LCMS (ESI) m/z 444 [M + H]⁺. | 0.5 |
| 130 | 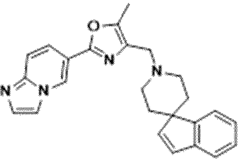 | 2-(imidazo[1,2-a]pyridin-6-yl)-5-methyl-4-(spiro[indene-1,4'-piperidin]-1'-ylmethyl)oxazole | ¹H NMR (400 MHz, CD₃OD) δ 1.38-1.34 (m, 2H), 2.27-2.21 (m, 2H), 2.50 (s, 3H), 2.66-2.60 (m, 2H), 3.15-3.13 (m, 2H), 3.66 (s, 2H), 6.78 (d, *J* = 6.0 Hz, 1H), 6.93 (d, *J* = 5.6 Hz, 1H), 7.23-7.16 (m, 2H), 7.30 (d, *J* = 6.4 Hz, 1H), 7.38 (d, *J =* 6.4 Hz, 1H), 7.66 (s, 1H), 7.69 (d, *J* = 9.6 Hz, 1H), 7.93 (dd, *J* = 9.6, 1.6 Hz, 1H), 8.00 (s, 1H), 9.16 (s, 1H); LCMS (ESI) m/z 397 [M + H]⁺. | >16 |
| 131 | 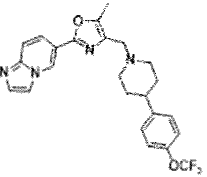 | 2-(imidazo[1,2-a]pyridin-6-yl)-5-methyl-4-((4-(4-(trifluoromethoxy)phenyl)p iperidin-1-yl)methyl)oxazole | ¹H NMR (400 MHz, CD₃OD) δ 2.03-1.83 (m, 4H), 2.36-2.30 (m, 2H), 2.48 (s, 3H), 2.64-2.60 (m, 1H), 3.19-3.16 (m, 2H), 3.58 (s, 2H), 7.20 (d, *J* = 8.4 Hz, 2H), 7.35 (d, *J* = 8.4 Hz, 2H), 7.69-7.67 (m, 2H), 7.91 (dd, *J* = 9.2, 1.6 Hz, 1H), 8.00 (s, 1H), 9.15 (s, 1H); LCMS (ESI) m/z 457 [M + H]⁺. | 4 |
| 132 | 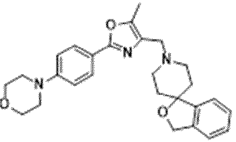 | 1'-((5-methyl-2-(4-morpholi nophenyl)oxazol-4-yl)methyl)-3H-spiro[isobenzofuran-1,4'-piperidine] | ¹H NMR (400 MHz, CD₃OD) δ 1.79-1.76 (m, 2H), 2.08-2.02 (m, 2H), 2.44 (s, 3H), 2.68-2.62 (m, 2H), 3.01-2.99 (m, 2H), 3.29-3.27 (m, 4H), 3.61 (s, 2H), 3.87-3.85 (m, 4H), 5.05 (s, 2H), 7.05 (d, *J* = 8.8 Hz, 2H), 7.29-7.19 (m, 4H), 7.89 (d, *J* = 8.8 Hz, 2H); LCMS (ESI) m/z 446 [M + H]⁺. | 0.5 |
| 133 | 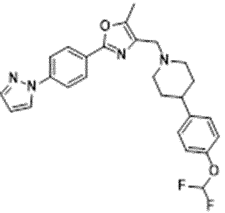 | 2-(4-(1H-pyrazol-1-yl)phenyl)-4-((4-(4-(difluoromethoxy)phenyl)p iperidin-1-yl)methyl)-5-methyloxazole | ¹H NMR (400 MHz, DMSO-*d₆*) δ 1.67-1.62 (m, 2H), 1.76-1.73 (m, 2H), 2.15-2.10 (m, 2H), 2.42 (s, 3H), 2.47 (m, 1H), 3.01-2.98 (m, 2H), 3.44 (s, 2H), 6.60 (s, 1H), 7.09 (d, *J* = 8.4 Hz, 2H), 7.16 (t, *J* = 74.4 Hz, 1H), 7.30 (d, *J* = 8.8 Hz, 2H), 7.81 (s, 1H), 8.00 (d, *J* = 9.2 Hz, 2H), 8.04 (d, *J* = 8.8 Hz, 2H), 8.60 (s, 1H); LCMS (ESI) m/z 465 [M + H]⁺. | 8 |
| 134 | 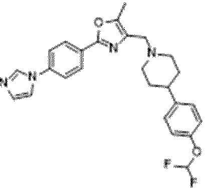 I | 2-(4-(1H-imidazol-1-yl)phenyl)-4-((4-(4-(difluoromethoxy)phenyl)p iperidin-1-yl)methyl)-5-methyloxazole | ¹H NMR (400 MHz, CD3OD) δ 1.88-1.79 (m, 4H), 2.34-2.29 (m, 2H), 2.48 (s, 3H), 2.62-2.56 (m, 1H), 3.17-3.15 (m, 2H), 3.57 (s, 2H), 6.75 (t, *J* = 74.4 Hz, 1H), 7.07 (d, *J* = 8.4 Hz, 2H), 7.20 (s, 1H), 7.28 (d, *J* = 8.4 Hz, 2H), 7.69 (s, 1H), 7.75 (d, *J* = 8.4 Hz, 2H), 8.16 (d, *J* = 8.4 Hz, 2H), 8.27 (s, 1H); LCMS (ESI) m/z 465 [M + H]⁺. | 4 |

### [Experimental Example 4] Evaluation of anti-tuberculosis activity of the new compound of chemical formula 1 according to the present invention against drug susceptibility and drug-resistant strains

The activity of various compounds against drug-susceptible and resistant strains was evaluated in a manner similar to Experiment 1 above. The results are summarized and shown in Table 4.

**[Table 4]**

| | | MIC100 (µg/ml) | | | | MIC100 (µM) | | |
|---|---|---|---|---|---|---|---|---|
| No. | Sample type | RIF | Del | LZD | BDQ | 54 | 94 | 95 |
| 1 | H37Rv | 0.008 | 0.25 | 1.2 | 0.5 | - | 1.6 | 0.8 |
| 2 | DS-1 | 0.008 | 0.063 | 0.6 | 0.25 | 12.8 | 12.8 | 0.8 |
| 3 | DS-2 | >4 | 0.063 | 1.2 | 0.125 | >12.8 | 12.8 | 1.6 |
| 4 | DS-3 | 0.008 | 0.125 | 0.6 | 0.5 | 6.4 | 1.6 | 0.8 |
| 5 | DS-4 | 0.008 | 0.063 | 0.6 | 0.25 | 6.4 | 1.6 | 1.6 |
| 6 | DS-5 | 0.008 | 0.063 | 0.6 | 0.25 | 12.8 | 3.2 | 0.8 |
| 7 | DS-6 | 0.008 | 0.063 | 0.6 | 0.25 | 1.6 | 0.8 | 0.8 |
| 8 | DS-7 | 0.008 | 0.031 | 0.8 | 1.6 | 6.4 | 0.8 | 1.6 |
| 9 | DS-8 | 0.008 | 0.031 | 0.6 | 0.5 | 3.2 | 0.4 | 0.8 |
| 10 | DS-9 | 0.016 | 0.031 | 0.6 | 0.25 | 6.4 | 0.8 | 0.8 |
| 11 | DS-10 | 0.016 | 0.125 | 1.2 | 0.25 | >12.8 | 12.8 | 1.6 |
| 12 | MDR-1 | >4 | 0.031 | 0.6 | 0.25 | 6.4 | 1.6 | 0.4 |
| 13 | MDR-2 | 4 | 0.063 | 0.6 | 0.25 | 12.8 | 6.4 | 0.8 |
| 14 | MDR-3 | >4 | 0.063 | 0.3 | 0.25 | 1.6 | 12.8 | 1.6 |
| 15 | MDR-4 | >4 | 0.063 | 0.6 | 0.25 | - | 1.6 | 0.8 |
| 16 | MDR-5 | >4 | 0.031 | 0.6 | 0.25 | 12.8 | 12.8 | 1.6 |
| 17 | MDR-6 | >4 | 0.063 | 0.6 | 0.25 | 6.4 | 0.8 | 0.8 |
| 18 | MDR-7 | >4 | 0.125 | 0.6 | 0.5 | 12.8 | 12.8 | 1.6 |
| 19 | MDR-8 | 4 | 0.031 | 0.6 | 0.125 | 6.4 | 0.8 | 0.8 |
| 20 | MDR-9 | >4 | 0.063 | 0.3 | 0.25 | - | 0.8 | 1.6 |
| 21 | MDR-10 | >4 | 0.125 | 0.6 | 0.125 | 6.4 | 12.8 | 0.8 |
| 22 | XDR-1 | >4 | 0.063 | 0.6 | 0.5 | 3.2 | 6.4 | 0.8 |
| 23 | XDR-2 | >4 | 0.125 | 0.6 | 0.5 | - | 6.4 | 0.8 |
| 24 | XDR-3 | >4 | 0.016 | 0.3 | 0.5 | >12.8 | 3.2 | 0.8 |
| 25 | XDR-4 | >4 | 0.031 | 1.2 | 0.125 | 6.4 | 3.2 | 0.4 |
| 26 | XDR-5 | >4 | 0.063 | 0.6 | 0.25 | - | 6.4 | 0.8 |
| 27 | XDR-6 | >4 | 0.125 | 0.6 | 0.25 | - | 6.4 | 0.8 |
| 28 | XDR-7 | >4 | 0.016 | 0.3 | 0.125 | >12.8 | 1.6 | 0.4 |
| 29 | XDR-8 | 2 | 0.016 | 0.6 | 0.125 | >12.8 | 6.4 | 3.2 |
| 30 | XDR-9 | 1 | 0.063 | 0.6 | 0.125 | >12.8 | 12.8 | 0.8 |
| 31 | XDR-10 | >4 | 0.016 | 0.6 | 0.125 | 6.4 | 0.8 | 0.4 |

| | | | | | | | | |
|---|---|---|---|---|---|---|---|---|
| DS : Drug susceptible, MDR : Multidrug resistant, XDR : Pre-extensively drug resistant | | | | | | | | |

## Claims

1. A compound of Chemical Formula 1: 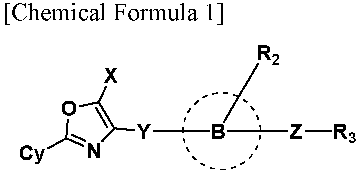
or a pharmaceutically acceptable salt thereof,
in the Chemical Formula 1,
Cy is phenyl, benzo[d][1,3]dioxole, 2,3-dihydrobenzofuran, 2,3-dihydrobenzo[b][1,4]dioxine, imidazo[1,2-a]pyridine, naphthalene, or quinoline, which are unsubstituted or substituted with one or more R₁,
R₁ is each independently halogen; C₁₋₄alkyl; C₁₋₄alkoxy; C₃₋₆cycloalkyl; C₁₋₄haloalkoxy;
C₁₋₄haloalkyl; -OH; phenoxy; phenyl; -CN; -NH₂; -N(CH₃)₂; -NO₂; -NHCH₃; -NHAc; -CO₂H; - CO₂CH₃; -C(O)NHCH₃; -C(O)N(CH₃)₂; -NHC(O)-C₁₋₄alkyl; -NHC(O)phenyl; phenyl substituted with one or more selected from the group consisting of halogen, C₁₋₄alkyl, C₃₋₆cycloalkyl, C₁₋₄haloalkoxy, C₁₋₄haloalkyl, -OH, phenoxy, phenyl, C₁₋₄alkoxy, -CN, -NH₂, and -N(CH₃)₂; pyridine unsubstituted or substituted with halogen; naphthalene unsubstituted or substituted with C₁₋₄alkoxy; benzotriazole; quinoline or isoquinoline unsubstituted or substituted with one or more selected from the group consisting of C₁₋₄alkyl, C₁₋₄alkoxy, and halogen; indazole unsubstituted or substituted with C₁₋₄alkyl; C₃₋₆cycloalkyl; benzothiophene; benzofuran; indole unsubstituted or substituted with C₁₋₄alkyl; pyrazole unsubstituted or substituted with C₁₋₄alkyl; imidazole unsubstituted or substituted with C₁₋₄alkyl; triazole; pyrrolidine; piperidine unsubstituted or substituted with C₁₋₄alkyl; piperazine unsubstituted or substituted with C₁₋₄alkyl; pyrrole; furan; morpholine; or thiophene,
X is hydrogen, halogen, C₁₋₄alkyl, C₁₋₄haloalkyl, C₃₋₆cycloalkyl, C₁₋₄alkoxy, C₁₋₄haloalkoxy, -OH, phenoxy, phenyl, -CN, -NH₂, or -N(CH₃)₂,
Y is -NH- or -CH₂-,
B is phenyl; piperidine; piperazine; or 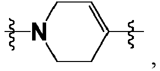
which are unsubstituted or substituted with one or more R₂; or B is 3 or 4-spiropiperidine which is a spiro compound linked by sharing carbon with chemical formula 2a, 2b or 2c, 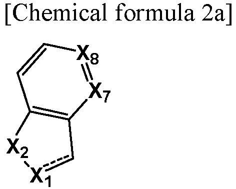 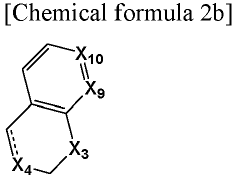 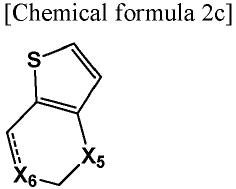
in the chemical formula 2a to 2c,
X₁ to X₆ are each independently CH, CH₂, O, NH, or N,
X₇ to X₁₀ are each independently CH or N,
-̅-̅-̅-̅-̅-̅ is a single bond or double bond,
R₂ is -OH, phenyl, halogen, C₁₋₄alkyl, C₃₋₆cycloalkyl, C₁₋₄haloalkoxy, C₁₋₄haloalkyl, phenoxy, C₁₋₄alkoxy, -CN, -NH₃, -N(CH₃)₂, or =O,
Z is absent, direct linking, -NH-, -N(CH₃)-, -O-, or -O(CH₂)-,
R₃ is hydrogen; halogen; C₁₋₄alkyl; C₃₋₆cycloalkyl; C₁₋₄haloalkoxy; C₁₋₄haloalkyl; -OH; phenoxy; phenyl; C₁₋₄alkoxy; -CN; -NH₂; -N(CH₃)₂; phenyl substituted with one or more selected from the group consisting of halogen, C₁₋₄alkyl, C₃₋₆cycloalkyl, C₁₋₄haloalkoxy, C₁₋₄haloalkyl, - OH, phenoxy, phenyl, C₁₋₄alkoxy, -CN, -NO₂, -NH₂, and -N(CH₃)₂; pyridine unsubstituted or substituted with halogen; naphthalene unsubstituted or substituted with C₁₋₄alkoxy; benzotriazole; quinoline or isoquinoline substituted with one or more selected from the group consisting of C₁₋₄alkyl, C₁₋₄alkoxy, and halogen; indazole unsubstituted or substituted with C₁₋₄alkyl; C₃₋₆ cycloalkyl; benzothiophene; benzofuran; indole unsubstituted or substituted with C₁₋₄alkyl; pyrazole; imidazole; pyrrolidine; piperidine unsubstituted or substituted with C₁₋₄alkyl; piperazine unsubstituted or substituted with C₁₋₄alkyl; pyrrole; furan; thiophene; morpholine or thiophene.

2. The compound of Claim 1 or a pharmaceutically acceptable salt thereof, in the Chemical Formula 1,
Cy is phenyl, benzo[d][1,3]dioxole, 2,3-dihydrobenzofuran, 2,3-dihydrobenzo[b][1,4]dioxine, imidazo[1,2-a]pyridine, naphthalene or quinoline, which are unsubstituted or substituted with one or more R₁,
R₁ is each independently halogen; C₁₋₄alkyl; C₁₋₄alkoxy; C₃₋₆cycloalkyl; C₁₋₄haloalkoxy; C₁₋₄haloalkyl; -OH; phenoxy; phenyl; -CN; -NH₂; -N(CH₃)₂; -NO₂; -NHCH₃; -NHAc; -CO₂H; - CO₂CH₃; -C(O)NHCH₃; -C(O)N(CH₃)₂; -NHC(O)-C₁₋₄alkyl; -NHC(O)phenyl; phenyl substituted with one or more selected from the group consisting of halogen, C₁₋₄alkyl, C₃₋₆cycloalkyl, C₁₋₄haloalkoxy, C₁₋₄haloalkyl, -OH, phenoxy, phenyl, C₁₋₄alkoxy, -CN, -NH₂, and -N(CH₃)₂; pyridine unsubstituted or substituted with halogen; naphthalene unsubstituted or substituted with C₁₋₄alkoxy; benzotriazole; quinoline or isoquinoline unsubstituted or substituted with one or more selected from the group consisting of C₁₋₄alkyl, C₁₋₄alkoxy, and halogen; indazole unsubstituted or substituted with C₁₋₄alkyl; C₃₋₆cycloalkyl; benzothiophene; benzofuran; indole unsubstituted or substituted with C₁₋₄alkyl; pyrazole unsubstituted or substituted with C₁₋₄alkyl; imidazole unsubstituted or substituted with C₁₋₄alkyl; triazole; pyrrolidine; piperidine unsubstituted or substituted with C₁₋₄alkyl; piperazine unsubstituted or substituted with C₁₋₄alkyl; pyrrole; furan; morpholine; or thiophene,
X is hydrogen, halogen, C₁₋₄alkyl, C₁₋₄haloalkyl, C₃₋₆cycloalkyl, C₁₋₄alkoxy, C₁₋₄haloalkoxy, -OH, phenoxy, phenyl, -CN, -NH₂, or -N(CH₃)₂,
Y is -CH₂-,
B is phenyl; piperidine; or piperazine, which are unsubstituted or substituted with one or more R₂; or B is 3 or 4-spiropiperidine which is a spiro compound linked by sharing carbon with chemical formula 2a, 2b or 2c,
R₂ is -OH, phenyl, halogen, C₁₋₄alkyl, C₃₋₆cycloalkyl, C₁₋₄haloalkoxy, C₁₋₄haloalkyl, phenoxy, C₁₋₄alkoxy, -CN, -NH₃, or -N(CH₃)₂,
Z is absent, direct linking, -O-, or -O(CH₂)-,
R₃ is hydrogen; halogen; C₁₋₄alkyl; C₃₋₆cycloalkyl; C₁₋₄haloalkoxy; C₁₋₄haloalkyl; -OH; phenoxy; phenyl; C₁₋₄alkoxy; -CN; -NH₂; -N(CH₃)₂; phenyl substituted with one or more selected from the group consisting of halogen, C₁₋₄alkyl, C₃₋₆cycloalkyl, C₁₋₄haloalkoxy, C₁₋₄haloalkyl, - OH, phenoxy, phenyl, C₁₋₄alkoxy, -CN, -NO₂, -NH₂, and -N(CH₃)₂; pyridine unsubstituted or substituted with halogen; naphthalene unsubstituted or substituted with C₁₋₄alkoxy; benzotriazole; quinoline or isoquinoline substituted with one or more selected from the group consisting of C₁-₄alkyl, C₁₋₄alkoxy, and halogen; indazole unsubstituted or substituted with C₁₋₄alkyl; C₃₋₆ cycloalkyl; benzothiophene; benzofuran; indole unsubstituted or substituted with C₁₋₄alkyl; pyrazole; imidazole; pyrrolidine; piperidine unsubstituted or substituted with C₁₋₄alkyl; piperazine unsubstituted or substituted with C₁₋₄alkyl; pyrrole; furan; thiophene; morpholine or thiophene.

3. The compound of Claim 2 or a pharmaceutically acceptable salt thereof, in the Chemical Formula 1,
Cy is phenyl unsubstituted or substituted with one or more R₁,
R₁ is each independently halogen; C₁₋₄alkyl; C₁₋₄alkoxy; C₃₋₆cycloalkyl; C₁₋₄haloalkoxy; C₁₋₄haloalkyl; -OH; phenoxy; phenyl; -CN; -NH₂; -N(CH₃)₂; -NO₂; -NHCH₃; -NHAc; -CO₂H; - CO₂CH₃; -C(O)NHCH₃; -C(O)N(CH₃)₂; -NHC(O)-C₁₋₄alkyl; -NHC(O)phenyl; phenyl substituted with one or more selected from the group consisting of halogen, C₁₋₄alkyl, C₃₋₆cycloalkyl, C₁₋₄haloalkoxy, C₁₋₄haloalkyl, -OH, phenoxy, phenyl, C₁₋₄alkoxy, -CN, -NH₂, and -N(CH₃)₂; pyridine unsubstituted or substituted with halogen; naphthalene unsubstituted or substituted with C₁₋₄alkoxy; benzotriazole; quinoline or isoquinoline unsubstituted or substituted with one or more selected from the group consisting of C₁₋₄alkyl, C₁₋₄alkoxy, and halogen; indazole unsubstituted or substituted with C₁₋₄alkyl; C₃₋₆cycloalkyl; benzothiophene; benzofuran; indole unsubstituted or substituted with C₁₋₄alkyl; pyrazole unsubstituted or substituted with C₁₋₄alkyl; imidazole unsubstituted or substituted with C₁₋₄alkyl; triazole; pyrrolidine; piperidine unsubstituted or substituted with C₁₋₄alkyl; piperazine unsubstituted or substituted with C₁₋₄alkyl; pyrrole; furan; morpholine; or thiophene,
X is hydrogen, halogen, C₁₋₄alkyl, C₁₋₄haloalkyl, C₃₋₆cycloalkyl, C₁₋₄alkoxy, C₁₋₄haloalkoxy, -OH, -CN, -NH₂, or -N(CH₃)₂,
Y is -CH₂-,
B is unsubstituted piperidine, 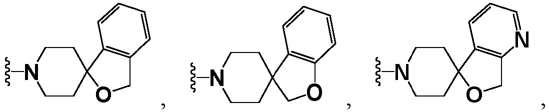 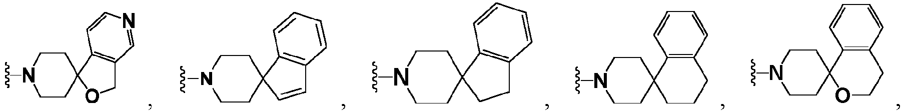
or 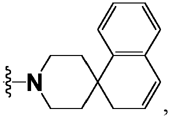
Z is absent or direct linking,
R₃ is hydrogen; halogen; C₁₋₄alkyl; C₃₋₆cycloalkyl; C₁₋₄haloalkoxy; C₁₋₄haloalkyl; -OH; phenoxy; phenyl; C₁₋₄alkoxy; -CN; -NH₂; -N(CH₃)₂; phenyl substituted with one or more selected from the group consisting of halogen, C₁₋₄alkyl, C₃₋₆cycloalkyl, C₁₋₄haloalkoxy, C₁₋₄haloalkyl, - OH, phenoxy, phenyl, C₁₋₄alkoxy, -CN, -NO₂, -NH₂, and -N(CH₃)₂; pyridine unsubstituted or substituted with halogen; naphthalene unsubstituted or substituted with C₁₋₄alkoxy; benzotriazole; quinoline or isoquinoline substituted with one or more selected from the group consisting of C₁₋₄alkyl, C₁₋₄alkoxy, and halogen; indazole unsubstituted or substituted with C₁₋₄alkyl; C₃₋₆ cycloalkyl; benzothiophene; benzofuran; indole unsubstituted or substituted with C₁₋₄alkyl; pyrazole; imidazole; pyrrolidine; piperidine unsubstituted or substituted with C₁₋₄alkyl; piperazine unsubstituted or substituted with C₁₋₄alkyl; pyrrole; furan; thiophene; morpholine or thiophene.

4. The compound of Claim 1 or a pharmaceutically acceptable salt thereof, wherein the compound is any one of the following compounds:
| cpd# | IUPAC NAME |
|---|---|
| 1 | 2-(6-chlorobenzo[d][1,3]dioxol-5-yl)-5-methyl-4-(4-phenoxybenzyl)oxazole |
| 2 | 4-(4-(4-fluorophenoxy)benzyl)-2-(4-methoxyphenyl)-5-methyloxazole |
| 3 | 4-([1,1'-biphenyl]-3-ylmethyl)-2-(4-methoxyphenyl)-5-methyloxazole |
| 4 | 2-(4-chlorophenyl)-5-methyl-4-(4-phenoxybenzyl)oxazole |
| 5 | 5-methyl-4-(4-phenoxybenzyl)-2-(4-(trifluoromethyl)phenyl)oxazole |
| 6 | 2-(4-methoxyphenyl)-5-methyl-4-(4-phenoxybenzyl)oxazole |
| 7 | 5-methyl-4-(4-phenoxybenzyl)-2-(4-(trifluoromethoxy)phenyl)oxazole |
| 8 | 4-(5-methyl-4-(4-phenoxybenzyl)oxazol-2-yl)benzonitrile |
| 9 | 2-(4-methoxyphenyl)-4-(4-phenoxybenzyl)-5-phenyloxazole |
| 10 | N-(4-(5-methyl-4-(4-phenoxybenzyl)oxazol-2-yl)phenyl)acetamide |
| 11 | methyl 4-(5-methyl-4-(4-phenoxybenzyl)oxazol-2-yl)benzoate |
| 12 | N-methyl-4-(5-methyl-4-(4-phenoxybenzyl)oxazol-2-yl)aniline |
| 13 | N,N-dimethyl-4-(5-methyl-4-(4-phenoxybenzyl)oxazol-2-yl)aniline |
| 14 | 5-methyl-4-(4-phenoxybenzyl)-2-(p-tolyl)oxazole |
| 15 | 5-methyl-2-(naphthalen-2-yl)-4-(4-phenoxybenzyl)oxazole |
| 16 | 5-methyl-4-(4-phenoxybenzyl)-2-(quinolin-6-yl)oxazole |
| 17 | 4-(4-(4-(4-fluorophenoxy)benzyl)-5-methyloxazol-2-yl)benzonitrile |
| 18 | 5-methyl-4-(4-phenoxybenzyl)-2-(quinolin-7-yl)oxazole |
| 19 | 2-(4-chlorophenyl)-4-(4-(4-fluorophenoxy)benzyl)-5-methyloxazole |
| 20 | 4-(4-(4-fluorophenoxy)benzyl)-5-methyl-2-(4-(trifluoromethyl)phenyl)oxazole |
| 21 | 4-(4-(4-fluorophenoxy)benzyl)-2-(4-fluorophenyl)-5-methyloxazole |
| 22 | 4-(4-(4-(4-fluorophenoxy)benzyl)-5-methyloxazol-2-yl)phenol |
| 23 | N-(4-(5-methyl-4-(4-phenoxybenzyl)oxazol-2-yl)phenyl)isobutyramide |
| 24 | N-(4-(5-methyl-4-(4-phenoxybenzyl)oxazol-2-yl)phenyl)benzamide |
| 25 | 4-(4-(4-fluorophenoxy)benzyl)-5-methyl-2-(4-nitrophenyl)oxazole |
| 26 | 2-(4-chlorophenyl)-4-(4-phenoxybenzyl)-5-phenyloxazole |
| 27 | 4-(4-phenoxybenzyl)-5-phenyl-2-(4-(trifluoromethyl)phenyl)oxazole |
| 28 | 4-(5-methyl-4-(4-phenoxybenzyl)oxazol-2-yl)benzoic acid |
| 29 | 2-(4-fluorophenyl)-5-methyl-4-(4-phenoxybenzyl)oxazole |
| 30 | 2-(4-(1H-pyrazol-1-yl)phenyl)-5-methyl-4-(4-phenoxybenzyl)oxazole |
| 31 | 2-(2-chloro-4-fluorophenyl)-5-methyl-4-(4-phenoxybenzyl)oxazole |
| 32 | 4-((5-methyl-2-(4-(trifluoromethyl)phenyl)oxazol-4-yl)methyl)-N-phenylaniline |
| 33 | 4-((5-methyl-2-(4-(trifluoromethyl)phenyl)oxazol-4-yl)methyl)-N-(4-(trifluoromethoxy)phenyl)aniline |
| 34 | N-(4-(5-methyl-4-(4-phenoxybenzyl)oxazol-2-yl)phenyl)propionamide |
| 35 | 2-(2,4-dichlorophenyl)-5-methyl-4-(4-phenoxybenzyl)oxazole |
| 36 | 2-(4-(difluoromethoxy)phenyl)-5-methyl-4-(4-phenoxybenzyl)oxazole |
| 37 | N-methyl-4-((5-methyl-2-(4-(trifluoromethyl)phenyl)oxazol-4-yl)methyl)-N-(4-(trifluoromethoxy)phenyl)aniline |
| 38 | 5-methyl-4-(4-(p-tolyloxy)benzyl)-2-(4-(trifluoromethyl)phenyl)oxazole |
| 39 | 4-([1,1'-biphenyl]-3-ylmethyl)-5-isopropyl-2-(4-(trifluoromethyl)phenyl)oxazole |
| 40 | N-methyl-4-(5-methyl-4-(4-phenoxybenzyl)oxazol-2-yl)benzamide |
| 41 | N,N-dimethyl-4-(5-methyl-4-(4-phenoxybenzyl)oxazol-2-yl)benzamide |
| 42 | 2-(4-chlorophenyl)-4-(4-phenoxybenzyl)oxazole |
| 43 | 2-(4-(1H-pyrazol-1-yl)phenyl)-4-(4-(4-fluorophenoxy)benzyl)-5-methyloxazole |
| 44 | 4-(4-(4-(4-fluorophenoxy)benzyl)-5-methyloxazol-2-yl)-N,N-dimethylaniline |
| 45 | 4-(4-(4-fluorophenoxy)benzyl)-5-methyl-2-(quinolin-6-yl)oxazole |
| 46 | 4-(4-(4-(4-(4-fluorophenoxy)benzyl)-5-methyloxazol-2-yl)phenyl)morpholine |
| 47 | 4-(4-(4-fluorophenoxy)benzyl)-5-methyl-2-(4-(4-methylpiperidin-1-yl)phenyl)oxazole |
| 49 | 2-(4-(1H-pyrazol-1-yl)phenyl)-5-methyl-4-(4-(4-(trifluoromethoxy)phenoxy)benzyl)oxazole |
| 51 | 4-((2-(4-(1H-pyrazol-1-yl)phenyl)-5-methyloxazol-4-yl)methyl)-N-(4-fluorophenyl)aniline |
| 52 | 4-((2-(4-(1H-pyrazol-1-yl)phenyl)-5-methyloxazol-4-yl)methyl)-N-(4-(trifluoromethoxy)phenyl)aniline |
| 53 | 4-(4-(4-fluorophenoxy)benzyl)-5-methyl-2-(4-(pyridin-3-yl)phenyl)oxazole |
| 54 | 4-(4-(4-fluorophenoxy)benzyl)-5-methyl-2-(4-(pyridin-4-yl)phenyl)oxazole |
| 55 | 5-methyl-2-(4-(pyridin-3-yl)phenyl)-4-(4-(4-(trifluoromethoxy)phenoxy)benzyl)oxazole |
| 56 | 5-methyl-2-(4-(pyridin-4-yl)phenyl)-4-(4-(4-(trifluoromethoxy)phenoxy)benzyl)oxazole |
| 57 | 4-(4-(2-fluorophenoxy)benzyl)-5-methyl-2-(4-(pyridin-3-yl)phenyl)oxazole |
| 58 | 4-(4-(3-fluorophenoxy)benzyl)-5-methyl-2-(4-(pyridin-3-yl)phenyl)oxazole |
| 59 | 4-(3-(4-fluorophenoxy)benzyl)-5-methyl-2-(4-(pyridin-3-yl)phenyl)oxazole |
| 60 | 2-(4-(4-ethylpiperazin-1-yl)phenyl)-4-(4-(4-fluorophenoxy)benzyl)-5-methyloxazole |
| 61 | 4-(4-(3-fluorophenoxy)benzyl)-5-methyl-2-(4-(pyridin-4-yl)phenyl)oxazole |
| 62 | 2-(4-(4-ethylpiperazin-1-yl)phenyl)-4-(4-(2-fluorophenoxy)benzyl)-5-methyloxazole |
| 63 | 2-(4-(4-ethylpiperazin-1-yl)phenyl)-4-(4-(3-fluorophenoxy)benzyl)-5-methyloxazole |
| 64 | 2-(4-(4-ethylpiperazin-1-yl)phenyl)-4-(3-(4-fluorophenoxy)benzyl)-5-methyloxazole |
| 65 | 4-(4-(4-fluorophenoxy)benzyl)-5-methyl-2-(2-methyl-4-(pyridin-4-yl)phenyl)oxazole |
| 66 | 2-(3-fluoro-4-(pyridin-4-yl)phenyl)-4-(4-(4-fluorophenoxy)benzyl)-5-methyloxazole |
| 67 | 4-((4-(4-fluorophenyl)piperidin-1-yl)methyl)-5-methyl-2-(4-(pyridin-4-yl)phenyl)oxazole |
| 68 | 4-(4-fluorophenyl)-1-((5-methyl-2-(4-(pyridin-4-yl)phenyl)oxazol-4-yl)methyl)piperidin-4-ol |
| 69 | 5-methyl-2-(4-(pyridin-4-yl)phenyl)-4-((4-(4-(trifluoromethoxy)phenoxy)piperidin-1-yl)methyl)oxazole |
| 70 | 5-methyl-2-(4-(pyridin-4-yl)phenyl)-4-((4-(4-(trifluoromethoxy)phenyl)piperidin-1-yl)methyl)oxazole |
| 71 | 2-(4-(1H-imidazol-1-yl)phenyl)-4-(4-(4-fluorophenoxy)benzyl)-5-methyloxazole |
| 72 | 2-(4-(2H-1,2,3-triazol-2-yl)phenyl)-4-((4-(4-fluorophenyl)piperidin-1-yl)methyl)-5-methyloxazole |
| 73 | 2-(4-(2H-1,2,3-triazol-2-yl)phenyl)-5-methyl-4-((4-(4-(trifluoromethoxy)phenoxy)piperidin-1-yl)methyl)oxazole |
| 74 | 2-(4-(2H-1,2,3-triazol-2-yl)phenyl)-5-methyl-4-((4-(4-(trifluoromethoxy)phenoxy)piperidin-1-yl)methyl)oxazole |
| 75 | 2-(4-(1H-imidazol-1-yl)phenyl)-5-methyl-4-((4-(4-(trifluoromethoxy)phenoxy)piperidin-1-yl)methyl)oxazole |
| 76 | 4-(4-((4-fluorobenzyl)oxy)benzyl)-5-methyl-2-(4-(pyridin-4-yl)phenyl)oxazole |
| 77 | 5-methyl-2-(4-(pyridin-4-yl)phenyl)-4-(4-((4-(trifluoromethyl)benzyl)oxy)benzyl)oxazole |
| 78 | 5-methyl-2-(4-(pyridin-4-yl)phenyl)-4-(4-((4-(trifluoromethoxy)benzyl)oxy)benzyl)oxazole |
| 79 | 2-(4-(1H-1,2,3-triazol-1-yl)phenyl)-4-(4-(4-fluorophenoxy)benzyl)-5-methyloxazole |
| 80 | 2-(4-(2H-1,2,3-triazol-2-yl)phenyl)-5-methyl-4-((4-(4-(trifluoromethoxy)phenyl)piperidin-1-yl)methyl)oxazole |
| 81 | 2-(benzo[d][1,3]dioxol-5-yl)-5-methyl-4-((4-(4-(trifluoromethoxy)phenyl)piperidin-1-yl)methyl)oxazole |
| 82 | 2-(benzo[d][1,3]dioxol-5-yl)-4-(4-(4-fluorophenoxy)benzyl)-5-methyloxazole |
| 83 | 2-(4-(1H-pyrazol-1-yl)phenyl)-5-methyl-4-((4-(4-(trifluoromethoxy)phenyl)piperidin-1-yl)methyl)oxazole |
| 84 | 5-methyl-2-(4-(pyridin-4-yl)phenyl)-4-((4-(4-(trifluoromethoxy)phenyl)piperazin-1-yl)methyl)oxazole |
| 85 | 2-(4-(1H-pyrazol-1-yl)phenyl)-5-methyl-4-((4-(4-(trifluoromethoxy)phenyl)piperazin-1-yl)methyl)oxazole |
| 86 | 2-(benzo[d][1,3]dioxol-5-yl)-5-methyl-4-((4-(4-(trifluoromethoxy)phenyl)piperazin-1-yl)methyl)oxazole |
| 87 | 1'-((2-(4-(1H-pyrazol-1-yl)phenyl)-5-methyloxazol-4-yl)methyl)-3H-spiro[isobenzofuran-1,4'-piperidine] |
| 88 | 2-(4-(1H-imidazol-1-yl)phenyl)-5-methyl-4-((4-(4-(trifluoromethoxy)phenyl)piperazin-1-yl)methyl)oxazole |
| 89 | 1'-((2-(4-(1H-imidazol-1-yl)phenyl)-5-methyloxazol-4-yl)methyl)-3H-spiro[isobenzofuran-1,4'-piperidine] |
| 90 | 2-(4-(1H-imidazol-1-yl)phenyl)-5-methyl-4-(spirofindene-1,4'-piperidin]-1'-ylmethyl)oxazole |
| 91 | 2-(4-(1H-pyrazol-1-yl)phenyl)-5-methyl-4-(spirofindene-1,4'-piperidin]-1'-ylmethyl)oxazole |
| 92 | 2-(benzo[d][1,3]dioxol-5-yl)4-((2,3-dihydrospiro[indene-1,4'-piperidin]-1'-yl)methyl)-5-methyloxazole |
| 93 | 1'-((2-(benzo[d][1,3]dioxol-5-yl)-5-methyloxazol-4-yl)methyl)-3H-spiro[isobenzofuran-1,4'-piperidine] |
| 94 | 2-(4-(1H-imidazol-1-yl)phenyl)-5-methyl-4-((4-(4-(trifluoromethoxy)phenyl)piperidin-1-yl)methyl)oxazole |
| 95 | 2-(4-(1H-pyrazol-1-yl)phenyl)-4-((2,3-dihydrospirofindene-1,4'-piperidin]-1'-yl)methyl)-5-methyloxazole |
| 96 | 2-(4-(1H-imidazol-1-yl)phenyl)-4-((2,3-dihydrospiro[indene-1,4'-piperidin]-1'-yl)methyl)-5-methyloxazole |
| 97 | 2-(4-(1H-pyrazol-1-yl)phenyl)-5-methyl-4-((4-(3-(trifluoromethoxy)phenyl)piperidin-1-yl)methyl)oxazole |
| 98 | 2-(4-(1H-imidazol-1-yl)phenyl)-5-methyl-4-((4-(3-(trifluoromethoxy)phenyl)piperidin-1-yl)methyl)oxazole |
| 99 | 2-(4-(1H-pyrazol-1-yl)phenyl)-5-methyl-4-((4-(2-(trifluoromethoxy)phenyl)piperidin-1-yl)methyl)oxazole |
| 100 | 2-(4-(1H-imidazol-1-yl)phenyl)-5-methyl-4-((4-(2-(trifluoromethoxy)phenyl)piperidin-1-yl)methyl)oxazole |
| 101 | 2-(4-(1H-pyrazol-1-yl)phenyl)-5-methyl-4-((4-(2-(trifluoromethoxy)phenyl)-3,6-dihydropyridin-1(2H)-yl)methyl)oxazole |
| 102 | 2-(4-(1H-pyrazol-1-yl)phenyl)-5-methyl-4-((4-(3-(trifluoromethoxy)phenyl)-3,6-dihydropyridin-1(2H)-yl)methyl)oxazole |
| 103 | 2-(4-(1H-pyrazol-1-yl)phenyl)-5-methyl-4-((4-(4-(trifluoromethoxy)phenyl)-3,6-dihydropyridin-1(2H)-yl)methyl)oxazole |
| 104 | 2-(4-(1H-imidazol-1-yl)phenyl)-5-methyl-4-((4-(2-(trifluoromethoxy)phenyl)-3,6-dihydropyridin-1(2H)-yl)methyl)oxazole |
| 105 | 2-(4-(1H-imidazol-1-yl)phenyl)-5-methyl-4-((4-(3-(trifluoromethoxy)phenyl)-3,6-dihydropyridin-1(2H)-yl)methyl)oxazole |
| 106 | 2-(4-(1H-imidazol-1-yl)phenyl)-5-methyl-4-((4-(4-(trifluoromethoxy)phenyl)-3,6-dihydropyridin-1(2H)-yl)methyl)oxazole |
| 107 | 5-methyl-2-(4-(pyridin-4-yl)phenyl)-4-(spiro[indene-1,4'-piperidin]-1'-ylmethyl)oxazole |
| 108 | 1'-((5-methyl-2-(4-(pyridin-4-yl)phenyl)oxazol-4-yl)methyl)-3H-spiro[isobenzofuran-1,4'-piperidine] |
| 109 | 2-(4-(1H-pyrazol-1-yl)phenyl)-5-methyl-4-((4-phenylpiperidin-1-yl)methyl)oxazole |
| 110 | 2-(4-(1H-pyrazol-1-yl)phenyl)-5-methyl-4-((4-(4-nitrophenyl)piperidin-1-yl)methyl)oxazole |
| 111 | 2-(4-(1H-imidazol-1-yl)phenyl)-5-methyl-4-((4-phenylpiperidin-1-yl)methyl)oxazole |
| 112 | 2-(4-(1H-imidazol-1-yl)phenyl)-5-methyl-4-((4-(4-nitrophenyl)piperidin-1-yl)methyl)oxazole |
| 113 | 4-((2,3-dihydrospiro[indene-1,4'-piperidin]-1'-yl)methyl)-5-methyl-2-(4-(pyridin-4-yl)phenyl)oxazole |
| 114 | 4-(4-(4-fluorophenoxy)benzyl)-5-methyl-2-(4-(2-methyl-1H-imidazol-1-yl)phenyl)oxazole |
| 115 | 5-methyl-2-(4-(2-methyl-1H-imidazol-1-yl)phenyl)-4-((4-(4-(trifluoromethoxy)phenyl)piperidin-1-yl)methyl)oxazole |
| 116 | 5-methyl-2-(4-(2-methyl-1H-imidazol-1-ylphenyl-4-(spiro[indene-1,4'-piperidin]-1-ylmethyl)oxazole |
| 117 | 2-(4-(1H-pyrazol-1-yl)phenyl)-4-((4-(4-chlorophenyl)piperidin-1-yl)methyl)-5-methyloxazole |
| 118 | 2-(4-(1H-pyrazol-1-yl)phenyl)-4-((4,4-diphenylpiperidin-1-yl)methyl)-5-methyloxazole |
| 119 | 2-(4-(1H-pyrazol-1-yl)phenyl)-4-((4-(4-methoxy-2-methylphenyl)piperidin-1-yl)methyl)-5-methyloxazole |
| 120 | 2-(4-(1H-pyrazol-1-yl)phenyl)-4-((4-(2,4-dimethoxyphenyl)piperidin-1-yl)methyl)-5-methyloxazole |
| 121 | 2-(4-(1H-imidazol-1-yl)phenyl)-4-((4-(4-chlorophenyl)piperidin-1-yl)methyl)-5-methyloxazole |
| 122 | 5-methyl-2-(4-(4-methyl-1H-imidazol-1-yl)phenyl)-4-((4-(4-(trifluoromethoxy)phenyl)piperidin-1-yl)methyl)oxazole |
| 123 | 2-(2,3-dihydrobenzofuran-7-yl)-4-((2,3-dihydrospiro[indene-1,4'-piperidin]-1'-yl)methyl)-5-methyloxazole |
| 124 | 2-(2,3-dihydrobenzofuran-7-yl)-5-methyl-4-((4-(4-(trifluoromethoxy)phenyl)piperidin-1-yl)methyl)oxazole |
| 125 | 2-(2,3-dihydrobenzo[b][1,4]dioxin-5-yl)-5-methyl-4-(spiro[indene-1,4'-piperidin]-1'-ylmethyl)oxazole |
| 126 | 2-(2,3-dihydrobenzo[b][1,4]dioxin-5-yl)-5-methyl-4-((4-(4-(trifluoromethoxy)phenyl)piperidin-1-yl)methyl)oxazole |
| 127 | 4-(4-(5-methyl-4-(spiro[indene-1,4'-piperidin]-1'-ylmethyl)oxazol-2-yl)phenyl)morpholine |
| 128 | 4-(4-(5-methyl-4-((4-(4-(trifluoromethoxy)phenyl)piperidin-1-yl)methyl)oxazol-2-yl)phenyl)morpholine |
| 129 | 4-(4-(4-((2,3-dihydrospiro[indene-1,4'-piperidin]-1'-yl)methyl)-5-methyloxazol-2-yl)phenyl)morpholine |
| 130 | 2-(imidazo[1,2-a]pyridin-6-yl)-5-methyl-4-(spiro[indene-1,4'-piperidin]-1'-ylmethyl)oxazole |
| 131 | 2-(imidazo[1,2-a]pyridin-6-yl)-5-methyl-4-((4-(4-(trifluoromethoxy)phenyl)piperidin-1-yl)methyl)oxazole |
| 132 | 1'-((5-methyl-2-(4-morpholinophenyl)oxazol-4-yl)methyl)-3H-spiro[isobenzofuran-1,4'-piperidine] |
| 133 | 2-(4-(1H-pyrazol-1-yl)phenyl)-4-((4-(4-(difluoromethoxy)phenyl)piperidin-1-yl)methyl)-5-methyloxazole |
| 134 | 2-(4-(1H-imidazol-1-yl)phenyl)-4-((4-(4-(difluoromethoxy)phenyl)piperidin-1-yl)methyl)-5-methyloxazole |

5. The compound of Claim 4 or a pharmaceutically acceptable salt thereof, wherein the compound is any one of the following compounds:
| cpd# | IUPAC NAME |
|---|---|
| 2 | 4-(4-(4-fluorophenoxy)benzyl)-2-(4-methoxyphenyl)-5-methyloxazole |
| 6 | 2-(4-methoxyphenyl)-5-methyl-4-(4-phenoxybenzyl)oxazole |
| 13 | N,N-dimethyl-4-(5-methyl-4-(4-phenoxybenzyl)oxazol-2-yl)aniline |
| 16 | 5-methyl-4-(4-phenoxybenzyl)-2-(quinolin-6-yl)oxazole |
| 17 | 4-(4-(4-(4-fluorophenoxy)benzyl)-5-methyloxazol-2-yl)benzonitrile |
| 18 | 5-methyl-4-(4-phenoxybenzyl)-2-(quinolin-7-yl)oxazole |
| 20 | 4-(4-(4-fluorophenoxy)benzyl)-5-methyl-2-(4-(trifluoromethyl)phenyl)oxazole |
| 30 | 2-(4-(1H-pyrazol-1-yl)phenyl)-5-methyl-4-(4-phenoxybenzyl)oxazole |
| 33 | 4-((5-methyl-2-(4-(trifluoromethyl)phenyl)oxazol-4-yl)methyl)-N-(4-(trifluoromethoxy)phenyl)aniline |
| 43 | 2-(4-(1H-pyrazol-1-yl)phenyl)-4-(4-(4-fluorophenoxy)benzyl)-5-methyloxazole |
| 44 | 4-(4-(4-(4-fluorophenoxy)benzyl)-5-methyloxazol-2-yl)-N,N-dimethylaniline |
| 45 | 4-(4-(4-fluorophenoxy)benzyl)-5-methyl-2-(quinolin-6-yl)oxazole |
| 46 | 4-(4-(4-(4-(4-fluorophenoxy)benzyl)-5-methyloxazol-2-yl)phenyl)morpholine |
| 49 | 2-(4-(1H-pyrazol-1-yl)phenyl)-5-methyl-4-(4-(4-(trifluoromethoxy)phenoxy)benzyl)oxazole |
| 51 | 4-((2-(4-(1H-pyrazol-1-yl)phenyl)-5-methyloxazol-4-yl)methyl)-N-(4-fluorophenyl)aniline |
| 53 | 4-(4-(4-fluorophenoxy)benzyl)-5-methyl-2-(4-(pyridin-3-yl)phenyl)oxazole |
| 54 | 4-(4-(4-fluorophenoxy)benzyl)-5-methyl-2-(4-(pyridin-4-yl)phenyl)oxazole |
| 55 | 5-methyl-2-(4-(pyridin-3-yl)phenyl)-4-(4-(4-(trifluoromethoxy)phenoxy)benzyl)oxazole |
| 56 | 5-methyl-2-(4-(pyridin-4-yl)phenyl)-4-(4-(4-(trifluoromethoxy)phenoxy)benzyl)oxazole |
| 58 | 4-(4-(3-fluorophenoxy)benzyl)-5-methyl-2-(4-(pyridin-3-yl)phenyl)oxazole |
| 59 | 4-(3-(4-fluorophenoxy)benzyl)-5-methyl-2-(4-(pyridin-3-yl)phenyl)oxazole |
| 61 | 4-(4-(3-fluorophenoxy)benzyl)-5-methyl-2-(4-(pyridin-4-yl)phenyl)oxazole |
| 65 | 4-(4-(4-fluorophenoxy)benzyl)-5-methyl-2-(2-methyl-4-(pyridin-4-yl)phenyl)oxazole |
| 66 | 2-(3-fluoro-4-(pyridin-4-yl)phenyl)-4-(4-(4-fluorophenoxy)benzyl)-5-methyloxazole |
| 67 | 4-((4-(4-fluorophenyl)piperidin-1-yl)methyl)-5-methyl-2-(4-(pyridin-4-yl)phenyl)oxazole |
| 69 | 5-methyl-2-(4-(pyridin-4-yl)phenyl)-4-((4-(4-(trifluoromethoxy)phenoxy)piperidin-1-yl)methyl)oxazole |
| 70 | 5-methyl-2-(4-(pyridin-4-yl)phenyl)-4-((4-(4-(trifluoromethoxy)phenyl)piperidin-1-yl)methyl)oxazole |
| 71 | 2-(4-(1H-imidazol-1-yl)phenyl)-4-(4-(4-fluorophenoxy)benzyl)-5-methyloxazole |
| 73 | 2-(4-(2H-1,2,3-triazol-2-yl)phenyl)-5-methyl-4-((4-(4-(trifluoromethoxy)phenoxy)piperidin-1-yl)methyl)oxazole |
| 74 | 2-(4-(2H-1,2,3-triazol-2-yl)phenyl)-5-methyl-4-((4-(4-(trifluoromethoxy)phenoxy)piperidin-1-yl)methyl)oxazole |
| 75 | 2-(4-(1H-imidazol-1-yl)phenyl)-5-methyl-4-((4-(4-(trifluoromethoxy)phenoxy)piperidin-1-yl)methyl)oxazole |
| 76 | 4-(4-((4-fluorobenzyl)oxy)benzyl)-5-methyl-2-(4-(pyridin-4-yl)phenyl)oxazole |
| 77 | 5-methyl-2-(4-(pyridin-4-yl)phenyl)-4-(4-((4-(trifluoromethyl)benzyl)oxy)benzyl)oxazole |
| 78 | 5-methyl-2-(4-(pyridin-4-yl)phenyl)-4-(4-((4-(trifluoromethoxy)benzyl)oxy)benzyl)oxazole |
| 79 | 2-(4-(1H-1,2,3-triazol-1-yl)phenyl)-4-(4-(4-fluorophenoxy)benzyl)-5-methyloxazole |
| 80 | 2-(4-(2H-1,2,3-triazol-2-yl)phenyl)-5-methyl-4-((4-(4-(trifluoromethoxy)phenyl)piperidin-1-yl)methyl)oxazole |
| 81 | 2-(benzo[d][1,3]dioxol-5-yl)-5-methyl-4-((4-(4-(trifluoromethoxy)phenyl)piperidin-1-yl)methyl)oxazole |
| 82 | 2-(benzo[d][1,3]dioxol-5-yl)-4-(4-(4-fluorophenoxy)benzyl)-5-methyloxazole |
| 83 | 2-(4-(1H-pyrazol-1-yl)phenyl)-5-methyl-4-((4-(4-(trifluoromethoxy)phenyl)piperidin-1-yl)methyl)oxazole |
| 84 | 5-methyl-2-(4-(pyridin-4-yl)phenyl)-4-((4-(4-(trifluoromethoxy)phenyl)piperazin-1-yl)methyl)oxazole |
| 85 | 2-(4-(1H-pyrazol-1-yl)phenyl)-5-methyl-4-((4-(4-(trifluoromethoxy)phenyl)piperazin-1-yl)methyl)oxazole |
| 86 | 2-(benzo[d][1,3]dioxol-5-yl)-5-methyl-4-((4-(4-(trifluoromethoxy)phenyl)piperazin-1-yl)methyl)oxazole |
| 87 | 1'-((2-(4-(1H-pyrazol-1-yl)phenyl)-5-methyloxazol-4-yl)methyl)-3H-spiro[isobenzofuran-1,4'-piperidine] |
| 88 | 2-(4-(1H-imidazol-1-yl)phenyl)-5-methyl-4-((4-(4-(trifluoromethoxy)phenyl)piperazin-1-yl)methyl)oxazole |
| 89 | 1'-((2-(4-(1H-imidazol-1-yl)phenyl)-5-methyloxazol-4-yl)methyl)-3H-spiro[isobenzofuran-1,4'-piperidine] |
| 90 | 2-(4-(1H-imidazol-1-yl)phenyl)-5-methyl-4-(spirofindene-1,4'-piperidin]-1'-ylmethyl)oxazole |
| 91 | 2-(4-(1H-pyrazol-1-yl)phenyl)-5-methyl-4-(spirofindene-1,4'-piperidin]-1'-ylmethyl)oxazole |
| 92 | 2-(benzo[d][1,3]dioxol-5-yl)-4-((2,3-dihydrospiro[indene-1,4'-piperidin]-1'-yl)methyl)-5-methyloxazole |
| 93 | 1'-((2-(benzo[d][1,3]dioxol-5-yl)-5-methyloxazol-4-yl)methyl)-3H-spiro[isobenzofuran-1,4'-piperidine] |
| 94 | 2-(4-(1H-imidazol-1-yl)phenyl)-5-methyl-4-((4-(4-(trifluoromethoxy)phenyl)piperidin-1-yl)methyl)oxazole |
| 95 | 2-(4-(1H-pyrazol-1-yl)phenyl)-4-((2,3-dihydrospiro[indene-1,4'-piperidin]-1'-yl)methyl)-5-methyloxazole |
| 96 | 2-(4-(1H-imidazol-1-yl)phenyl)-4-((2,3-dihydrospirofindene-1,4'-piperidin]-1'-yl)methyl)-5-methyloxazole |
| 98 | 2-(4-(1H-imidazol-1-yl)phenyl)-5-methyl-4-((4-(3-(trifluoromethoxy)phenyl)piperidin-1-yl)methyl)oxazole |
| 99 | 2-(4-(1H-pyrazol-1-yl)phenyl)-5-methyl-4-((4-(2-(trifluoromethoxy)phenyl)piperidin-1-yl)methyl)oxazole |
| 100 | 2-(4-(1H-imidazol-1-yl)phenyl)-5-methyl-4-((4-(2-(trifluoromethoxy)phenyl)piperidin-1-yl)methyl)oxazole |
| 101 | 2-(4-(1H-pyrazol-1-yl)phenyl)-5-methyl-4-((4-(2-(trifluoromethoxy)phenyl)-3,6-dihydropyridin-1(2H)-yl)methyl)oxazole |
| 103 | 2-(4-(1H-pyrazol-1-yl)phenyl)-5-methyl-4-((4-(4-(trifluoromethoxy)phenyl)-3,6-dihydropyridin-1(2H)-yl)methyl)oxazole |
| 106 | 2-(4-(1H-imidazol-1-yl)phenyl)-5-methyl-4-((4-(4-(trifluoromethoxy)phenyl)-3,6-dihydropyridin-1(2H)-yl)methyl)oxazole |
| 107 | 5-methyl-2-(4-(pyridin-4-yl)phenyl)-4-(spiro[indene-1,4'-piperidin]-1'-ylmethyl)oxazole |
| 108 | 1'-((5-methyl-2-(4-(pyridin-4-yl)phenyl)oxazol-4-yl)methyl)-3H-spiro[isobenzofuran-1,4'-piperidine] |
| 109 | 2-(4-(1H-pyrazol-1-yl)phenyl)-5-methyl-4-((4-phenylpiperidin-1-yl)methyl)oxazole |
| 112 | 2-(4-(1H-imidazol-1-yl)phenyl)-5-methyl-4-((4-(4-nitrophenyl)piperidin-1-yl)methyl)oxazole |
| 113 | 4-((2,3-dihydrospiro[indene-1,4'-piperidin]-1'-yl)methyl)-5-methyl-2-(4-(pyridin-4-yl)phenyl)oxazole |
| 114 | 4-(4-(4-fluorophenoxy)benzyl)-5-methyl-2-(4-(2-methyl-1H-imidazol-1-yl)phenyl)oxazole |
| 115 | 5-methyl-2-(4-(2-methyl-1 H-imidazol-1-yl)phenyl)-4-((4-(4-(trifluoromethoxy)phenyl)piperidin-1-yl)methyl)oxazole |
| 116 | 5-methyl-2-(4-(2-methyl-1H-imidazol-1-yl)phenyl)-4-(spiro[indene-1,4'-piperidin]-1'-ylmethyl)oxazole |
| 117 | 2-(4-(1H-pyrazol-1-yl)phenyl)-4-((4-(4-chlorophenyl)piperidin-1-yl)methyl)-5-methyloxazole |
| 121 | 2-(4-(1 H-imidazol-1-yl)phenyl)-4-((4-(4-chlorophenyl)piperidin-1-yl)methyl)-5-methyloxazole |
| 122 | 5-methyl-2-(4-(4-methyl-1H-imidazol-1-yl)phenyl)-4-((4-(4-(trifluoromethoxy)phenyl)piperidin-1-yl)methyl)oxazole |
| 127 | 4-(4-(5-methyl-4-(spiro[indene-1,4'-piperidin]-1'-ylmethyl)oxazol-2-yl)phenyl)morpholine |
| 128 | 4-(4-(5-methyl-4-((4-(4-(trifluoromethoxy)phenyl)piperidin-1-yl)methyl)oxazol-2-yl)phenyl)morpholine |
| 129 | 4-(4-(4-((2,3-dihydrospiro[indene-1,4'-piperidin]-1'-yl)methyl)-5-methyloxazol-2-yl)phenyl)morpholine |
| 131 | 2-(imidazo[1,2-a]pyridin-6-yl)-5-methyl-4-((4-(4-(trifluoromethoxy)phenyl)piperidin-1-yl)methyl)oxazole |
| 132 | 1'-((5-methyl-2-(4-morpholinophenyl)oxazol-4-yl)methyl)-3H-spiro[isobenzofuran-1,4'-piperidine] |
| 133 | 2-(4-(1H-pyrazol-1-yl)phenyl)-4-((4-(4-(difluoromethoxy)phenyl)piperidin-1-yl)methyl)-5-methyloxazole |
| 134 | 2-(4-(1H-imidazol-1-yl)phenyl)-4-((4-(4-(difluoromethoxy)phenyl)piperidin-1-yl)methyl)-5-methyloxazole |

6. An antibacterial pharmaceutical composition comprising the compound of any one of claims 1 to 5 or a pharmaceutically acceptable salt thereof as an active ingredient.

7. The antibacterial pharmaceutical composition of claim 6, wherein the bacteria are Mycobacterium genus bacteria.

8. The antibacterial pharmaceutical composition of claim 7, wherein the bacteria are Mycobacterium tuberculosis.

9. A pharmaceutical composition for treating or preventing tuberculosis, comprising the compound of any one of claims 1 to 5 or a pharmaceutically acceptable salt thereof as an active ingredient.

10. A method of treating a disease caused by a bacterial infection, comprising administering a therapeutically effective amount of the compound of any one of claims 1 to 5 or a pharmaceutically acceptable salt thereof to a subject in need of treatment.

11. The method of claim 10, wherein the bacteria are bacteria of the genus Mycobacterium.

12. The method of claim 11, wherein the disease is tuberculosis.
